(19) Europäisches Patentamt
European Patent Office
Office européen des brevets

(11) EP 4 157 853 B1

(12) **EUROPEAN PATENT SPECIFICATION**

(45) Date of publication and mention of the grant of the patent:
**31.07.2024 Bulletin 2024/31**

(21) Application number: **21813912.9**

(22) Date of filing: **28.05.2021**

(51) International Patent Classification (IPC):
***C07H 17/02*** $^{(2006.01)}$ ***A61K 31/706*** $^{(2006.01)}$
***A61P 1/00*** $^{(2006.01)}$ ***A61P 9/00*** $^{(2006.01)}$
***A61P 11/00*** $^{(2006.01)}$ ***A61P 17/00*** $^{(2006.01)}$
***A61P 19/00*** $^{(2006.01)}$ ***A61P 29/00*** $^{(2006.01)}$
***A61P 35/00*** $^{(2006.01)}$ ***A61P 37/06*** $^{(2006.01)}$
***A61P 43/00*** $^{(2006.01)}$

(52) Cooperative Patent Classification (CPC):
(C-Sets available)
**C07H 17/02; A61K 31/706; A61K 45/06; A61P 1/00; A61P 9/00; A61P 11/00; A61P 17/00; A61P 19/00; A61P 29/00; A61P 35/00; A61P 37/06; A61P 43/00** (Cont.)

(86) International application number:
**PCT/CA2021/050725**

(87) International publication number:
**WO 2021/237364 (02.12.2021 Gazette 2021/48)**

(54) **GUT MICROBIOTA BIOACTIVATED PDE4 INHIBITOR PRECURSORS**

BIOAKTIVIERTE PDE4-INHIBITORVORLÄUFER DER DARMFLORA

PRÉCURSEURS D'INHIBITEUR DE PDE4 BIOACTIVÉS DU MICROBIOTE INTESTINAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2020 US 202063031023 P**

(43) Date of publication of application:
**05.04.2023 Bulletin 2023/14**

(73) Proprietor: **Giiant Pharma Inc.**
**Montréal, QC H3B 4W5 (CA)**

(72) Inventor: **CÔTÉ, Bernard**
**Québec J7V 8P6 (CA)**

(74) Representative: **Leonard, Thomas Charles et al**
**Kilburn & Strode LLP**
**Lacon London**
**84 Theobalds Road**
**London WC1X 8NL (GB)**

(56) References cited:
**WO-A1-2004/005258 WO-A2-01/70738**
**CA-A1- 2 369 323**

- **LI CHUN ET AL: "Investigation of the in vitro metabolism profile of a phosphodiesterase-IV inhibitor, CDP-840: leading to structural optimization", vol. 39, no. 3, 1 March 2001 (2001-03-01), pages 232 - 241, XP009548798, ISSN: 0090-9556, Retrieved from the Internet <URL:https://dmd.aspetjournals.org/content/29/3/232>**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/706, A61K 2300/00**

## Description

### CROSS-REFERENCE TO RELATED APPLICATIONS

This application claims priority from and the benefit of United States Provisional Patent Application No. 63/031,023, filed on May 28, 2020.

### BACKGROUND

(a) Field

The subject matter disclosed generally relates to compounds that are glycoside prodrugs of phosphodiesderase 4 (PDE4) inhibitors. In particular, this invention is directed to di-aryl-substituted-ethane pyridones conjugated with (a) a $\beta$-D-glucuronide, (b) an $\alpha$-D-glucuronide, (c) a $\beta$-D-glucopyranoside, (d) an $\alpha$-D-glucopyranoside, (e) a $\beta$-D-galactopyranoside, (f) an $\alpha$-D-galactopyranoside, (g) a $\beta$-D-mannopyranoside, (h) an $\alpha$-D-mannopyranoside, (i) a *N*-acetyl-$\beta$-D-glucosaminide, (j) a *N*-acetyl-$\alpha$-D-glucosaminide, (k) a *N*-acetyl-$\beta$-D-galactosaminide, (l) a *N*-acetyl-$\alpha$-D-galactosaminide, (m) a $\beta$-D-glucosaminide, (n) an $\alpha$-D-glucosaminide, (o) a $\beta$-D-galactosaminide, (p) an $\alpha$-D-galactosaminide, (q) a $\beta$-D-fucopyranoside, (r) an $\alpha$-L-fucopyranoside, (s) an $\alpha$-L-rhamnopyranoside, (t) an $\alpha$-L-arabinofuranoside, (u) a $\beta$-D-ribofuranoside, (v) a polysaccharide such as a $\beta$-D-cellobioside or an $\alpha$-D-cellobioside or a $\beta$-*N,N*-diacetyl chitobioside, (w) a D-xylopyranoside, (x) a D-xylofuranoside, (y) a $\beta$-D-galacturonide or (z) an $\alpha$-D-galacturonide moiety which are colon-specific prodrug of PDE4 inhibitors.

(b) Related Prior Art

Cyclic adenosine monophosphate (adenosine 3', 5'-cyclic monophosphate, "cAMP" or "cyclic AMP") is known as a second messenger for hormones including epinephrine, glucagon, calcitonin, corticotrophin, lipotropin, luteinizing hormone, norepinephrine, parathyroid hormone, thyroid-stimulating hormone, and vasopressin. Thus, cAMP mediates cellular responses to hormones. Cyclic AMP also mediates cellular responses to various neurotransmitters.

Phosphodiesterases ("PDE") are a family of enzymes that metabolize 3', 5' cyclic nucleotides to 5' nucleoside monophosphates, thereby terminating cAMP second messenger activity. A particular phosphodiesterase, phosphodiesterase-4 ("PDE4", also known as "PDE-IV"), which is a high affinity, cAMP specific, type IV PDE, has generated interest as potential targets for the development of novel anti-inflammatory compounds. PDE4 is known to exist as at least four isoenzymes (A, B, C and D), each of which is encoded by a distinct gene. Each of the four known PDE4 gene products are believed to play varying roles in allergic and/or inflammatory responses. Thus, it is believed that inhibition of PDE4, particularly the specific PDE4 isoforms that produce detrimental responses, can beneficially affect allergy and inflammation symptoms. It would be desirable to provide novel compounds and compositions that inhibit PDE4 activity.

Tumor necrosis factor alpha, (TNF-$\alpha$) is a cytokine that is released primarily by mononuclear phagocytes in response to immunostimulators. TNF$\alpha$ is capable of enhancing most cellular processes, such as differentiation, recruitment, proliferation, and proteolytic degradation. At low levels, TNF-$\alpha$ confers protection against infective agents, tumors, and tissue damage. But TNF-$\alpha$ also has a role in many diseases. When administered to mammals or humans, TNF-$\alpha$ causes or aggravates inflammation, fever, cardiovascular effects, hemorrhage, coagulation, and acute phase responses similar to those seen during acute infections and shock states. Enhanced or unregulated TNF-$\alpha$ production has been implicated in a number of diseases and medical conditions, for example, cancers, such as solid tumors and blood-born tumors; heart disease, such as congestive heart failure; and viral, genetic, inflammatory, allergic, and autoimmune diseases.

Inflammatory diseases such as arthritis, related arthritic conditions (e.g., osteoarthritis and rheumatoid arthritis), inflammatory bowel disease (e.g., Crohn's disease and ulcerative colitis), sepsis, psoriasis, psoriatic arthritis, atopic dermatitis, contact dermatitis, chronic obstructive pulmonary disease and chronic inflammatory pulmonary diseases are also prevalent and problematic ailments. TNF-$\alpha$ plays a central role in the inflammatory response and the administration of their antagonists block chronic and acute responses in animal models of inflammatory disease.

Pharmaceutical compounds such as PDE4 inhibitors that can block the activity or inhibit the production of certain cytokines, including TNF-$\alpha$, may be beneficial therapeutics to treat or prevent inflammatory diseases implicated by TNF-$\alpha$.

Ulcerative colitis is a relapsing chronic inflammatory bowel disease that affects the lining of the colon and rectum. Current therapies include 5-aminosalicylates, corticosteroids, immunomodulatory agent, TNF-$\alpha$ suppressors such as infliximab (Remicade™), adalimumab (Humira™), golimumab (Simponi™) and $\alpha 4\beta 7$ integrin suppressor such as vedolizumab (Entyvio™).

PDE4 inhibitors are well known to suppress the synthesis of the pro-inflammatory cytokine TNF-$\alpha$ that plays a

crucial role in the pathogenesis of ulcerative colitis. The demonstrated efficacy of TNF-α suppressors in the treatment of ulcerative colitis further supports the utility of PDE4 inhibitors as an alternative therapy.

One of the very first medications used in the treatment of ulcerative colitis is a prodrug called sulfasalazine. The sulfonamide moiety acts as a carrier to deliver the active component 5-aminosalicylic acid (5-ASA, also known as mesalazine or mesalamine) to the colon. The specific bacterial action of the colonic microbiota is responsible for the cleavage of the diazo bond of sulfasalazine.

Orally administered topical corticosteroid budesonide as a colon-specific controlled release formulation (Uceris™), which reduces systemic exposure, is efficacious in the management of ulcerative colitis and minimize the side effects (Sandborn et al. Gastroenterology 2012, 143, 1218).

The primary rational for topical therapy in the treatment of ulcerative colitis is that it treats directly the inflamed colon mucosa while minimizing systemic side effects.

In oral drug disposition of actives in the body, it is known that during intrahepatic recirculation, a variety of drug glucuronide conjugates that undergo biliary excretion are reabsorbed in the gastrointestinal tract and hydrolyzed by the colon *β*-glucuronidase to release the parent active principle.

Bacteria that colonize the mammalian intestine possess a large repertoire of carbohydrate processing enzymes such as, but not restricted to, glycoside hydrolases (glycosidases), polysaccharide lyases and carbohydrate esterases with degradative and metabolic capabilities (Flint et al. Gut Microbes, 2012, 3(4), 289).

Numerous companies have invested in the development of specific PDE4 inhibitors as anti-inflammatory agents for which two of those, roflumilast (Daliresp™, Takeda, COPD) and apremilast (Otezla™, Celgene, psoriasis/psoriatic arthritis) have gained approval from regulatory agency and reach the market. Apremilast has also demonstrated its ability to induce and maintain clinical remission up to 52 weeks in patients suffering from moderate-to-severe ulcerative colitis in a phase 2 clinical trial (Danese, S. et al. Clin. Gastroenterol. Hepato. 2020, 18(11), 2526-2534). Regardless of the indication, a common side-effect of these treatments has been headache and gastrointestinal disturbance such as nausea, emesis and diarrhea. Further related prior art may be found in Li Chun et Al. : Drug Metabolism and Disposition, Pharmacology and Experimental Therapeutics, vol. 39, no. 3(1), 2001, pp. 232-241, WO 2004/005258 A1,WO01/70738 A2 and CA 2369323 A1.

Accordingly, there is a need for PDE4 inhibiting compounds that reduce or mitigate the disadvantages of the compounds known in the art.

Accordingly, there is a need for PDE4 inhibiting compounds that cause little or no headache, nausea, emesis and/or diarrhea.

## SUMMARY

The present invention is directed to compounds that are glycoside prodrugs of phosphodiesderase 4 (PDE4) inhibitors. In particular, this invention is directed to di-aryl-substituted-ethane pyridones conjugated with (a) a *β*-D-glucuronide, (b) an *α*-D-glucuronide, (c) a *β*-D-glucopyranoside, (d) an *α*-D-glucopyranoside, (e) a *β*-D-galactopyranoside, (f) an *α*-D-galactopyranoside, (g) a *β*-D-mannopyranoside, (h) an *α*-D-mannopyranoside, (i) a *N*-acetyl-*β*-D-glucosaminide, (j) a *N*-acetyl-*α*-D-glucosaminide, (k) a *N*-acetyl-*β*-D-galactosaminide, (l) a *N*-acetyl-*α*-D-galactosaminide, (m) a*β*-D-glucosaminide, (n) an *α*-D-glucosaminide, (o) a *β*-D-galactosaminide, (p) an *α*-D-galactosaminide, (q) a *β*-D-fucopyranoside, (r) an *α*-L-fucopyranoside, (s) an *α*-L-rhamnopyranoside, (t) an *α*-L-arabinofuranoside, (u) a *β*-D-ribofuranoside, (v) a polysaccharide such as a *β*-D-cellobioside or an a *α*-D-cellobioside or a *β*-*N,N*-diacetyl chitobioside, (w) a D-xylopyranoside, (x) a D-xylofuranoside, (y) a *β*-D-galacturonide or (z) an *α*-D-galacturonide moiety which are colon-specific prodrug of PDE4 inhibitors.

According to an embodiment, there is provided a compound of Formula (I):

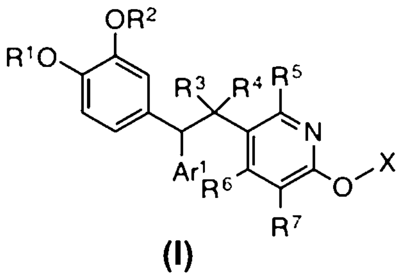

**(I)**

or a pharmaceutically acceptable salt thereof, wherein

X is a *β*-D-glucuronide, an *α*-D-glucuronide, a *β*-D-glucopyranoside, an *α*-D-glucopyranoside, a *β*-D-galactopyranoside, an *α*-D-galactopyranoside, a *β*-D-mannopyranoside, an *α*-D-mannopyranoside, an *N*-acetyl-*β*-D-glucosaminide, an *N*-acetyl-*α*-D-glucosaminide, an *N*-acetyl-*β*-D-galactosaminide, an *N*-acetyl-*α*-D-galactosaminide, a *β*-D-glucosaminide, an *α*-D-glucosaminide, a *β-D*-galactosaminide, an *α*-D-galactosaminide, a *β*-D-fucopyranoside, an *α*-L-fucopyranoside, an *α*-L-rhamnopyranoside, an *α*-L-arabinofuranoside, a *β*-D-ribofuranoside, a *β*-D-cellobioside, an *α*-D-cellobioside, a *β-N,N*-diacetyl chitobioside, a D-xylopyranoside, a D-xylofuranoside, a *β*-D-galacturonide or an *α*-D-galacturonide;

$R^1$ and $R^2$ are each independently -$C_{1-6}$alkyl, -$C_{3-6}$cycloalkyl, any of which is unsubstituted or substituted with 1-6 independent halogen;

$R^3$ and $R^4$ are each independently H, or-$C_{1-6}$alkyl;

$R^5$, $R^6$ and $R^7$ are each independently H, halogen, -$C_{1-6}$alkyl, -C(O)$C_{1-6}$alkyl, or CN;

$Ar^1$ is independently selected from the group consisting of:

(a) 6-$R^8$-3-pyridyl or 6-$R^9$-3-pyridyl,
(b) 2-$R^8$-5-thiazolyl or 5-$R^8$-2-thiazolyl,
(c) 2-$R^8$-5-pyrimidinyl or 2-$R^9$-5-pyrimidinyl,
(d) 6-$R^8$-3-pyridazinyl or 6-$R^9$-3-pyridazinyl,
(e) 5-$R^8$-2-furyl,
(f) 5-$R^8$-2-thienyl,
(g) 2-$R^8$-5-oxazolyl or 5-$R^8$-2-oxazolyl,
(h) 5-$R^8$-3-isoxazolyl or 3-$R^8$-5-isoxazolyl,
(i) 5-$R^8$-3-isothiazolyl or 3-$R^8$-5-isothiazolyl, and
(j) *p*-$R^8$-phenyl;

$R^8$ is selected from the group consisting of: H, halogen, -$C_{1-6}$alkyl, -$C_{3-6}$cycloalkyl, -$C_{1-6}$alkyl$Ar^2$, $Ar^2$, $C_{1-6}$alkoxy, $C_{1-6}$alkylthio, CN, -C($R^{10}$)($R^{11}$)OH, -C($R^{10}$)($R^{11}$)O$C_{1-6}$alkyl, - C($R^{10}$)($R^{11}$)O$Ar^2$, -$CO_2$H, -$CO_2C_{1-6}$alkyl, -C(O)N$R^{12}R^{13}$, -$SO_2$NHC(O)$Ar^2$, -C(O)$C_{1-6}$alkyl and - C(O)$Ar^2$;

$R^9$ is selected from the group consisting of: -N$R^{12}R^{13}$, -N$R^{12}$C(O)$R^{13}$, - N$R^{12}$C(O)NH$R^{13}$, -N$R^{12}SO_2Ar^2$, and -N$R^{12}CO_2Ar^2$;

$R^{10}$ and $R^{11}$ are each independently H, -$C_{1-6}$alkyl, -$C_{1-6}$haloalkyl, -$C_{3-6}$cycloalkyl, or $Ar^2$;

or when $R^{10}$ and $R^{11}$ are -$C_{1-6}$alkyl, they may connect together through a $C_{1-3}$alkyl to form $C_{3-6}$cycloalkyl;

$R^{12}$ and $R^{13}$ are each independently H, -$C_{1-6}$alkyl, -$C_{3-6}$cycloalkyl, or-$C_{1-6}$alkyl$Ar^2$;

or when $R^{12}$ and $R^{13}$ are -$C_{1-6}$alkyl, they may connect together through a $C_{1-3}$alkyl to form a $C_{3-6}$heterocycloalkyl;

$Ar^2$ is selected from the group consisting of: phenyl, pyridinyl, quinolinyl, isoquinolinyl, pyridazinyl, pyrimidinyl, pyrazinyl, quinoxalinyl, furyl, benzofuryl, dibenzofuryl, thienyl, benzothienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl, and tetrazolyl;

each $Ar^2$ being unsubstituted or substituted with 1-3 members selected from the group consisting of: halo, -$C_{1-6}$alkyl, -$C_{1-6}$haloalkyl, CN, $C_{1-6}$alkoxy, $C_{1-6}$alkylthio, -C($R^{10}$)($R^{11}$)OH, -$CO_2$H, - $CO_2C_{1-6}$alkyl, -C(O)N$R^{12}R^{13}$, and -$SO_2CH_3$.

The *β*-D-glucuronide may be *β*-D-glucuronic acid, methyl *β*-D-glucuronidate, methyl 2,3,4-tri-*O*-acetyl-*β*-D-glucuronidate, ethyl 2,3,4-tri-*O*-acetyl-*β*-D-glucuronidate, ethyl *β*-D-glucuronidate, *i*-propyl *β*-D-glucuronidate, tert-butyl *β*-D-glucuronidate, or methyl *β*-D-glucuronamide.

The *β*-D-glucopyranoside may be *β*-D-glucopyranosyl, 2,3,4,6-tetra-*O*-acetyl-*β*-D-glucopyranosyl, or 3,4,6-tri-*O*-acetyl-*β*-D-glucopyranosyl.

The *β*-D-galactopyranoside may be *β*-D-galactopyranosyl, or 2,3,4,6-tetra-*O*-acetyl-*β*-D-galactopyranosyl.

The *α*-D-mannopyranoside may be *α*-D-mannopyranosyl, or 2,3,4,6-tetra-*O*-acetyl-*α*-D-mannopyranosyl.

The *β*-D-glucosaminide may be *β*-D-glucosaminyl, or *α*-D-glucosaminyl.

The *N*-acetyl-*β*-D-glucosaminide may be *N*-acetyl-*β*-D-glucosaminyl, 3,4,6-tri-O-acetyl-*N*-acetyl-*β*-D-glucosaminyl, *N*,*N*,*N*-trimethyl-*β*-D-glucosaminyl, or *N*,*N*-dimethyl-*β*-D-glucosaminyl.

The *β*-D-cellobioside may be *β*-D-cellobiosyl, or 2,3,6,2',3',4',6'-hepta-*O*-acetyl-*β*-D-cellobiosyl.

The $Ar^1$ may be 6-$R^8$-3-pyridyl or 2-$R^8$-5-thiazolyl. The $Ar^1$ may be 6-$R^8$-3-pyridyl.

The $R^3$ and $R^4$ may each be H.

The $R^5$, $R^6$ and $R^7$ may each be H.

The $R^8$ may be -C($R^{10}$)($R^{11}$)OH.

The *β*-D-glucuronide may be *β*-D-glucuronyl.

The *β*-D-glucuronyl may be methyl glucuronate.

The compound of formula (I) may be one of the following compounds:

| Cmpd | Structure | Cmpd | Structure |
| --- | --- | --- | --- |
| 1 | 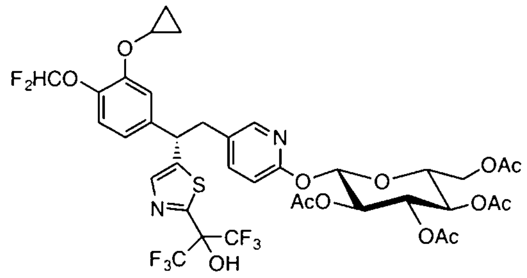 | 17 | 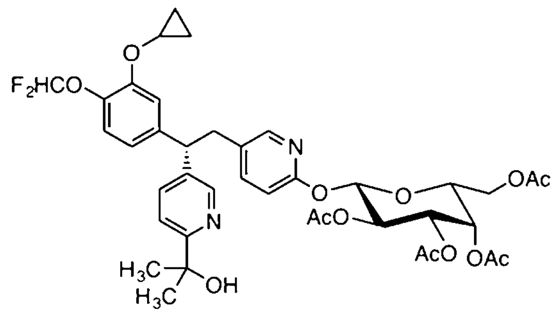 |
| 2 | 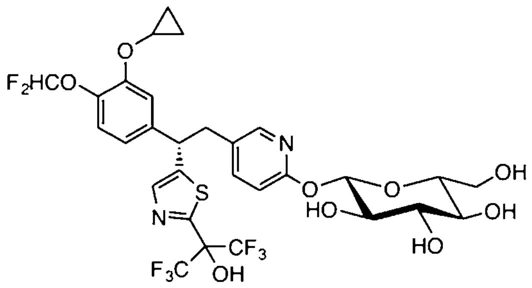 | 18 | 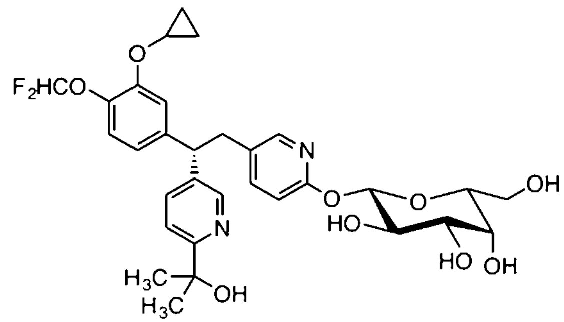 |
| 3 | 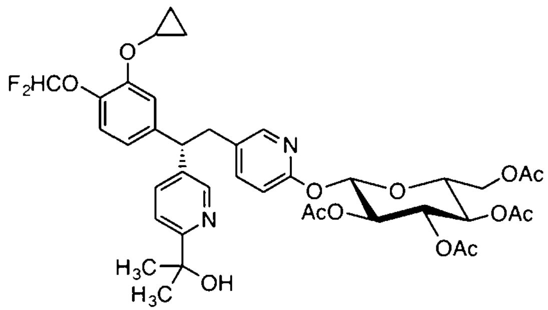 | 19 | 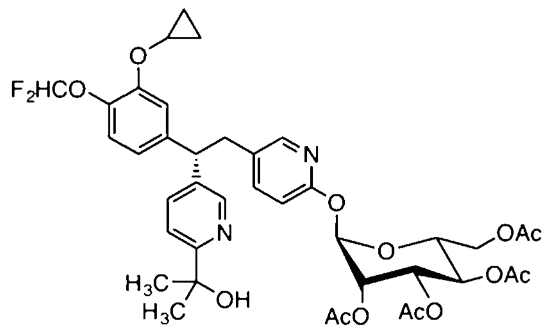 |

(continued)

| Cmpd | Structure | Cmpd | Structure |
|---|---|---|---|
| 4 | 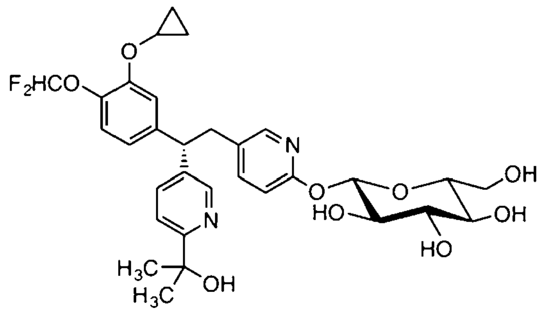 | 20 | 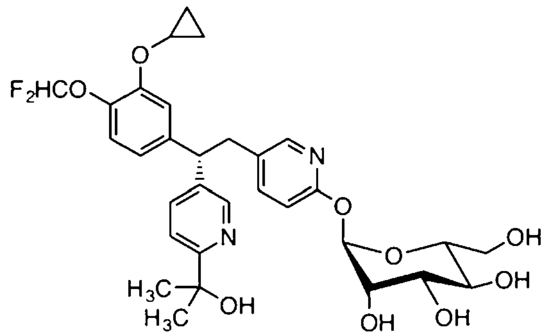 |
| 5 | 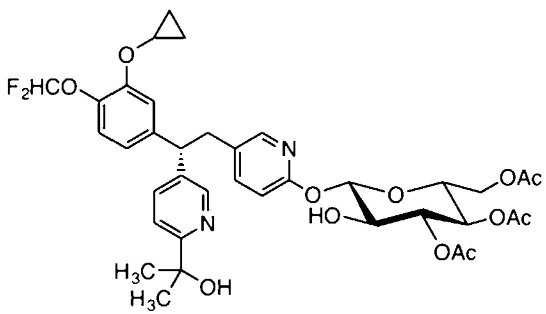 | 21 | 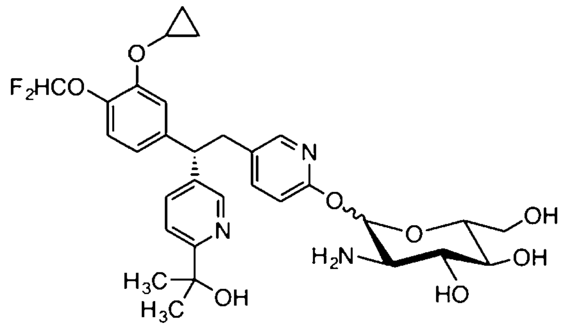 |
| 6 | 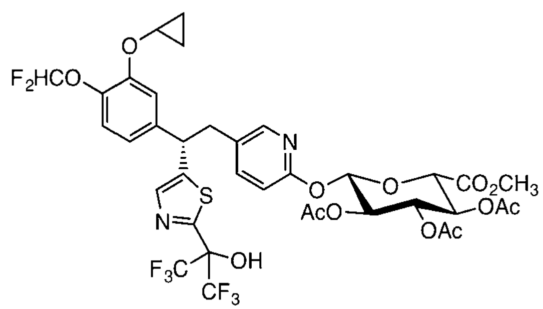 | 22 | 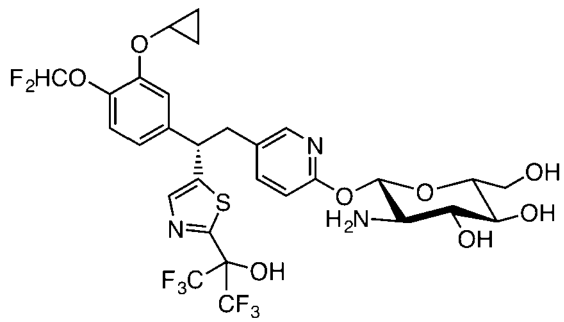 |

(continued)

| Cmpd | Structure | Cmpd | Structure |
|---|---|---|---|
| 7 | 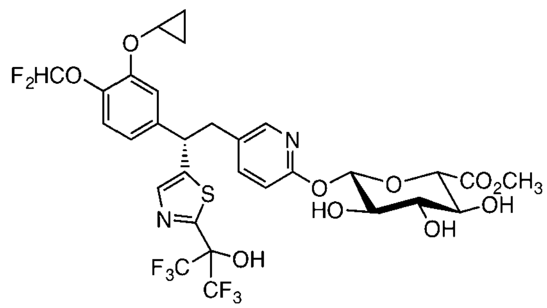 | 23 | 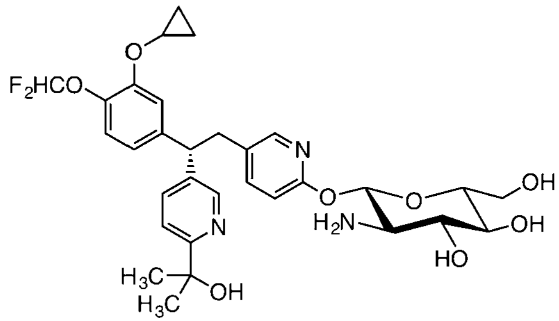 |
| 8 | 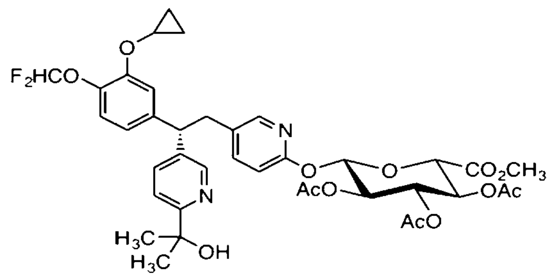 | 24 | 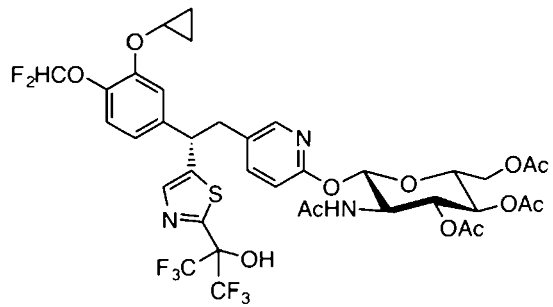 |
| 9 | 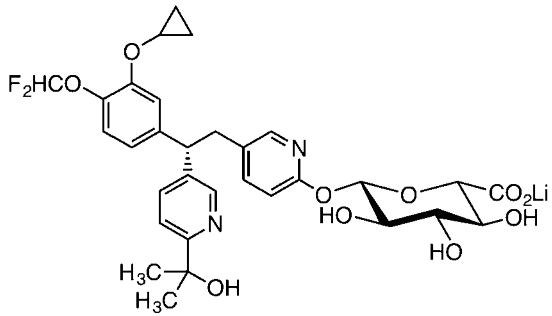 | 25 | 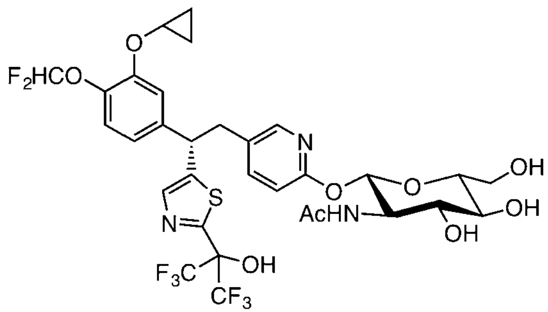 |

(continued)

| Cmpd | Structure | Cmpd | Structure |
|---|---|---|---|
| 10 | 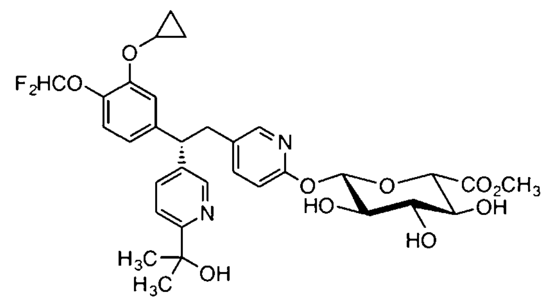 | 26 | 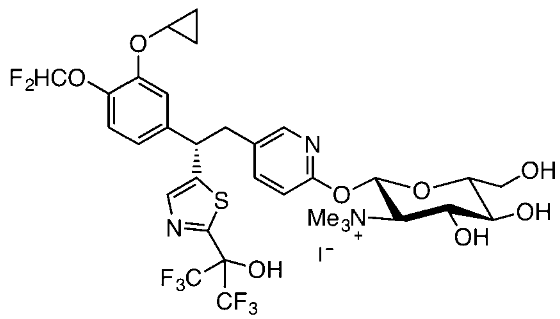 |
| 11 | 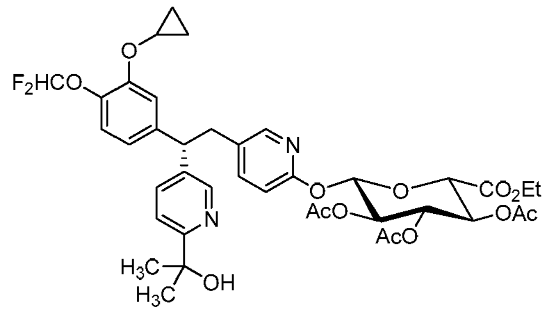 | 27 | 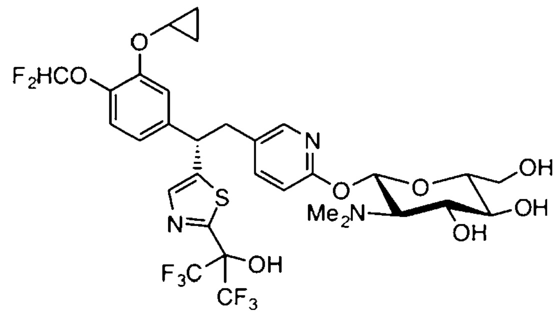 |
| 12 | 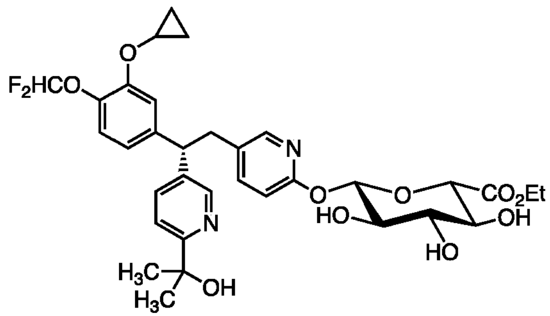 | 28 | 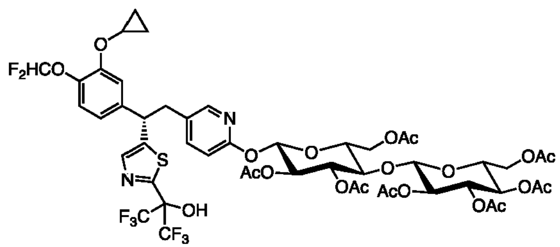 |

(continued)

| Cmpd | Structure | Cmpd | Structure |
|---|---|---|---|
| 13 | 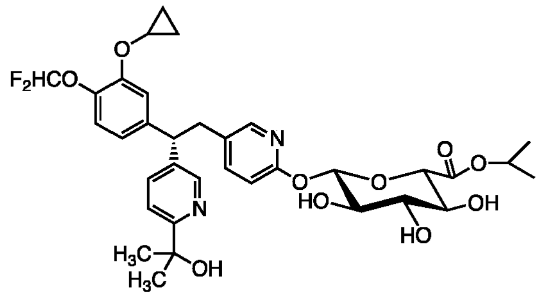 | 29 | 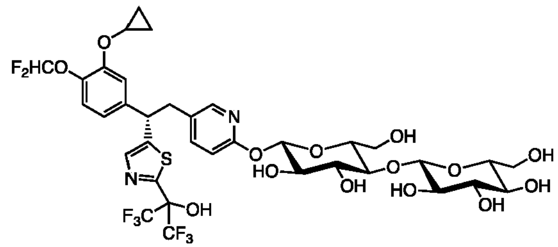 |
| 14 | 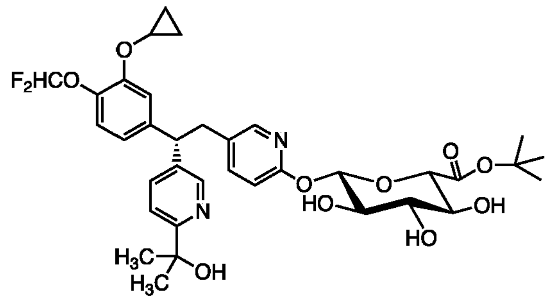 | 30 | 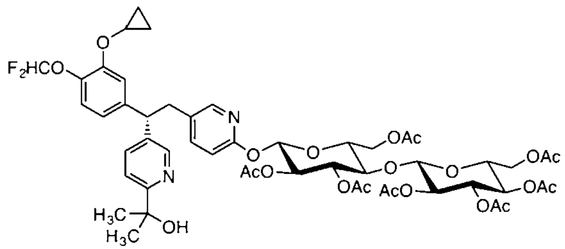 |
| 15 | 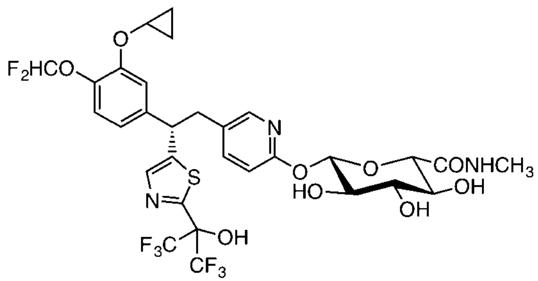 | 31 | 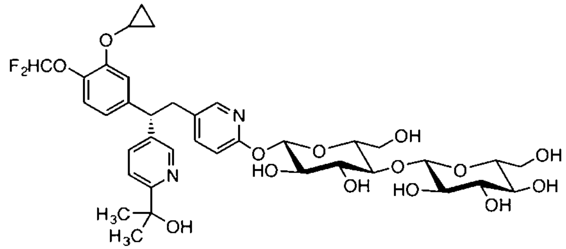 |

(continued)

| Cmpd | Structure | Cmpd | Structure |
|---|---|---|---|
| 16 | 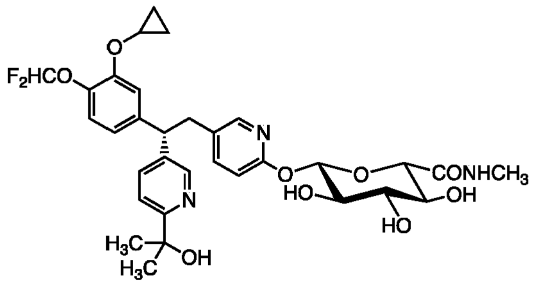 | | |

or a pharmaceutically acceptable salt thereof.

The compound of formula (I) may be represented by

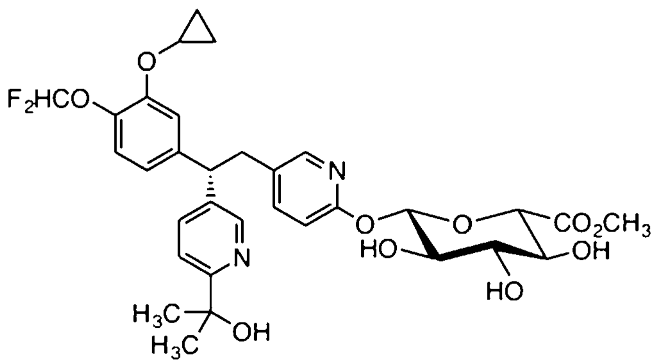

or a pharmaceutically acceptable salt thereof.

The compound of formula (I) may be represented by

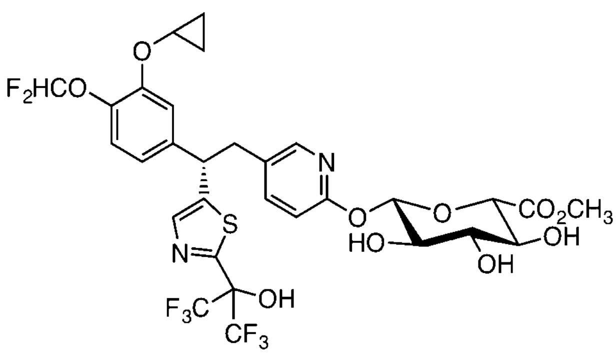

or a pharmaceutically acceptable salt thereof.

The compound of formula (I) may be represented by

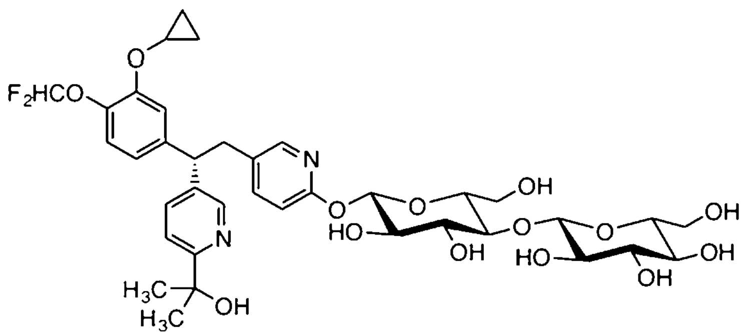

or a pharmaceutically acceptable salt thereof.

This invention also provides a pharmaceutical composition which includes an effective amount of the novel di-aryl-substituted-ethane pyridone glycoside conjugate and a pharmaceutically acceptable carrier.

According to another embodiment, there is provided a pharmaceutical composition comprising a therapeutically effective amount of the compound of formula (**I**), or a pharmaceutically acceptable salt thereof, and a pharmaceutically acceptable carrier or diluent or excipients.

The therapeutically effective amount may be about 0.001 mg, 0.005 mg, 0.025mg, 0.1 mg, 0.5 mg, 2.5 mg, 10 mg, 50 mg, 250 mg, or 1000 mg of the compound of formula (I).

The composition may be at least one of an immediate release formulation, a sustained release formulation or a delayed release formulation, or a combination thereof.

The composition may be in the form of a lotion or a liquid.

The pharmaceutical composition may further comprise a leukotriene receptor antagonist, a leukotriene biosynthesis inhibitor, an M2/M3 antagonist, a corticosteroid, an HI receptor antagonist, a $\beta_2$ adrenoceptor agonist, a selective COX-2 inhibitor, an NSAID, an immunomodulator, 5-ASA, a 5-ASA prodrug, a janus kinase inhibitor or a combination thereof.

According to another embodiment, there is provided a use of a compound of formula (**I**), or of the composition of the present invention, in the manufacture of a medicament for the treatment or prevention of asthma, chronic bronchitis, chronic obstructive pulmonary disease (COPD), adult respiratory distress syndrome, infant respiratory distress syndrome, cough, chronic obstructive pulmonary disease in animals, ulcerative colitis, Crohn's disease, diverticulitis, irritable bowel syndrome, hypersecretion of gastric acid, sepsis or septic shock, endotoxic shock, an endotoxic shock associated condition, spinal cord trauma, head injury, neurogenic inflammation, pain, reperfusion injury of the brain, psoriatic arthritis, rheumatoid arthritis, ankylosing spondylitis, osteoarthritis, inflammation and cytokine-mediated chronic tissue degeneration, allergic rhinitis, allergic conjunctivitis, eosinophilic granuloma, depression, memory impairment, monopolar depression, Parkinson disease, Alzheimer's disease, acute and chronic multiple sclerosis, psoriasis, a benign proliferative skin disease, a malignant proliferative skin disease, atopic dermatitis, urticaria, cancer, tumor growth, cancerous invasion of normal tissues, diabetes insipidus, osteoporosis, arterial restenosis, atherosclerosis, reperfusion injury of the myocardium, chronic glomerulonephritis, vernal conjunctivitis, transplant rejection and graft versus host disease, and cachexia.

According to another embodiment, there is provided a use of a compound of formula (**I**), or of the composition of the present invention, in the manufacture of a medicament for the treatment or prevention of ulcerative colitis, Crohn's disease, chronic obstructive pulmonary disease (COPD), psoriatic arthritis or psoriasis.

According to another embodiment, there is provided a compound of formula (**I**), or the composition of the present invention for use in the treatment or prevention of asthma, chronic bronchitis, chronic obstructive pulmonary disease (COPD), adult respiratory distress syndrome, infant respiratory distress syndrome, cough, chronic obstructive pulmonary disease in animals, ulcerative colitis, Crohn's disease, diverticulitis, irritable bowel syndrome, hypersecretion of gastric acid, sepsis or septic shock, endotoxic shock, an endotoxic shock associated condition, spinal cord trauma, head injury, neurogenic inflammation, pain, reperfusion injury of the brain, psoriatic arthritis, rheumatoid arthritis, ankylosing spondylitis, osteoarthritis, inflammation and cytokine-mediated chronic tissue degeneration, allergic rhinitis, allergic conjunctivitis, eosinophilic granuloma, depression, memory impairment, monopolar depression, Parkinson disease, Alzheimer's disease, acute and chronic multiple sclerosis, psoriasis, a benign proliferative skin disease, a malignant proliferative skin disease, atopic dermatitis, urticaria, cancer, tumor growth, cancerous invasion of normal tissues, diabetes insipidus, osteoporosis, arterial restenosis, atherosclerosis, reperfusion injury of the myocardium, chronic glomerulonephritis, vernal conjunctivitis, transplant rejection and graft versus host disease, and cachexia.

According to another embodiment, there is provided a compound of formula (**I**), or the composition of the present invention for use in the treatment or prevention of ulcerative colitis, Crohn's disease, chronic obstructive pulmonary disease (COPD), psoriatic arthritis or psoriasis.

According to another embodiment, there is provided a use of a compound formula (**I**), or of the composition of the present invention in the treatment or prevention of asthma, chronic bronchitis, chronic obstructive pulmonary disease (COPD), adult respiratory distress syndrome, infant respiratory distress syndrome, cough, chronic obstructive pulmonary disease in animals, ulcerative colitis, Crohn's disease, diverticulitis, irritable bowel syndrome, hypersecretion of gastric acid, sepsis or septic shock, endotoxic shock, an endotoxic shock associated condition, spinal cord trauma, head injury, neurogenic inflammation, pain, reperfusion injury of the brain, psoriatic arthritis, rheumatoid arthritis, ankylosing spondylitis, osteoarthritis, inflammation and cytokine-mediated chronic tissue degeneration, allergic rhinitis, allergic conjunctivitis, eosinophilic granuloma, depression, memory impairment, monopolar depression, Parkinson disease, Alzheimer's disease, acute and chronic multiple sclerosis, psoriasis, a benign proliferative skin disease, a malignant proliferative skin disease, atopic dermatitis, urticaria, cancer, tumor growth, cancerous invasion of normal tissues, diabetes insipidus, osteoporosis, arterial restenosis, atherosclerosis, reperfusion injury of the myocardium, chronic glomerulonephritis, vernal conjunctivitis, transplant rejection and graft versus host disease, and cachexia.

According to another embodiment, there is provided a use of a compound formula (**I**), or of the composition of the present invention in the treatment or prevention of ulcerative colitis, Crohn's disease, chronic obstructive pulmonary disease (COPD), psoriatic arthritis or psoriasis.

Features and advantages of the subject matter hereof will become more apparent in light of the following detailed description of selected embodiments, as illustrated in the accompanying figures. As will be realized, the subject matter disclosed and claimed is capable of modifications in various respects, all without departing from the scope of the claims. Accordingly, the drawings and the description are to be regarded as illustrative in nature, and not as restrictive and the full scope of the subject matter is set forth in the claims.

## DETAILED DESCRIPTION

In embodiments, there are disclosed compounds that are inactive precursors of PDE4 inhibiting compounds that are believed to cause little or no nausea, emesis and/or diarrhea.

In embodiment there is disclosed a compound represented by Formula (I):

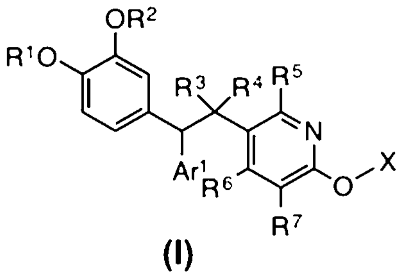

**(I)**

or a pharmaceutically acceptable salt thereof, wherein

X is a *β*-D-glucuronide, an *α*-D-glucuronide, a *β*-D-glucopyranoside, an *α*-D-glucopyranoside, a *β*-D-galactopyranoside, an *α*-D-galactopyranoside, a *β*-D-mannopyranoside, an *α*-D-mannopyranoside, an *N*-acetyl-*β*-D-glucosaminide, an *N*-acetyl-*α*-D-glucosaminide, an *N*-acetyl-*β*-D-galactosaminide, an *N*-acetyl-*α*-D-galactosaminide, a *β*-D-glucosaminide, an *α*-D-glucosaminide, a *β*-D-galactosaminide, an *α*-D-galactosaminide, a *β*-D-fucopyranoside, an *α*-L-fucopyranoside, an *α*-L-rhamnopyranoside, an *α*-L-arabinofuranoside, a *β*-D-ribofuranoside, a *β*-D-cellobioside, an *α*-D-cellobioside, a *β-N,N*-diacetyl chitobioside, a D-xylopyranoside, a D-xylofuranoside, an *β*-D-galacturonide or an *α*-D-galacturonide;

$R^1$ and $R^2$ are each independently $-C_{1-6}$alkyl, $-C_{3-6}$cycloalkyl, any of which is unsubstituted or substituted with 1-6 independent halogen;

$R^3$ and $R^4$ are each independently H, or $-C_{1-6}$alkyl;

$R^5$, $R^6$ and $R^7$ are each independently H, halogen, $-C_{1-6}$alkyl, $-C(O)C_{1-6}$alkyl, or CN;

$Ar^1$ is independently selected from the group consisting of:

(a) 6-$R^8$-3-pyridyl or 6-$R^9$-3-pyridyl,
(b) 2-$R^8$-5-thiazolyl or 5-$R^8$-2-thiazolyl,
(c) 2-$R^8$-5-pyrimidinyl or 2-$R^9$-5-pyrimidinyl,
(d) 6-$R^8$-3-pyridazinyl or 6-$R^9$-3-pyridazinyl,
(e) 5-$R^8$-2-furyl,
(f) 5-$R^8$-2-thienyl,
(g) 2-$R^8$-5-oxazolyl or 5-$R^8$-2-oxazolyl,
(h) 5-$R^8$-3-isoxazolyl or 3-$R^8$-5-isoxazolyl,
(i) 5-$R^8$-3-isothiazolyl or 3-$R^8$-5-isothiazolyl, and
(j) *p*-$R^8$-phenyl;

$R^8$ is selected from the group consisting of: H, halogen, $-C_{1-6}$alkyl, $-C_{3-6}$cycloalkyl, $-C_{1-6}$alkyl$Ar^2$, $Ar^2$, $C_{1-6}$alkoxy, $C_{1-6}$alkylthio, CN, $-C(R^{10})(R^{11})OH$, $-C(R^{10})(R^{11})OC_{1-6}$alkyl, $-C(R^{10})(R^{11})OAr^2$, $-CO_2H$, $-CO_2C_{1-6}$alkyl, $-C(O)NR^{12}R^{13}$, $-SO_2NHC(O)Ar^2$, $-C(O)C_{1-6}$alkyl, and $-C(O)Ar^2$;

$R^9$ is selected from the group consisting of: $-NR^{12}R^{13}$, $-NR^{12}C(O)R^{13}$, $-NR^{12}C(O)NHR^{13}$, $-NR^{12}SO_2Ar^2$, and $-NR^{12}CO_2Ar^2$;

$R^{10}$ and $R^{11}$ are each independently H, $-C_{1-6}$alkyl, $-C_{1-6}$haloalkyl, $-C_{3-6}$cycloalkyl, or $Ar^2$;

or when $R^{10}$ and $R^{11}$ are $-C_{1-6}$alkyl, they may connect together through a $C_{1-3}$alkyl and form a $C_{3-6}$cycloalkyl;

$R^{12}$ and $R^{13}$ are each independently H, $-C_{1-6}$alkyl, $-C_{3-6}$cycloalkyl, $-C_{1-6}$alkyl$Ar^2$;

or when $R^{12}$ and $R^{13}$ are $-C_{1-6}$alkyl, they may connect together through a $C_{1-3}$alkyl and form a $C_{3-6}$heterocycloalkyl;

$Ar^2$ is selected from the group consisting of: phenyl, pyridinyl, quinolinyl, isoquinolinyl, pyridazinyl, pyrimidinyl, pyrazinyl, quinoxalinyl, furyl, benzofuryl, dibenzofuryl, thienyl, benzothienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl, and tetrazolyl;

each $Ar^2$ may be unsubstituted or substituted with 1-3 members selected from the group consisting of: halo, $-C_{1-6}$alkyl, $-C_{1-6}$haloalkyl, CN, $C_{1-6}$alkoxy, $C_{1-6}$alkylthio, $-C(R^{10})(R^{11})OH$, $-CO_2H$, $-CO_2C_{1-6}$alkyl, $-C(O)NR^{12}R^{13}$, and $-SO_2CH_3$.

In one aspect of this invention, a compound of this invention is represented by Formula (**I**), or a pharmaceutically acceptable salt thereof, wherein

the *β*-D-glucuronide may be *β*-D-glucuronic acid, methyl *β*-D-glucuronidate, methyl 2,3,4-tri-*O*-acetyl-*β*-D-glucuronidate, ethyl *β*-D-glucuronidate, ethyl 2,3,4-tri-*O*-acetyl-*β*-D-glucuronidate, *i*-propyl *β*-D-glucuronidate, tert-butyl *β*-D-glucuronidate, methyl *β*-D-glucuronamide; the *β*-D-glucopyranoside may be *β*-D-glucopyranosyl, 2,3,4,6-tetra-*O*-acetyl-*β*-D-glucopyranosyl, 3,4,6-tri*O*-acetyl-*β*-D-glucopyranosyl; the *β*-D-galactopyranoside may be *β*-D-galactopyranosyl, 2,3,4,6-tetra-*O*-acetyl-*β*-D-galactopyranosyl; the *α*-D-mannopyranoside may be *α*-D-mannopyranosyl, 2,3,4,6-tetra-*O*-acetyl-*α*-o-mannopyranosyl; the *β*-D-glucosaminide may be *β*-D-glucosaminyl, *α*-D-glucosaminyl; the *N*-acetyl-*β*-D-glucosaminide may be *N*-acetyl-*β*-D-glucosaminyl, 3,4,6-tri-*O*-acetyl-*N*-acetyl-*β*-D-glucosaminyl, *N,N,N*-trimethyl-*β*-D-glucosaminyl, *N,N*-dimethyl-*β*-D-glucosaminyl; and the *β*-D-cellobioside may be *β*-D-cellobiosyl or 2,3,6,2',3',4',6'-hepta-*O*-acetyl-*β*-D-cellobiosyl;

$R^1$ and $R^2$ are each independently -$C_{1-6}$alkyl, -$C_{3-6}$cycloalkyl, any of which optionally substituted with 1-6 independent halogen;

$R^3$ and $R^4$ are each independently H, or-$C_{1-6}$alkyl;

$R^5$, $R^6$ and $R^7$ are each independently H, halogen, -$C_{1-6}$alkyl, or CN;

$Ar^1$ is independently selected from the group consisting of 6-$R^8$-3-pyridyl or 2-$R^8$-5-thiazolyl;

$R^8$ is selected from the group consisting of: H, halogen, -$C_{1-6}$alkyl, -$C_{3-6}$cycloalkyl, -$C_{1-6}$alkyl$Ar^2$, $Ar^2$, $C_{1-6}$alkoxy, $C_{1-6}$alkylthio, CN, -C($R^{10}$)($R^{11}$)OH, -C($R^{10}$)($R^{11}$)O$C_{1-6}$alkyl, - C($R^{10}$)($R^{11}$)O$Ar^2$, -$CO_2$H, -$CO_2C_{1-6}$alkyl, -C(O)N$R^{12}R^{13}$, -$SO_2$NHC(O)$Ar^2$, -C(O)$C_{1-6}$alkyl, and - C(O)$Ar^2$;

$R^9$ is selected from the group consisting of: -N$R^{12}R^{13}$, -N$R^{12}$C(O)$R^{13}$, - N$R^{12}$C(O)NH$R^{13}$, -N$R^{12}SO_2Ar^2$, and -N$R^{12}CO_2Ar^2$;

$R^{10}$ and $R^{11}$ are each independently H, -$C_{1-6}$alkyl, -$C_{1-6}$haloalkyl, -$C_{3-6}$cycloalkyl, or $Ar^2$;

or when $R^{10}$ and $R^{11}$ are -$C_{1-6}$alkyl, they may connect together through a $C_{1-3}$alkyl and form a $C_{3-6}$cycloalkyl;

$R^{12}$ and $R^{13}$ are each independently H, -$C_{1-6}$alkyl, -$C_{3-6}$cycloalkyl, -$C_{1-6}$alkyl$Ar^2$;

or when $R^{12}$ and $R^{13}$ are -$C_{1-6}$alkyl, they may connect together through a $C_{1-3}$alkyl and form a $C_{3-6}$heterocycloalkyl;

$Ar^2$ is selected from the group consisting of: phenyl, pyridinyl, quinolinyl, isoquinolinyl, pyridazinyl, pyrimidinyl, pyrazinyl, quinoxalinyl, furyl, benzofuryl, dibenzofuryl, thienyl, benzothienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl, and tetrazolyl;

each $Ar^2$ may be unsubstituted or substituted with 1-3 members selected from the group consisting of: halo, -$C_{1-6}$alkyl, -$C_{1-6}$haloalkyl, CN, $C_{1-6}$alkoxy, $C_{1-6}$alkylthio, -C($R^{10}$)($R^{11}$)OH, - $CO_2$H, -$CO_2C_{1-6}$alkyl, -C(O)N$R^{12}R^{13}$, and -$SO_2CH_3$.

In another embodiment of the present invention, the compounds of the present invention are represented by Formula (**I**), or a pharmaceutically acceptable salt thereof, wherein

$R^1$ and $R^2$ are each independently -$C_{1-6}$alkyl, or -$C_{3-6}$cycloalkyl, any of which is unsubstituted or substituted with 1-6 independent halogen;

$R^3$ and $R^4$ are each H;

$R^5$, $R^6$ and $R^7$ are each H;

$Ar^1$ is 6-$R^8$-3-pyridyl;

$R^8$ is selected from the group consisting of: -$C_{1-6}$alkyl$Ar^2$, -C($R^{10}$)($R^{11}$)OH, -C(O)$C_{1-6}$alkyl, and -C(O)$Ar^2$;

$R^{10}$ and $R^{11}$ are each independently H, -$C_{1-6}$alkyl, -$C_{1-6}$haloalkyl, -$C_{3-6}$cycloalkyl, or $Ar^2$;

$R^{12}$ and $R^{13}$ are each independently H, -$C_{1-6}$alkyl, -$C_{3-6}$cycloalkyl, or-$C_{1-6}$alkyl$Ar^2$;

or when $R^{12}$ and $R^{13}$ are -$C_{1-6}$alkyl, they may connect together through a $C_{1-3}$alkyl and form a $C_{3-6}$heterocycloalkyl;

$Ar^2$ is phenyl.

According to another embodiment the compounds of the present invention are represented by Formula (**I**), or a pharmaceutically acceptable salt thereof, wherein

X is methyl *β*-D-glucuronidate;

$R^1$ and $R^2$ are each independently-$C_{1-6}$alkyl, or -$C_{3-6}$cycloalkyl, any of which optionally substituted with 1-6 independent halogen;

$R^3$ and $R^4$ are each H;

$R^5$, $R^6$ and $R^7$ are each H;

$Ar^1$ is 6-$R^8$-3-pyridyl;

$R^8$ is -C($R^{10}$)($R^{11}$)OH;

$R^{10}$ and $R^{11}$ are each independently -$C_{1-6}$alkyl.

According to another embodiment of the compound of the present invention represented by Formula (**I**), or a pharmaceutically acceptable salt thereof, wherein the $\beta$-D-glucuronide is $\beta$-D-glucuronyl, preferably methyl glucuronate.

According to another embodiment of the compounds of the present invention represented by Formula (**I**), or a pharmaceutically acceptable salt thereof, the compound is one of the following compounds:

| Cmpd | Structure | Cmpd | Structure |
|---|---|---|---|
| 1 | 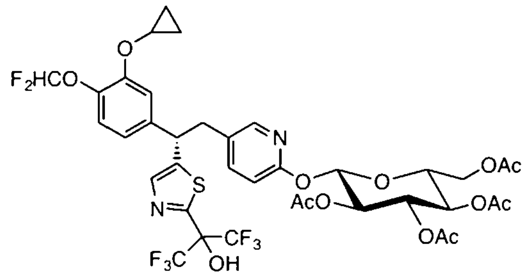 | 17 | 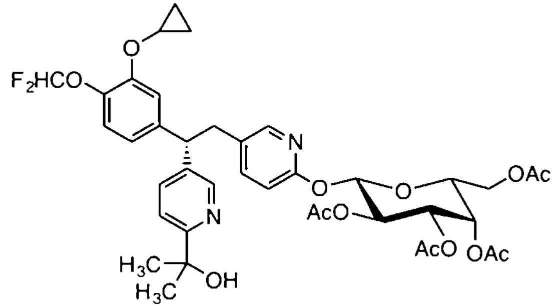 |
| 2 | 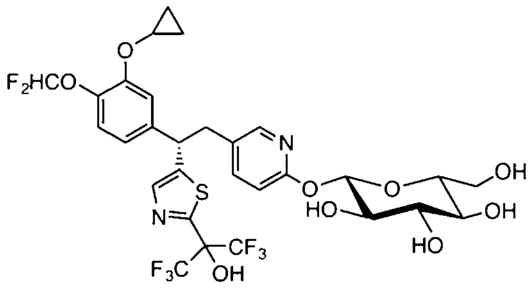 | 18 | 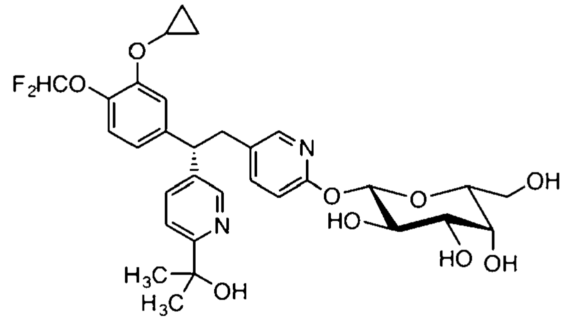 |
| 3 | 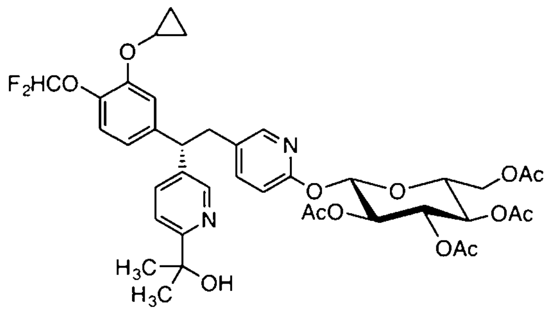 | 19 | 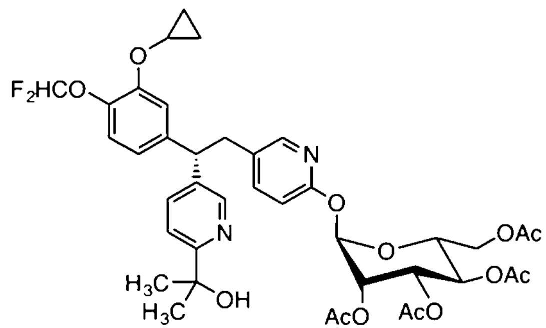 |

(continued)

| Cmpd | Structure | Cmpd | Structure |
|---|---|---|---|
| 4 | 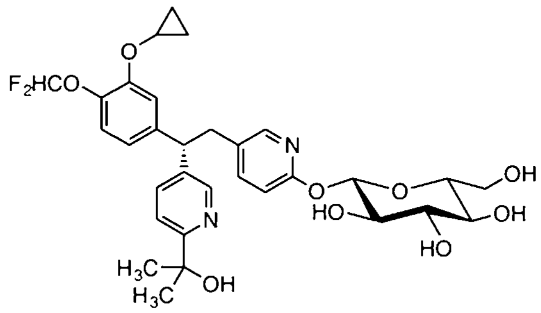 | 20 | 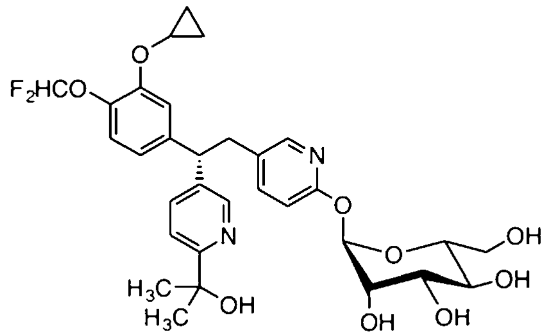 |
| 5 | 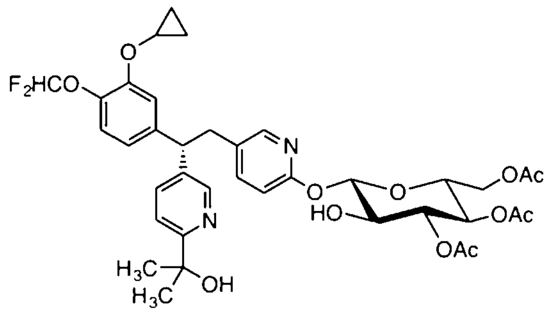 | 21 | 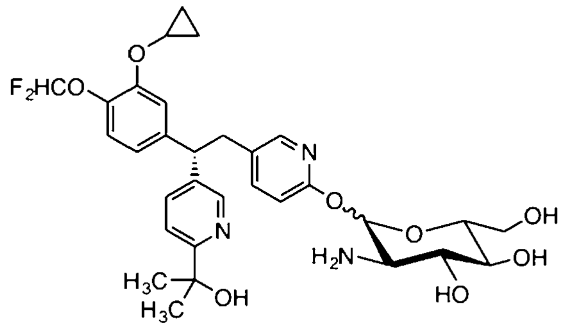 |
| 6 | 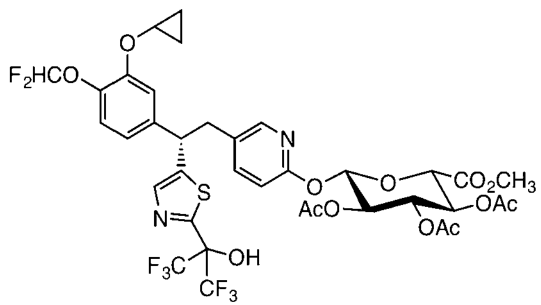 | 22 | 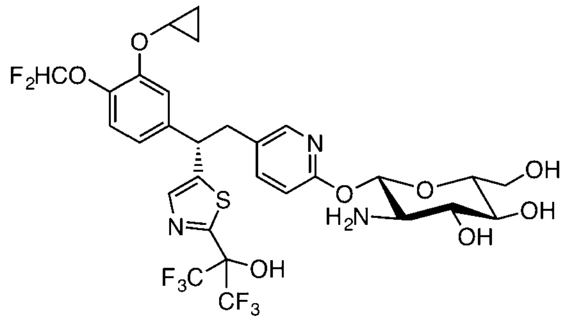 |

(continued)

| Cmpd | Structure | Cmpd | Structure |
|---|---|---|---|
| 7 | 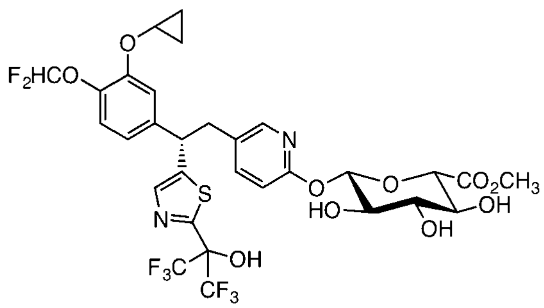 | 23 | 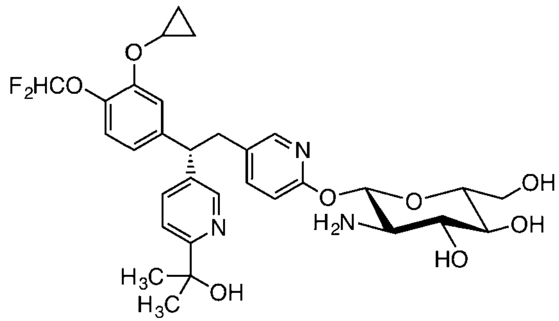 |
| 8 | 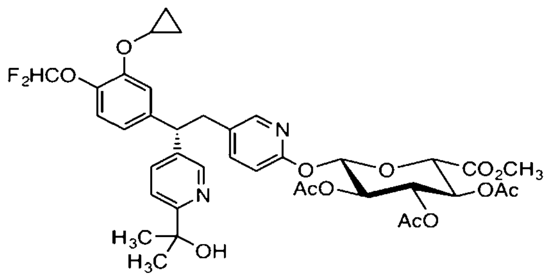 | 24 | 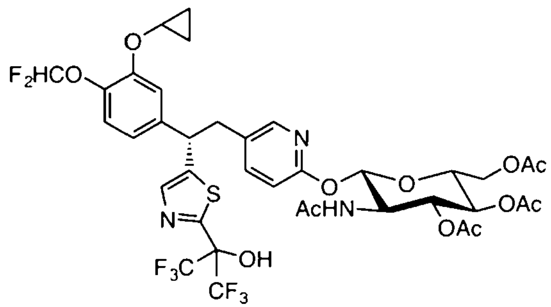 |
| 9 | 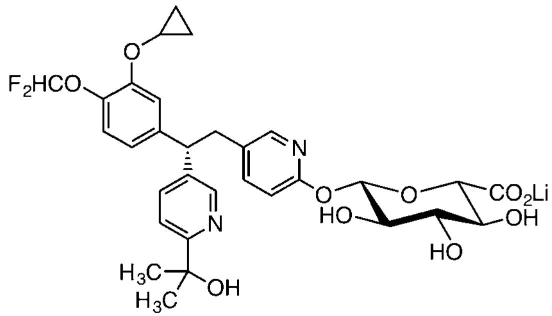 | 25 | 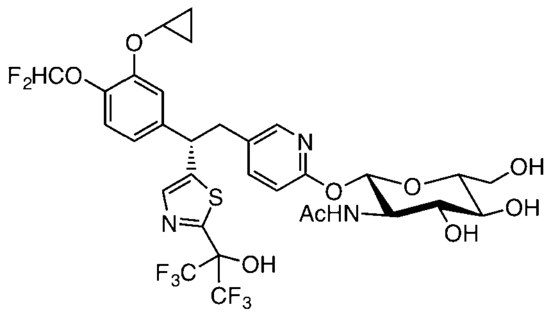 |

(continued)

| Cmpd | Structure | Cmpd | Structure |
|---|---|---|---|
| 10 | 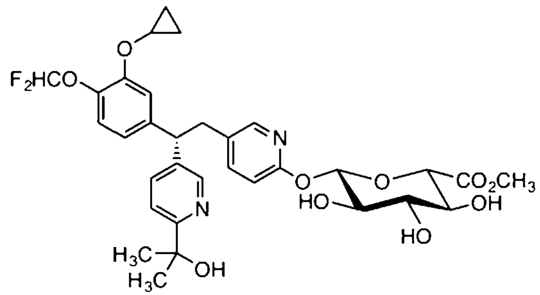 | 26 | 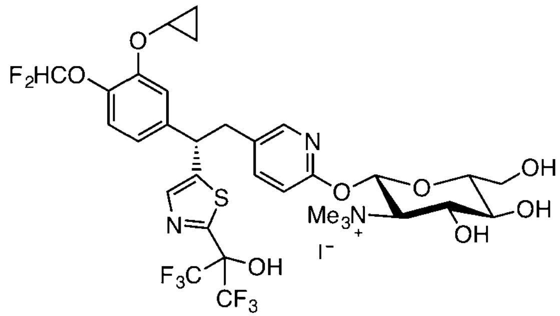 |
| 11 | 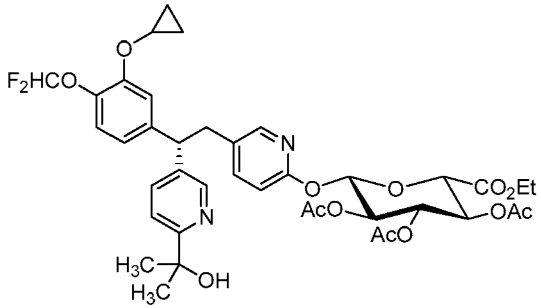 | 27 | 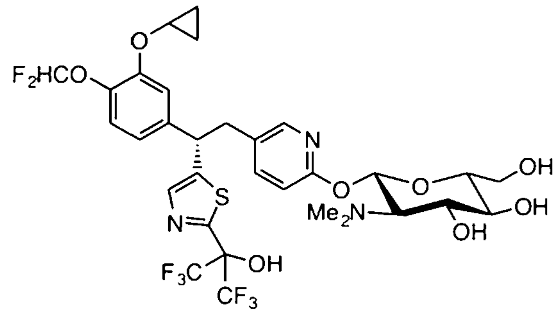 |
| 12 | 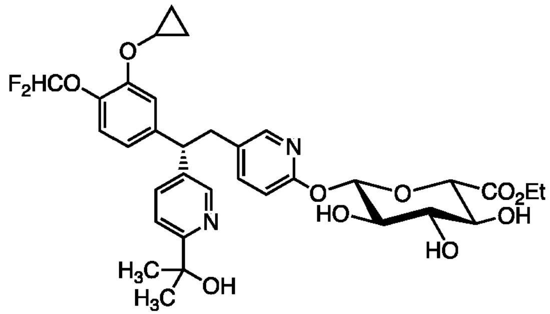 | 28 | 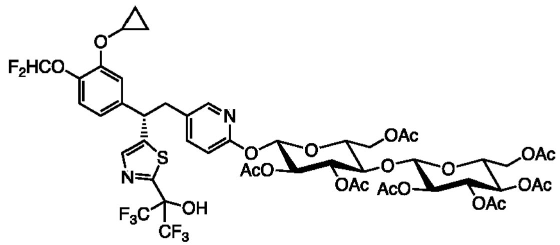 |

(continued)

| Cmpd | Structure | Cmpd | Structure |
|---|---|---|---|
| 13 | 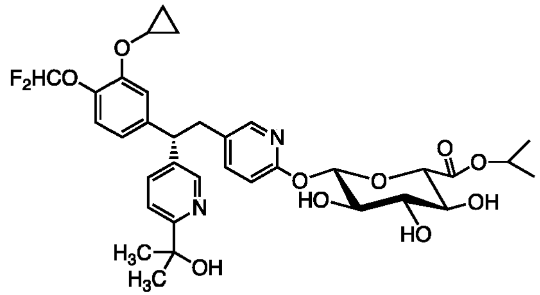 | 29 | 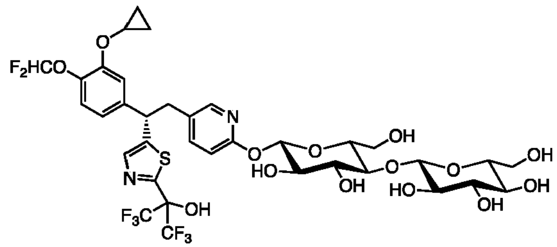 |
| 14 | 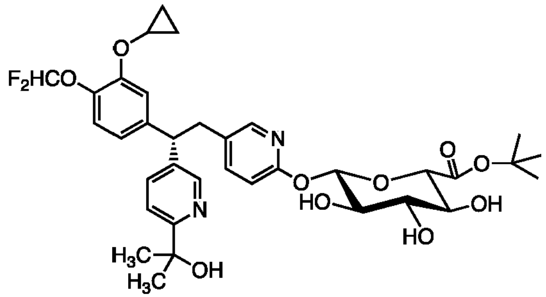 | 30 | 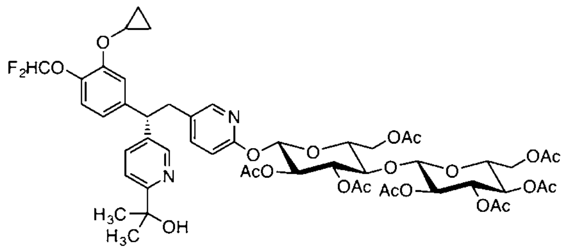 |
| 15 | 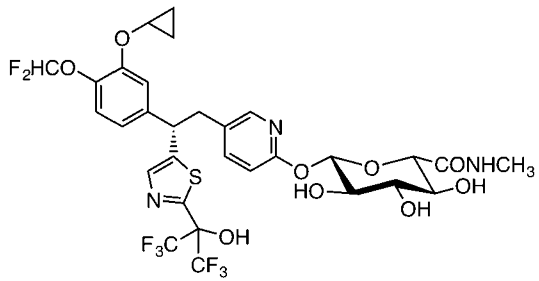 | 31 | 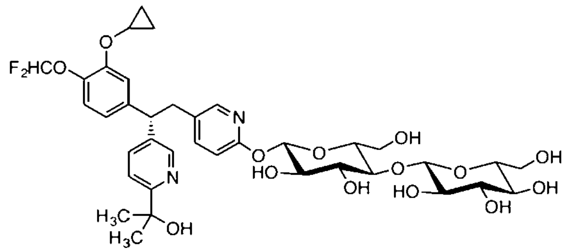 |

(continued)

| Cmpd | Structure | Cmpd | Structure |
|---|---|---|---|
| 16 | 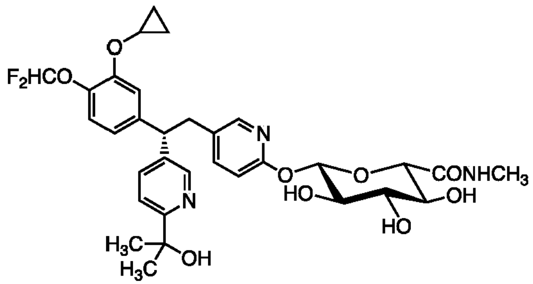 | | |

or a pharmaceutically acceptable salt thereof.

According to another embodiment the compound of the present invention represented by Formula (**I**), or a pharmaceutically acceptable salt thereof may be

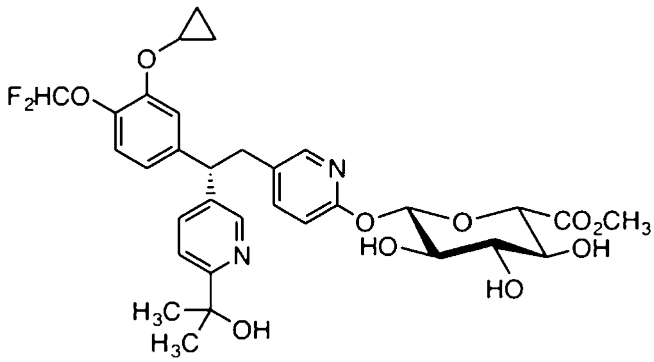

or a pharmaceutically acceptable salt thereof.

According to another embodiment the compound of the present invention represented by Formula (**I**), or a pharmaceutically acceptable salt thereof may be

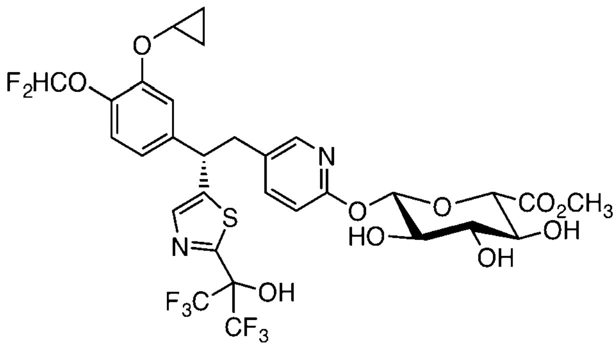

or a pharmaceutically acceptable salt thereof.

According to another embodiment the compound of the present invention represented by Formula (**I**), or a pharmaceutically acceptable salt thereof may be

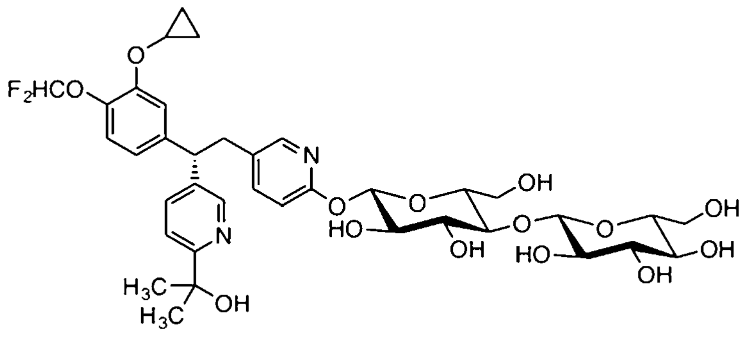

or a pharmaceutically acceptable salt thereof.

Orally administered inactive PDE4 inhibitor glycosides are believed to bypass the triggers of emesis of the upper gastrointestinal tract and reduce the emetogenic potential of such a drug. The enhanced hydrophilic character of the glycoside prodrug compared to the parent PDE4 inhibitor may improve the ability of the said delivery system to uniformly distribute and release the active principle in the entire colon where drug dissolution is restricted by lower water content, irregular motility and the lack of bile salts.

PDE4 inhibitor glycosides delivered to the colon are believed to be enzymatically hydrolyzed by the colon specific glycosidases to release the biologically active di-aryl-substituted-ethane pyridone PDE4 inhibitors, to exert a

local anti-inflammatory effect on the colon mucosa. While this system has never been used in human, in embodiments, it is believed that it could advantageously represent a way to slowly release an active agent specifically to the colon by properly selecting the glycoside/glycosidase system and the administered dose to minimize systemic exposure.

PDE4 inhibitor glycosides releasing the biologically active di-aryl-substituted-ethane pyridone PDE4 inhibitors specifically in the colon are believed to act as a systemic controlled release system by properly selecting the administered dose. In contrast with the 2-5 hr small intestine transit, the colon residence time is significantly longer with transit time such as 5-12 hr, 12-24 hr, 24-36 hr and 36-72 hr. This would allow a slow and sustained absorption of the active principle, to prolong effect duration while minimizing peak plasma concentration that would trigger undesired adverse effects.

As used herein, "alkyl" as well as other groups having the prefix "alk" such as, for example, alkoxy, alkanoyl, alkenyl, alkynyl and the like, means carbon chains which may be linear or branched or combinations thereof. Examples of alkyl groups include methyl, ethyl, propyl, isopropyl, butyl, sec- and tert-butyl, pentyl, hexyl, heptyl and the like. "Alkenyl", "alkynyl" and other like terms include carbon chains containing at least one unsaturated C-C bond.

The term "haloalkyl" refers to an alkyl group having 1-9 halo groups attached. Examples include $-CH_2F$, $-CHF_2$, $-CF_3$, $-CH_2CH_2F$, $-CHFCH_2F$, $-CF_2CH_2F$, $-CF_2CHF_2$ and $-CF_2CF_3$.

The term "cycloalkyl" means carbocycles containing no heteroatoms, and includes mono-, bi- and tricyclic saturated carbocycles, as well as fused ring systems. Such fused ring systems can include one ring that is partially or fully unsaturated such as a benzene ring to form fused ring systems such as benzofused carbocycles. Cycloalkyl includes such fused ring systems as spirofused ring systems. Examples of cycloalkyl include cyclopropyl, cyclobutyl, cyclopentyl, cyclohexyl, decahydronaphthalenyl, adamantanyl, indanyl, indenyl, fluorenyl, 1,2,3,4-tetrahydronaphthalenyl and the like. Similarly, "cycloalkenyl" means carbocycles containing no heteroatoms and at least one nonaromatic C-C double bond, and include mono-, bi- and tricyclic partially saturated carbocycles, as well as benzofused cycloalkenes. Examples of cycloalkenyl include cyclohexenyl, indenyl, and the like.

The term "cycloalkyloxy" unless specifically stated otherwise includes a cycloalkyl group connected to the oxy connecting atom.

The term "alkoxy" unless specifically stated otherwise includes an alkyl group connected to the oxy connecting atom.

The term "aryl" unless specifically stated otherwise includes multiple ring systems as well as single ring systems such as, for example, phenyl or naphthyl.

The term "aryloxy" unless specifically stated otherwise includes multiple ring systems as well as single ring systems such as, for example, phenyl or naphthyl, connected through the oxy connecting atom to the connecting site.

The term "$C_0$-$C_6$alkyl" includes alkyls containing 6, 5, 4, 3, 2, 1, or no carbon atoms. An alkyl with no carbon atoms is a hydrogen atom substituent when the alkyl is a terminus moiety. An alkyl with no carbon atoms is a direct bond when the alkyl is a bridging moiety.

The term "hetero" unless specifically stated otherwise includes one or more O, S, or N atoms. For example, heterocycloalkyl and heteroaryl include ring systems that contain one or more O, S, or N atoms in the ring, including mixtures of such atoms. The heteroatoms replace ring carbon atoms. Thus, for example, a heterocyclo$C_5$alkyl is a five membered ring containing from 5 to no carbon atoms. Examples of heteroaryl include, pyridinyl, quinolinyl, isoquinolinyl, pyridazinyl, pyrimidinyl, pyrazinyl, quinoxalinyl, furyl, benzofuryl, dibenzofuryl, thienyl, benzothienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl, tetrazolyl.

The term "heteroaryloxy" unless specifically stated otherwise describes a heteroaryl group connected through an oxy connecting atom to the connecting site. Examples of heteroaryl($C_{1-6}$)alkyl include, for example, furylmethyl, furylethyl, thienylmethyl, thienylethyl, pyrazolylmethyl, oxazolylmethyl, oxazolylethyl, isoxazolylmethyl, thiazolylmethyl, thiazolylethyl, imidazolylmethyl, imidazolylethyl, benzimidazolylmethyl, oxadiazolylmethyl, oxadiazolylethyl, thiadiazolylmethyl, thiadiazolylethyl, triazolylmethyl, triazolylethyl, tetrazolylmethyl, tetrazolylethyl, pyridinylmethyl, pyridinylethyl, pyridazinylmethyl, pyrimidinylmethyl, pyrazinylmethyl, quinolinylmethyl, isoquinolinylmethyl and quinoxalinylmethyl. Examples of heterocyclo$C_{3-7}$alkyl include, for example, azetidinyl, pyrrolidinyl, piperidinyl, perhydroazepinyl, piperazinyl, morpholinyl, tetrahydrofuranyl, imidazolinyl, pyrolidin-2-one, piperidin-2-one, and thiomorpholinyl.

The term "N-heterocyclo$C_{4-7}$alkyl" describes nonaryl heterocyclic compounds having 3-6 carbon atoms and one nitrogen atom forming the ring. Examples include azetidinyl, pyrrolidinyl, piperidinyl, and perhydroazepinyl. Examples of aryl($C_{1-6}$)alkyl include, for example, phenyl($C_{1-6}$)alkyl, and naphthyl($C_{1-6}$)alkyl. Examples of heterocyclo$C_{3-}$ alkylcarbonyl($C_{1-6}$)alkyl include, for example, azetidinyl carbonyl($C_{1-6}$)alkyl, pyrrolidinyl carbonyl($C_{1-6}$)alkyl, piperidinyl carbonyl($C_{1-6}$)alkyl, piperazinyl carbonyl($C_{1-6}$)alkyl, morpholinyl carbonyl($C_{1-6}$)alkyl, and thiomorpholinyl carbonyl($C_{1-6}$)alkyl.

The term "amine" unless specifically stated otherwise includes primary, secondary and tertiary amines.

Unless otherwise stated, the term "carbamoyl" is used to include $-NHC(O)OC_1$-$C_4$alkyl, and $-OC(O)NHC_1$-$C_4$alkyl.

The term "halogen" includes fluorine, chlorine, bromine and iodine atoms.

The term "optionally substituted" is intended to include both substituted and unsubstituted. Thus, for example,

optionally substituted aryl could represent a pentafluorophenyl or a phenyl ring. Further, the substitution can be made at any of the groups. For example, substituted aryl($C_{1-6}$)alkyl includes substitution on the aryl group as well as substitution on the alkyl group.

The term "oxide" of heteroaryl groups is used in the ordinary well-known chemical sense and include, for example, N-oxides of nitrogen heteroatoms.

Compounds described herein contain one or more double bonds and may thus give rise to cis/trans isomers as well as other conformational isomers. The present invention includes all such possible isomers as well as mixtures of such isomers.

Compounds described herein can contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof. The above Formula (**I**) is shown without a definitive stereochemistry at certain positions. The present invention includes all stereoisomers of Formula (**I**) and pharmaceutically acceptable salts thereof. Further, mixtures of stereoisomers as well as isolated specific stereoisomers are also included.

During the course of the synthetic procedures used to prepare such compounds, or in using racemization or epimerization procedures known to those skilled in the art, the products of such procedures can be mixtures of stereoisomers.

The term "pharmaceutically acceptable salts" refers to salts prepared from pharmaceutically acceptable non-toxic bases or acids or co-crystal formers. The crystalline form can exist as salt, solvate, hydrate, or clathrate. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases or co-crystals from which salts or co-crystals can be formed include ion exchange resins such as, for example, arginine, betaine, caffeine, choline, N,N -dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the likes.

When the compound of the present invention is basic, its corresponding salt or co-crystals can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particularly preferred are benzenesulfonic, citric, hydrobromic, hydrochloric, maleic, phosphoric, sulfuric, and tartaric acids.

Structural modification such as removing or substituting hydroxyl or carboxyl group of naturally occurring glycosides has an impact on the binding interaction between the glycosidase and the substrate glycone. This may result in change in kinetic of the enzyme hydrolytic process that could be advantageously utilized to either accelerate or slow down the rate at which the parent PDE4 aglycone will be released in the large intestine.

According to another embodiment, pharmaceutical compositions comprising a compound represented by Formula (**I**) (or pharmaceutically acceptable salts or co-crystals thereof) as an active ingredient, and a pharmaceutically acceptable carrier and optionally other therapeutic ingredients or adjuvants may be prepared. Such additional therapeutic ingredients include, for example, i) Leukotriene receptor antagonists, ii) Leukotriene biosynthesis inhibitors, iii) corticosteroids, iv) HI receptor antagonists, v) $\beta_2$ adrenoceptor agonists, vi) COX-2 selective inhibitors, vii) statins, viii) nonsteroidal anti-inflammatory drugs ("NSAID"), ix) M2/M3 antagonists x) 5-ASA and 5-ASA prodrugs, xi) azathioprine, xii) cyclosporine and xiii) methotrexate. The compositions include compositions suitable for oral, aerosol (including inhalers, intranasal spray), rectal, topical(including mucosal, ocular, buccal , aural, transdermal or transcutaneous), and parenteral (including ocular, subcutaneous, intramuscular, intraarterial and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy. Non-limiting examples of dosage forms include tablets; caplets; capsules such as soft elastic gelatin capsules, HPMC or any common hard gelatin capsule (including any chemically modified capsules for target delivery), cachets, troches, lozenges; dispersions; or any 3-D printed solid dosage forms, oromucosal films, suppositories; powders; aerosols (including pMDI or similar pulmonary delivery systems); gels; liquid dosage forms suitable for oral or mucosal administration to a patient, including suspensions (e.g., aqueous or non-aqueous liquid suspension, oil in water emulsions or water in oil liquid emulsions), solution, elixirs, liquid

dosage forms suitable for parenteral administration to a patient; eye drops or other ophthalmic preparations, transdermal or transcutaneous preparation in ointment, lotion, creams suitable for topical administration directly or via intradermal devices or injections or needleless or needle-free device or microneedle patches; Mouth washes and gargles are included within the scope of topical use for the purposes of this invention, and sterile solids (e.g., crystalline or amorphous solids) that can be reconstituted to provide liquid dosage forms suitable for parenteral administration to a patient; formulations that might delay release of the active ingredient or control release of the active ingredient as this is delivered in the GIT are also considered.

According to another embodiment, the pharmaceutical compositions of this invention may include a pharmaceutically acceptable carrier/excipients, a compound or a pharmaceutically acceptable salt/co-crystal of Formula (**I**) and the corresponding parent PDE4 inhibitor of the compound of Formula (I).

Colonic absorption of drug to systemic circulation is mainly determined by its permeability and by its solubility (Tannergren et al. Mol. Pharmacol. 2009, 6(1), 60). Accordingly, a fraction of the di-aryl-substituted-ethane pyridone PDE4 inhibitor absorbed by the colon mucosa may reach the blood stream to be absorbed systemically. Therefore, according to another embodiment, a dose that exerts a local anti-inflammatory effect and minimizes the systemic exposure to the active agent is desired to reduce the potential for headache, nausea, emesis and diarrhea reported for drug such as rolipram, cilomilast, roflumilast and apremilast.

According to another embodiment, colonic absorption of the di-aryl-substituted-ethane pyridone PDE4 inhibitor to systemic circulation may be desired if pre-clinical or clinical evidences are suggesting that it is better tolerated than known drug of the same class such as cilomilast, roflumilast and apremilast. Bypassing the local triggers of emesis of the upper gastrointestinal tract by the inactive PDE4 inhibitor glycoside is desirable and considered as a benefit to improve tolerability. Slow absorption associated with long transit time in the large intestine is also considered as a benefit since it reduces the value of the maximal peak plasma concentration that may trigger adverse effect such as headache, nausea, emesis or diarrhea.

According to another embodiment, systemic exposition to di-aryl-substituted-ethane pyridone PDE4 inhibitors through colonic absorption may be indicated for the treatment of inflammatory diseases including ulcerative colitis.

Dosage levels from about 0.0001 mg/kg to about 100 mg/kg, or about 0.001 mg/kg to about 100 mg/kg, or about 0.01 mg/kg to about 100 mg/kg, or about 0.1 mg/kg to about 100 mg/kg, or about 1 mg/kg to about 100 mg/kg, or about 10 mg/kg to about 100 mg/kg, or about 0.0001 mg/kg to about 10 mg/kg, or about 0.001 mg/kg to about 10 mg/kg, or about 0.01 mg/kg to about 10 mg/kg, or about 0.1 mg/kg to about 10 mg/kg, or about 1 mg/kg to about 10 mg/kg, or about 0.0001 mg/kg to about 1 mg/kg, or about 0.001 mg/kg to about 1 mg/kg, or about 0.01 mg/kg to about 1 mg/kg, or about 0.1 mg/kg to about 1 mg/kg, about 0.0001 mg/kg to about 0.1 mg/kg, or about 0.001 mg/kg to about 0.1 mg/kg, or about 0.01 mg/kg to about 0.1 mg/kg, about 0.0001 mg/kg to about 0.01 mg/kg, or about 0.001 mg/kg to about 0.01 mg/kg, about 0.0001 mg/kg to about 0.001 mg/kg of body weight per day may be useful in the treatment of conditions such as i) Pulmonary disorders such as asthma, chronic bronchitis, chronic obstructive pulmonary disease (COPD), adult respiratory distress syndrome, infant respiratory distress syndrome, cough, chronic obstructive pulmonary disease in animals, and ii) Gastrointestinal disorders such as ulcerative colitis, Crohn's disease, hypersecretion of gastric acid, diverticulitis and irritable bowel syndrome, iii) Infectious diseases such as bacterial, fungal or viral induced sepsis or septic shock, endotoxic shock (and associated conditions such as laminitis and colic in horses), and septic shock, iv) Neurological disorders such as spinal cord trauma, head injury, neurogenic inflammation, pain, and reperfusion injury of the brain, v) Inflammatory disorders such as psoriatic arthritis, rheumatoid arthritis, ankylosing spondylitis, osteoarthritis, inflammation and cytokine-mediated chronic tissue degeneration, vi) Allergic disorders such as allergic rhinitis, allergic conjunctivitis, and eosinophilic granuloma, vii) Psychiatric disorders such as depression, memory impairment, and monopolar depression, viii) Neurodegenerative disorders such as Parkinson disease, Alzheimer's disease, acute and chronic multiple sclerosis, ix) Dermatological disorders such as psoriasis and other benign or malignant proliferative skin diseases, atopic dermatitis, and urticaria, x) Oncological diseases such as cancer, tumor growth and cancerous invasion of normal tissues, xi) Metabolic disorders such as diabetes insipidus, xii) Bone disorders such as osteoporosis, xiii) Cardiovascular disorders such as arterial restenosis, atherosclerosis, reperfusion injury of the myocardium, and xiv) Other disorders such as chronic glomerulonephritis, vernal conjunctivitis, transplant rejection and graft versus host disease, and cachexia - which are responsive to PDE4 inhibition, or alternatively about 0.007 mg to about 7 g, or about 0.07 mg to about 7 g, or about 0.7 mg to about 7 g, or about 0.007 mg to about 0.7 g, or about 0.07 mg to about 0.7 g, or about 0.007 mg to about 0.07 g per patient per day. For example, inflammation may be effectively treated by the administration of from about 0.0001 mg to 100 mg, or about 0.001 mg to 100 mg, or about 0.01 mg to 100 mg, or about 0.1 mg to 100 mg, or about 1 mg to 100 mg, or about 10 mg to 100 mg, or about 0.0001 mg to 10 mg, or about 0.001 mg to 10 mg, or about 0.01 mg to 10 mg, or about 0.1 mg to 10 mg, or about 1 mg to 10 mg, or about 0.0001 mg to 1 mg, or about 0.001 mg to 1 mg, or about 0.01 mg to 1 mg, or about 0.1 mg to 1 mg, or about 0.0001 mg to 0.1 mg, or about 0.001 mg to 0.1 mg, or about 0.01 mg to 10. mg, or about 0.0001 mg to 0.01 mg, or about 0.001 mg to 0.01 mg, or about 0.0001 mg to 0.001 mg of the compound per kilogram of body weight per day, or alternatively about 0.007 mg to about 7 g, or about 0.07 mg to about 7 g, or about 0.7 mg to about 7 g, or about 0.007 mg to about 0.7 g, or about

0.07 mg to about 0.7 g, or about 0.007 mg to about 0.07 g per patient per day. Further, it is understood that the PDE4 inhibitor glycoside prodrug compounds of this invention may be administered at prophylactically effective dosage levels to prevent the above-specified conditions.

The amount of active ingredient that may be combined with the carrier materials to produce a single dosage form will vary depending upon the treated target and the particular mode of administration. For example, a formulation intended for the oral administration to humans may conveniently contain from about 0.5 mg to about 5 g, or about 0.5 mg to about 500 mg, or about 0.5 mg to about 50 mg, or about 0.5 mg to about 5 mg, or about 5 mg to about 5 g, or about 5 mg to about 500 mg, or about 5 mg to about 50 mg, or about 50 mg to about 5 g, or about 50 mg to about 500 mg, or about 500 mg to about 5 g of active agent, formulated with an appropriate and acceptable amount of generally recognized as safe ("GRAS") materials which may vary from about 5 to about 95 percent of the total composition. Unit dosage forms will generally contain between about 0.001 mg to about 5000 mg, or about 0.01 mg to about 5000 mg, or about 0.1 mg to about 5000 mg, or about 1 mg to about 5000 mg, or about 10 mg to about 5000 mg, or about 100 mg to about 5000 mg, or about 1000 mg to about 5000 mg, or about 0.001 mg to about 1000 mg, or about 0.01 mg to about 1000 mg, or about 0.1 mg to about 1000 mg, or about 1 mg to about 1000 mg, or about 10 mg to about 1000 mg, or about 100 mg to about 1000 mg, or about 0.001 mg to about 100 mg, or about 0.01 mg to about 100 mg, or about 0.1 mg to about 100 mg, or about 1 mg to about 100 mg, or about 10 mg to about 100 mg, or about 0.001 mg to about 10 mg, or about 0.01 mg to about 10 mg, or about 0.1 mg to about 10 mg, or about 1 mg to about 10 mg, or about 0.001 mg to about 1 mg, or about 0.01 mg to about 1 mg, or about 0.1 mg to about 1 mg, or about 0.001 mg to about 0.1 mg, or about 0.01 mg to about 0.1 mg, or about 0.001 mg to about 0.01 mg of the active ingredient, typically 0.001 mg, 0.005 mg, 0.025 mg, 0.1 mg, 0.5 mg, 2.5 mg, 5.0 mg, 10 mg, 30 mg, 60 mg, 100 mg, 300 mg, 600 mg, 1000 mg, 3000 mg, 5000 mg or any dose in-between.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

The composition, shape and type of dosage forms provided herein will typically vary depending on their use. For example, a dosage form used in the acute treatment of a disease may contain large amounts of one or more of the active ingredients including Formula (I) it comprises than a dosage form used in the chronic treatment of the same disease. Similarly, a parenteral dosage form may contain smaller amounts of one or more of the active ingredients including Formula (I) it comprises than an oral dosage form used to treat the same disease. These and other ways in which specific dosage forms provided herein will vary from one another will be readily apparent to those skilled in the art. See e.g., Remington's Pharmaceutical Sciences, 20th ed., Mack Publishing, Easton, Pa (2000). In practice, the compounds represented by Formula (I), or pharmaceutically acceptable salts/co-crystals thereof, of this invention can be combined as the active ingredient in intimate admixture with a pharmaceutical excipients, carrier, or diluents according to conventional pharmaceutical compounding techniques. The carrier may take a wide variety of forms depending on the form of preparation desired for administration, e.g., oral, mucosal (e.g., nasal, sublingual, vaginal, inhalational, cystic, rectal, ocular, buccal or aural), parenteral (including intravenous, intradermal, subcutaneous, bolus injection, intramuscular or intraarterial) or topical (e.g., transdermal, transcutaneous, eye drops or other ophthalmic preparations). Thus, the pharmaceutical compositions of the present invention can be presented as discrete units suitable for oral administration such as capsules (coated or non-coated with polymers as sustained release or enteric coated or modified for target delivery), sachets or tablets (coated or uncoated or bilayers or sustained release or delayed release including micro-encapsulation) or tablets containing spray dried intermediates each containing a predetermined amount of the active ingredient. Further, the compositions can be presented as a powder, as granules, as a coated sustained release particles, as a solution, as a suspension in an aqueous liquid, as a non-aqueous liquid, as an oil-in-water emulsion or as a water-in-oil liquid emulsion, liposomes, nanosuspension. In addition to the common dosage forms set out above, the compound represented by Formula (I), or pharmaceutically acceptable salts or co-crystals thereof, may also be administered by controlled or modified release formulation and/or delivery devices. The compositions may be prepared by any of the methods of pharmacy. In general, such methods include a step of bringing into association the active ingredient with the excipients or carriers that constitutes one or more necessary ingredients. In general, the compositions are prepared by uniformly and intimately admixing the active ingredient with liquid carriers /excipients or finely divided solid carriers /excipients or both. The product can then be conveniently shaped into the desired presentation.

According to another embodiment, it is known that glycosidic linkage can be hydrolyzed under acidic conditions. Therefore, as part of the compound formulation of the present invention, the PDE4 glycoside prodrug may comprise excipient or coating to prevent premature hydrolysis in the stomach or any other part of the gastrointestinal tract where the pH is inferior to 5.

Thus, the pharmaceutical compositions of this invention may include a pharmaceutically acceptable carrier/excipients and a compound or a pharmaceutically acceptable salt/co-crystal of Formula (I). The compounds of Formula **(I),** or pharmaceutically acceptable salts/co-crystals thereof, can also be included in pharmaceutical compositions in combination with one or more other therapeutically active compounds.

The pharmaceutical carrier employed can be, for example, to form oral solid preparations such as powders, capsules and tablets include fillers such as talc, calcium carbonate, microcrystalline cellulose, kaolin, mannitol, silicic acid, sorbitol, starch, and mixture thereof. Binder such as Kollidon. Disintegrants such as croscarmellose sodium, crospovidone, sodium starch glycolate, pre-gelatinized starch, gums and other starches and mixtures thereof. Lubricants such as calcium stearate, magnesium stearate, syloid silica gel, mineral oil, glycerine, sorbitol, mannitol, polyethylene glycol, stearic acid, sodium lauryl sulphate, talc, hydrogenated vegetable oil (e.g., peanut oil, sesame oil, cor oil or soybean oil), ethyl oleate agar or other lipid formulation lubricants and mixtures thereof.. Because of their ease of administration, tablets and capsules are the preferred oral dosage units whereby solid pharmaceutical carriers are employed. Each of the solid oral dosage units can be further coated with specialized polymers that can delay release or sustained release the contents of the dosage units. Formula (I) can be administered by delayed release or sustained release means or by delivery devices that are well known to those of ordinary skill in the art. Non-limiting examples of delayed release or sustained release include those described in U.S. Patent Nos. 3,845,770; 3 916,899; 3, 536, 809; 5, 059,595. Such dosage forms can be used to provide slow or controlled release of one or more ingredients using for example polymers such as hydroxylpropylmethyl cellulose usually in a matrix form such as gel, permeable membranes, micro-emulsions, osmotic systems, liposomes, microspheres or combinations thereof. Controlled release formulation can be used to protect the dosage units from exposure to the gastric environment; delay release of active ingredient to the lower gastrointestinal tract such as the colon; or slow the release of the active ingredient such that blood levels of the drug can be lowered and affect the occurrence of side effects.

Examples of gaseous carriers include carbon dioxide and nitrogen.

In preparing the oral liquid compositions for oral dosage form, any convenient pharmaceutical media may be employed. For example, water, glycols, oils, alcohols, flavoring agents, preservatives, coloring agents and the like may be used to form oral liquid preparations such as suspensions, elixirs and solutions;

A tablet containing the composition of this invention may be prepared by compression or molding, optionally with one or more accessory ingredients or adjuvants.

Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.001 mg to about 5000 mg, or about 0.01 mg to about 5000 mg, or about 0.1 mg to about 5000 mg, or about 1 mg to about 5000 mg, or about 10 mg to about 5000 mg, or about 100 mg to about 5000 mg, or about 1000 mg to about 5000 mg, or about 0.001 mg to about 1000 mg, or about 0.01 mg to about 1000 mg, or about 0.1 mg to about 1000 mg, or about 1 mg to about 1000 mg, or about 10 mg to about 1000 mg, or about 100 mg to about 1000 mg, or about 0.001 mg to about 100 mg, or about 0.01 mg to about 100 mg, or about 0.1 mg to about 100 mg, or about 1 mg to about 100 mg, or about 10 mg to about 100 mg, or about 0.001 mg to about 10 mg, or about 0.01 mg to about 10 mg, or about 0.1 mg to about 10 mg, or about 1 mg to about 10 mg, or about 0.001 mg to about 1 mg, or about 0.01 mg to about 1 mg, or about 0.1 mg to about 1 mg, or about 0.001 mg to about 0.1 mg, or about 0.01 mg to about 0.1 mg, or about 0.001 mg to about 0.01 mg of the active ingredient and each cachet or capsule preferably containing from about 0.001 mg to about 5000 mg of the active ingredient.

Pharmaceutical compositions of the present invention suitable for parenteral administration (including intravenous, intramuscular, subcutaneous, ocular, and intraarterial) may be prepared as solutions or suspensions of the active compounds in injectable ingredients. Parenteral dosage forms are preferably sterile or capable of being sterilized prior to administration to a patient. Non-limiting examples of suitable vehicles include Water for Injection USP; Dextrose Injection; Sodium Chloride Injection and lactated Ringer's Injection. A suitable surfactant can be included such as, for example, polysorbate 80. Dispersions can also be prepared in glycerol, liquid polyethylene glycols, ethyl alcohol, polypropylene glycol and mixtures thereof in non-aqueous vehicles such as oils (e.g., corn oil, sesame oil, isopropyl myristate). An anti-oxidant to help stabilize the formulation such as Vit C palmitate. Further, a preservative can be included to prevent the detrimental growth of microorganisms.

Furthermore, the compositions can be in the form of sterile powders for the extemporaneous preparation of such sterile injectable solutions or dispersions. In all cases, the final injectable form must be sterile, non-irritating with addition of tonicity agents and must be effectively fluid for easy syringeability. The pharmaceutical compositions must be stable under the conditions of manufacture and storage; thus, preferably should be preserved against the contaminating action of microorganisms such as bacteria and fungi such as benzalkonium chloride, chlorobutanol, methyl aparaben, propyl paraben, edetate disodium, sorbic acid or other agents known to those skilled in the art. Pharmaceutical compositions of the present invention can be in a form suitable for topical applied locally to the skin and its adnexa or to a variety of mucous membranes such as, for example, an aerosol, patch, cream, ointment, lotion, dusting powder, emulsions or the like. The routes that can be used include nasal, sublingual, vaginal, rectal, ocular, buccal or aural. Further, the compositions can be in a form suitable for use in transdermal or intradermal micro-needle devices. These formulations may be prepared, utilizing a compound represented by Formula (I) of this invention, or pharmaceutically acceptable salts thereof, via conventional processing methods. As an example, a lotion, cream or ointment is prepared

by mixing hydrophilic material and water, together with about 5wt% to about 30wt% of the compound, to produce a cream, lotion or ointment having a desired consistency. Examples of typical excipients include water, acetone, ethanol, ethylene glycol, propylene glycol, isopropyl myristate, mineral oil and mixtures thereof. Moisturizers such as occlusive, humectant, emollients can also be added to the pharmaceutical compositions and dosage forms if desired. pH of a pharmaceutical composition or dosage form may also be adjusted to improve delivery of Formula (I). Dosage forms suitable for treating mucosal tissues within the oral cavity can be formulated as mouthwashes or as oral gel.

Pharmaceutical compositions of this invention can be in a form suitable for rectal administration wherein the carrier is a solid or liquid or spray. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in moulds.

In addition to the aforementioned carrier ingredients, the pharmaceutical formulations described above may include, as appropriate, one or more additional carrier ingredients such as diluents, buffers, binders, surface-active agents, thickeners, lubricants, preservatives (including anti-oxidants) and the like. Furthermore, other adjuvants can be included to render the formulation isotonic with the blood of the intended recipient. Compositions containing a compound described by Formula (I), or pharmaceutically acceptable salts thereof, may also be prepared in powder or liquid concentrate form. Addition of preservatives such as anti-oxidants are widely acceptable in pharmaceutical arts as a means of simulating long-term storage in order to determine characteristics such as shelf life or stability of formulations over time (See e.g., Jens T. Carstensen, Drug stability: Principles & Practice. 2nd Ed., Marcel Dekker, NY, NY. 1995, pp 379-80).

The compounds and pharmaceutical compositions of this invention have been found to exhibit biological activity as PDE4 inhibitors when locally activated in the colon. Accordingly, another aspect of the invention is the treatment in mammals of, for example, i) Pulmonary disorders such as asthma, chronic bronchitis, chronic obstructive pulmonary disease (COPD), adult respiratory distress syndrome, infant respiratory distress syndrome, cough, and chronic obstructive pulmonary disease in animals ii) Gastrointestinal disorders such as ulcerative colitis, Crohn's disease, diverticulitis, irritable bowel syndrome and hypersecretion of gastric acid, iii) Infectious diseases such as bacterial, fungal or viral induced sepsis or septic shock, endotoxic shock (and associated conditions such as laminitis and colic in horses), and septic shock, iv) Neurological disorders such as spinal cord trauma, head injury, neurogenic inflammation, pain, and reperfusion injury of the brain, v) Inflammatory disorders such as psoriatic arthritis, rheumatoid arthritis, ankylosing spondylitis, osteoarthritis, inflammation and cytokine-mediated chronic tissue degeneration, vi) Allergic disorders such as allergic rhinitis, allergic conjunctivitis, and eosinophilic granuloma, vii) Psychiatric disorders such as depression, memory impairment, and monopolar depression, viii) Neurodegenerative disorders such as Parkinson disease, Alzheimer's disease, acute and chronic multiple sclerosis, ix) Dermatological disorders such as psoriasis and other benign or malignant proliferative skin diseases, atopic dermatitis, and urticaria, x) Oncological diseases such as cancer, tumor growth and cancerous invasion of normal tissues, xi) Metabolic disorders such as diabetes insipidus, xii) Bone disorders such as osteoporosis, xiii) Cardiovascular disorders such as arterial restenosis, atherosclerosis, reperfusion injury of the myocardium, and xiv) Other disorders such as chronic glomerulonephritis, vernal conjunctivitis, transplant rejection and graft versus host disease, and cachexia - maladies that are amenable to amelioration through inhibition of the PDE4 isoenzyme and the resulting elevated cAMP levels - by the administration of an effective amount of the compounds of this invention. The term "mammals" includes humans, as well as other animals such as, for example, dogs, cats, horses, pigs, and cattle. Accordingly, it is understood that the treatment of mammals other than humans is the treatment of clinical correlating afflictions to those above recited examples that are human afflictions.

Further, as described above, the compound of this invention may be utilized in combination with other therapeutic compounds. In particular, the combinations of the PDE4 inhibiting compound of this invention may be advantageously used in combination with i) Leukotriene receptor antagonists, ii) Leukotriene biosynthesis inhibitors, iii) COX-2 selective inhibitors, iv) statins, v) NSAIDs, vi) M2/M3 antagonists, vii) corticosteroids, viii) HI (histamine) receptor antagonists, ix) $\beta_2$ adrenoceptor agonist, x) 5-ASA and 5ASA prodrugs, xi) azathioprine, xii) cyclosporine, xiii) methotrexate and xiv) Janus kinase (JAK) inhibitors.

Thus, for example, pulmonary disorders such as asthma, chronic bronchitis, chronic obstructive pulmonary disease (COPD), adult respiratory distress syndrome, infant respiratory distress syndrome, cough, chronic obstructive pulmonary disease in animals, and infant respiratory distress syndrome may be conveniently treated with capsules, cachets or tablets each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Gastrointestinal disorders such as ulcerative colitis, Crohn's disease, diverticulitis, irritable bowel syndrome and hypersecretion of gastric acid may be conveniently treated with capsules, cachets or tablets each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Infectious diseases such as bacterial, fungal or viral induced sepsis or septic shock, endotoxic shock (and associated conditions such as laminitis and colic in horses), and septic shock may be conveniently treated with capsules, cachets or tablets each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Neurological disorders such as spinal cord trauma, head injury, neurogenic inflammation, pain, and reperfusion injury of the brain may be conveniently treated with capsules, cachets or tablets each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Inflammatory disorders such as psoriatic arthritis, rheumatoid arthritis, ankylosing spondylitis, osteoarthritis, inflammation and cytokine-mediated chronic tissue degeneration may be conveniently treated with capsules, cachets or tablets or topical formulations each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Allergic disorders such as allergic rhinitis, allergic conjunctivitis, and eosinophilic granuloma may be conveniently treated with capsules, cachets or tablets or nasal sprays each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Psychiatric disorders such as depression, memory impairment, and monopolar depression may be conveniently treated with capsules, cachets or tablets or injectables each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Neurodegenerative disorders such as Parkinson disease, Alzheimer's disease, acute and chronic multiple sclerosis may be conveniently treated with capsules, cachets or tablets or injectables each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Dermatological disorders such as psoriasis and other benign or malignant proliferative skin diseases, atopic dermatitis, and urticaria may be conveniently treated with capsules, cachets or tablets or topical delivery systems each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Oncological diseases such as cancer, tumor growth and cancerous invasion of normal tissues may be conveniently treated with capsules, cachets or tablets or parenteral formulations each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Metabolic disorders such as diabetes insipidus may be conveniently treated with capsules, cachets or tablets or injectables each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

Bone disorders such as osteoporosis, cardiovascular disorders such as arterial restenosis, atherosclerosis, reperfusion injury of the myocardium, and other disorders such as chronic glomerulonephritis, vernal conjunctivitis, transplant rejection and graft versus host disease, and cachexia may be conveniently treated with capsules, cachets or tablets each containing 0.001 mg, 0.005 mg, 0.025mg, 0.1 mg, 0.5 mg, 2.5 mg, 10 mg, 50 mg, 250 mg, or 1000 mg of the active ingredient of the compound of the present application, or a pharmaceutically acceptable salt thereof, administered once, twice, or three times daily.

## EXAMPLE 1

## METHODS OF SYNTHESIS

The compounds of Formula **(I)** of the present invention can be prepared according to the proposed synthetic routes outlined in Schemes 1-7 below. The glycosidation reactions are described for the coupling of racemic pyridone but it is obvious to one skilled in the art that the same reactions apply to optically pure or optically enriched pyridones **III.** The substituents are the same as in Formula **(I)** except where defined otherwise. The PDE4 inhibitors pyridine-*N*-oxide II can be prepared using procedures such as the one described in Friesen et al. (J. Med. Chem. 2003, 46(12), 2413) and/or O'Shea et al. (J. Org. Chem. 2005, 70, 3021).

The glucopyranoside of Formula **Ia-c** may be prepared in a multi-step sequence from the requisite pyridone **III** and an appropriate glucosyl donor **IV** as presented in Scheme 1 below. The requisite pyridone **III** can be synthesized

by the rearrangement of the pyridine-*N*-oxide **II** in the presence of an activating agent such as trifluoroacetic anhydride or tosyl anhydride in the presence of a tertiary amine such as triethylamine or Hunig's base in a solvent such as toluene, chlorobenzene, THF, diethyl ether, 1,4-dioxane, dichloromethane or a mixture of solvents such as toluene and 2-methyl-THF at a temperature such as 0°C to room temperature. Once the rearrangement has been completed, the resulting mixture can be further treated with a base such as LiOH, NaOH or $NaHCO_3$ to afford the pyridone **III.** The glucosyl donor **IV** is characterized by a functionality Z at the anomeric position that can be activated under appropriate conditions with an activating agent such as a protic acid, a Lewis acid, a silver salt or a mercuric salt. When Z = OH, the coupling can be accomplished under Mitsunobu type reaction. In one method, pyridone **III** would be coupled to the 1-halogeno-α-glucopyranoside (α-**IV**, Z = Cl, Br, I) under standard Koenigs-Knorr conditions known to those skilled in the art to afford the protected β-glucopyranoside **Ia** (P = P'). Using another procedure according to Saad et al. (Curr. Org. Synth. 2012, 9(3), 413), the pyridone **III** would be coupled to the bromo-α-glucopyranoside (α-**IV,** Z = Br) in the presence of a base such as $K_2CO_3$ in an aprotic polar solvent such as DMF. In another method, the pyridone **III** would be coupled to a 1-*O*-trichloroacetimidate-α-glucopyranoside (α-**IV**, Z = OC(NH)$CCl_3$) in the presence of a Lewis acid such as $BF_3$ $Et_2O$ in a suitable solvent such as dichloromethane. In another method, according to the procedure described by Sokolov et al. (Russian J. General Chem. 2002, 72(5), 806), the pyridone **III** would be coupled to a 1-O-acetyl-β-glucopyranoside (β-**IV**, Z = OAc) in the presence of a catalytic amount of Lewis acid such as $BF_3$ $Et_2O$ in a suitable solvent such as benzene at a controlled temperature such as room temperature to afford the protected β-glucopyranoside **Ia**. In another method, according to the procedure described by Ko et al. (Org. Lett. 2009, 11(3), 609.), the pyridone **III** would be coupled to α-**IV** (Z = I), where P = acetyl and P' = iodoacetyl, in the presence of a silver salt such as AgOTf in a solvent such as nitromethane or dichloromethane at ambient temperature. Removal of the alcohol protecting group P and P' of β-glucopyranoside **Ia** can be accomplished in the presence of a basic agent such as sodium methoxide, LiOH, NaOH, KOH or $K_2CO_3$, when P and P' are alkyl/aryl esters, in an appropriate solvent such as methanol or ethanol to afford the β-glucopyranoside **Ib**. If P and P' are benzyl, deprotection may be accomplished under standard conditions known to those skilled in the art such $H_2$ hydrogenolysis using Pd/C as a catalyst in a solvent such as methanol to afford the β-glucopyranoside **Ib**. In the event that the ester protecting group P is different from the ester protecting group P', according to the procedure of Ko et al. (Org. Lett. 2009, 11(3), 609.) the protecting group P' may be hydrolyzed selectively using a reagent such as thiourea to afford the partially protected 2-hydroxy-β-glucopyranoside **1c.**

**Scheme 1**

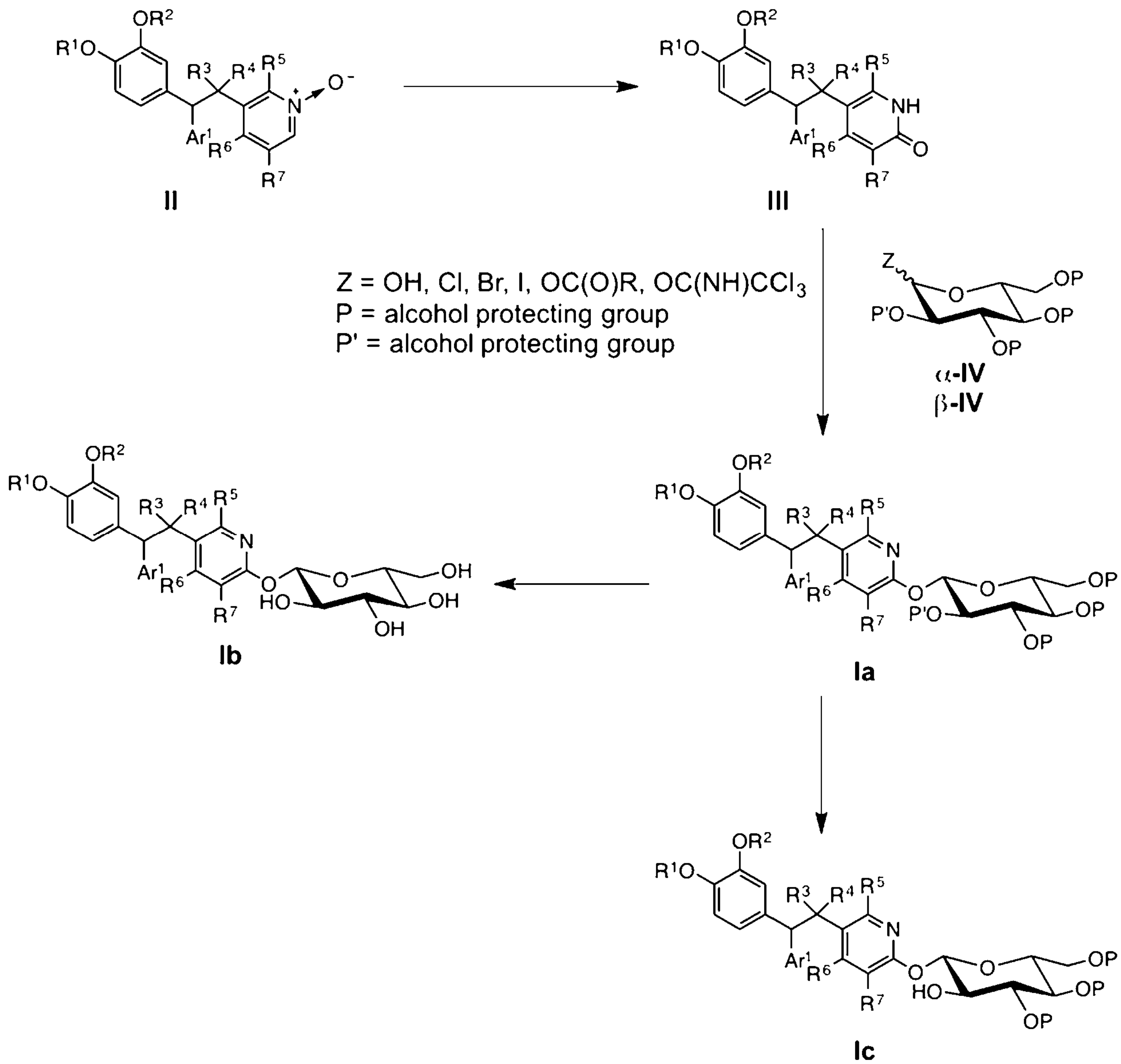


II III α-IV β-IV Ia Ib Ic

The glucuronide of Formula **Id-f** may be prepared in a multi-step sequence from the requisite pyridone **III** and an appropriate glucuronyl donor **V** as presented in Scheme 2 below. The glucuronyl donor is characterized by a functionality Z at the anomeric position that can be activated under appropriate conditions with an activating agent such as a protic acid, a Lewis acid, a silver salt or a mercuric salt. When Z = OH, the coupling can be accomplished under Mitsunobu type reaction. In one method, according to the procedure described by Zhang et al. (Tetrahedron, 2012, 68, 4194), the pyridone **III** would be coupled to a 1-*O*-trichloroacetimidate-*α*-glucuronide (α-**V**, Z = OC(NH)$CCl_3$) in the presence of a Lewis acid such as $BF_3$ $Et_2O$ in a suitable solvent such as dichloromethane at a controlled temperature such as -20 °C to afford the protected *β*-glucuronide **Id**. In another method according to the procedure described by Arewang et al. (Carbohydr. Res. 2007, 342(7), 970) the pyridone **III** would be coupled to a 1-*O*-acetyl-*α*-glucuronide (α-**V**, Z = OAc) in the presence of a Lewis acid such as $BF_3$ $Et_2O$ in a suitable solvent such as dichloromethane at a controlled temperature such as 0 °C to room temperature to afford the protected *β*-glucuronide **Id**. In another method according to the procedure described by Berrang et al. (Synth. Commun. 1975, 5, 231), the pyridone **III** would be initially deprotonated with a base such as LiOH and coupled to a 1-bromo-*α*-glucuronide (α-**V**, Z = Br) in a solvent such as ethanol at room temperature. In an alternative procedure using the 1-bromo-*α*-glucuronide (α-**V**, Z = Br), the pyridone **III** would be coupled according to the procedure of WO2011/147296 by deprotonation of the pyridone with an hydride such as NaH in a solvent such as dichloromethane followed by the addition the bromide in the presence of a silver salt such as $AgNO_3$. The protected *β*-glucuronide **Id** could also be obtained under standard Koenigs-Knorr conditions known

to those skilled in the art such as the procedure described by Friend et al. (J. Med. Chem. 1985, 28, 51) where the pyridone **III** would be coupled to the 1-bromo-α-glucuronide (α-**V**, Z = Br) in a solvent such as $CHCl_3$ or toluene in the presence of a silver salt such as $Ag_2CO_3$ or $Ag_2O$. When P is an alkyl/aryl ester, removal of the alcohol protecting group P of β-glucuronide **Id** can be accomplished in the presence of a basic agent such as LiOH, NaOH, KOH or $K_2CO_3$, in an appropriate solvent such as methanol/water or ethanol/water to afford the β-glucuronide **Ie**. If Alkyl of **Id** is methyl and P is and alkyl ester such an acetate, **If** may be prepared by treating **Id** with a methanolic alkoxide such as sodium methoxide in an appropriate solvent such methanol. If P = benzyl, deprotection may be accomplished under standard conditions known to those skilled in the art such $H_2$ hydrogenolysis using Pd/C as a catalyst in a solvent such as methanol to afford the β-glucuronide **Ie**. The acid functionality or the corresponding salt of glucuronide **Ie** may be reacted selectively with a reagent such as diazomethane or trimethylsilyl diazomethane in a solvent such as methanol to afford the methyl ester β-glucuronide **If** (alkyl = methyl). In another method, **If** may be prepared by treating **Ie** or its corresponding carboxylate salt, with an alcohol such as methanol, ethanol or i-propanol and a coupling agent such as DCC or EDC in a solvent such as DMF in the presence of DMAP. In an alternative procedure, If (alky = tert-butyl) may be prepared by reacting **Ie** or its corresponding carboxylate salt, with *tert*-butyltrichloroacetimidate and a Lewis acid such as $BF_3$ $Et_2O$ in a suitable solvent such as dichloromethane.

## Scheme 2

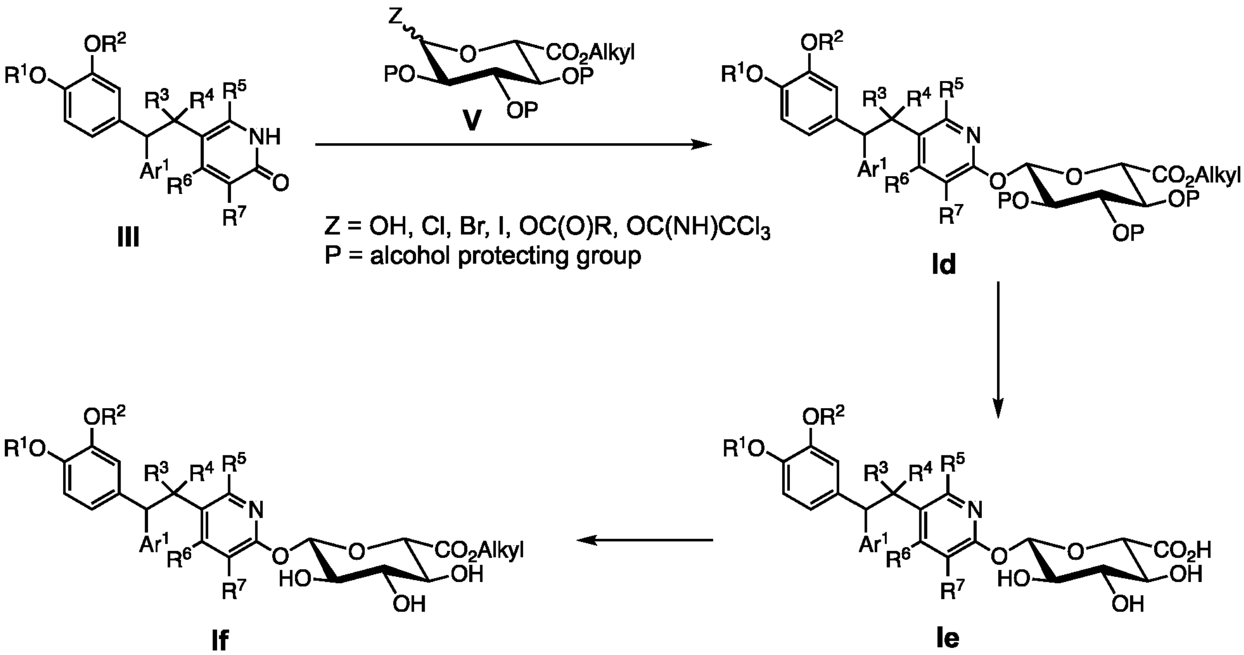

III
V
Id
Ie
If

The glucosamine of Formula **Ig** may be prepared in a multi-step sequence from the requisite pyridone **III** and a glycosyl donor such as **VI**, as presented in Scheme 3 below. Glycosyl donor **VI** (X = Cl) may be synthesized according to the procedure of St-Pierre et al. (Synthesis 2016, 48, 3575). Glycosyl azide **VII** could be obtained as a mixture of anomers by coupling the pyridone **III** and the glycosyl donor **VI** (X = Cl) in the presence of a silver salt such as $Ag_2O$ in a solvent such as toluene under refluxing conditions. Hydrolysis of the ester functionalities of the protected glycosyl azide **VII** can be accomplished in the presence of a basic agent such as sodium methoxide, LiOH, NaOH, KOH or $K_2CO_3$ in an appropriate solvent such as methanol. The 2-azido functionality could be reduced to the 2-amino functionality under an atmosphere of $H_2$ in the presence of a catalyst such as Pd/C in a solvent such as methanol to afford an anomeric mixture of glucosamine **Ig**. The anomeric mixture could be separated by chromatographic method known to those skilled in the art to afford the α-glucosamine α-**Ig** and the β-glucosamine β-**Ig**. Alternatively, the single glucosamine isomer of Formula β-**Ig** may be prepared in a multi-step sequence from the requisite pyridone **III** and a glycosyl donor such as **VIII,** as presented in Scheme 4 below. Glycosyl donor **VIII** may be synthesized according to the procedure of Morais et al. (Carbohydr. Res. 2003, 338, 1369.). Glucosamine **IX** could be obtained by coupling the pyridone **III** and the glycosyl donor **VIII** in the presence of a silver salt such as $Ag_2O$ in a solvent such as toluene under refluxing conditions. Hydrolysis of the ester functionalities of the protected glucosamine **IX** can be accomplished in the presence of a basic agent such as sodium methoxide, LiOH, NaOH, KOH or $K_2CO_3$ in an appropriate solvent such as methanol. The benzyloxycarbonyl (CBZ) protecting group could be hydrogenolyzed to the 2-amino functionality under an atmosphere of $H_2$ in the presence

of a catalyst such as Pd/C in a solvent such as methanol to afford the glucosamine β-**Ig.**

**Scheme 3**

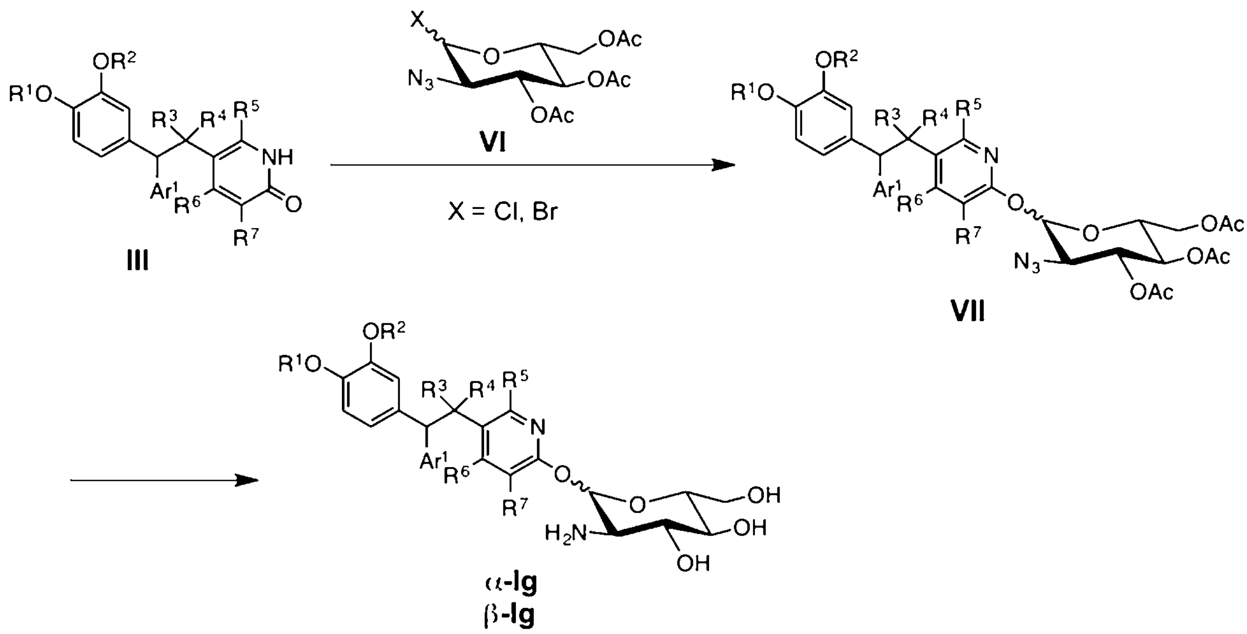


III

VI

VII

α-Ig

β-Ig

**Scheme 4**

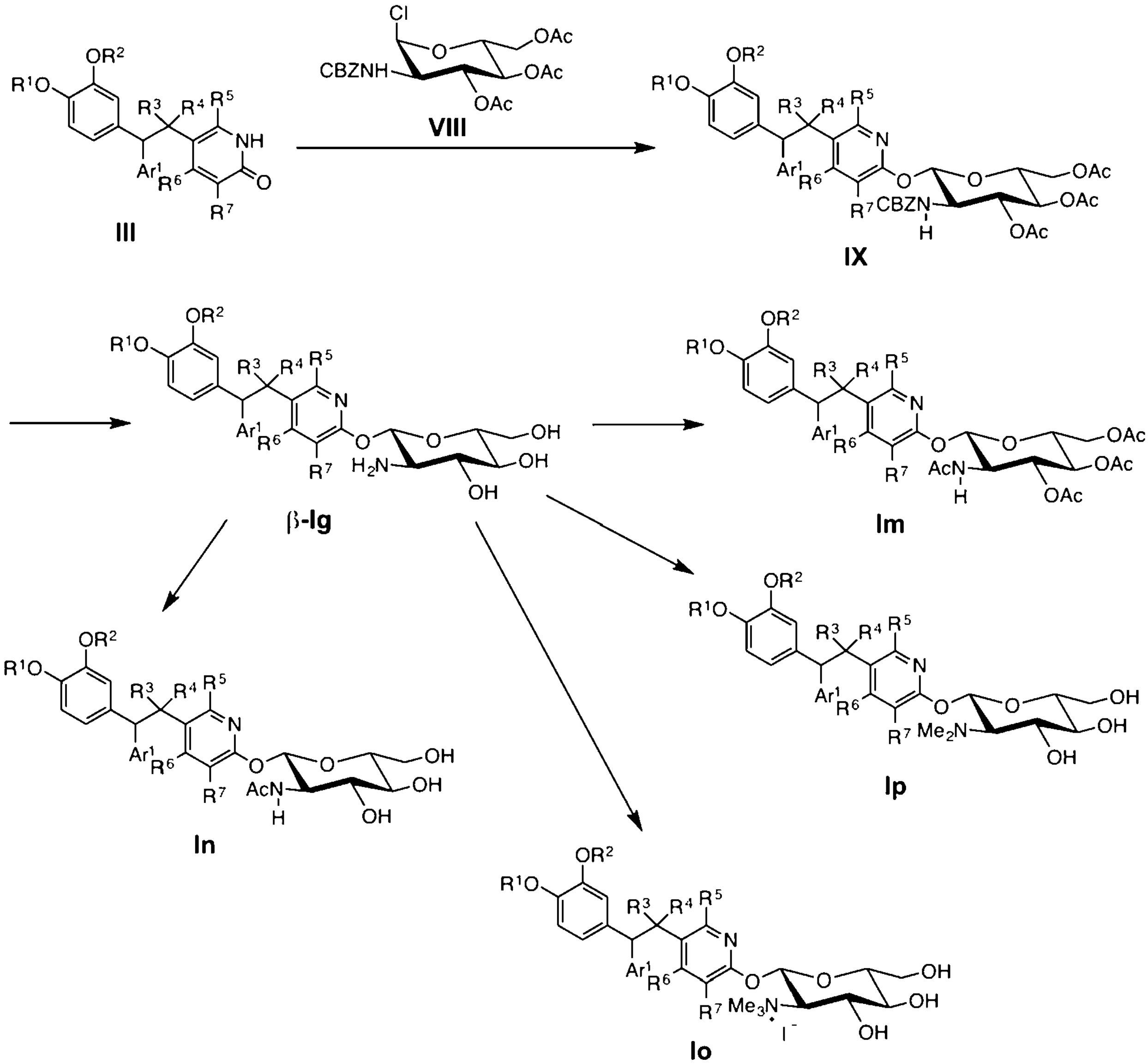

III VIII IX β-Ig Im In Ip Io

As describe in scheme 4 above, the glucosamine β-**Ig** could be further derivatized to access the glucosamines of Formula **Im, In, Io** and **Ip**. The polyacetylated glucosamine of Formula **Im** may be prepared by the action of an acetylating agent, such as acetic anhydride, on the intermediate β-**Ig** in a solvent such as pyridine at room temperature. Alternatively, β-**Ig** could be treated with an acetylating agent, such as acetic anhydride, in the presence of a base such as triethylamine in a solvent such as methanol at 0 °C to afford the monoacetylated glucosamine of Formula **In.** Alternatively, β-**Ig** could be treated with an excess of an alkylating agent such as methyl iodide in the presence of a base such as $i$-$Pr_2NEt$ in a solvent such as THF at room temperature to afford the quaternary ammonium salt **Io**. Alternatively, the glucosamine of Formula **Ip** could be prepared by placing β-**Ig** under reductive alkylation conditions using formaldehyde and a reducing agent such as sodium cyanoborohydride in methanol at room temperature.

The galactopyranosides of Formula **Ii** and **Ij** may be prepared in a multi-step sequence from the requisite pyridone **III** and an appropriate glycosyl donor **X** as presented in Scheme 5 below. The glycosyl donor **X** is characterized by a functionality Z at the anomeric position that can be activated under appropriate conditions with an activating agent such as a protic acid, a Lewis acid, a silver salt or a mercuric salt. When Z = Cl, Br or I, the coupling can be accomplished under standard Koenigs-Knorr conditions known to those skilled in the art to afford the polyacetate β-galactopyranoside **Ii**. In another method, the pyridone **III** would be coupled to a 1-*O*-trichloroacetimidate-α-galactopyranoside (Z = $OC(NH)CCl_3$) in the presence of a Lewis acid such as $BF_3$ $Et_2O$ in a suitable solvent such as dichloromethane. Removal of the acetyl protecting groups of β-galactopyranoside **Ii** can be accomplished in the presence of a basic agent such as sodium methoxide, LiOH, NaOH, KOH or $K_2CO_3$ in an appropriate solvent such as methanol or a mixture of solvent

such as THF/water to afford the β-galactopyranoside **Ij**.

**Scheme 5**

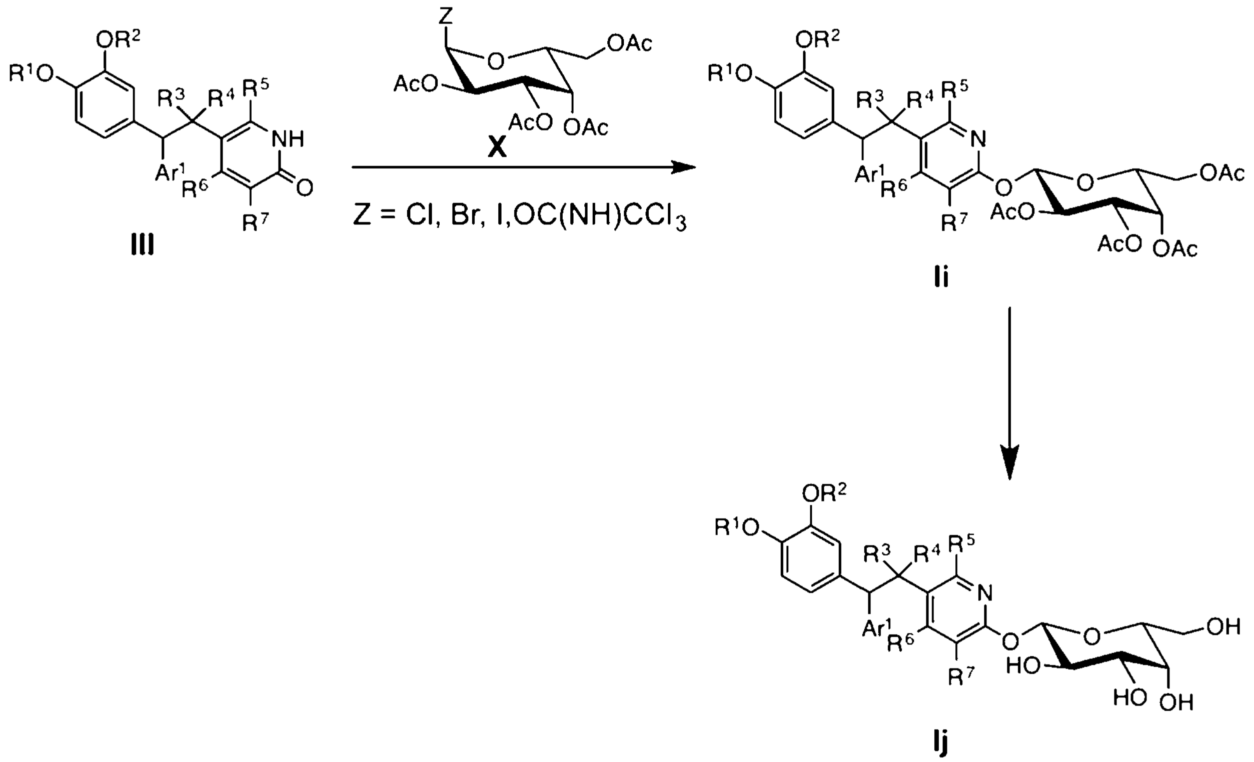


III

X

Ii

Ij

The mannopyranosides of Formula **Ik** and **Il** may be prepared in a multi-step sequence from the requisite pyridone **III** and an appropriate glycosyl donor **XI** as presented in Scheme 6 below. The glycosyl donor **XI** is characterized by a functionality Z at the anomeric position that can be activated under appropriate conditions with an activating agent such as a protic acid, a Lewis acid, a silver salt or a mercuric salt. When Z = Cl, Br or I, the coupling can be accomplished under standard Koenigs-Knorr conditions known to those skilled in the art to afford the polyacetate β-galactopyranoside **Ik.** In another method, the pyridone **III** would be coupled to a 1-*O*-trichloroacetimidate-α-mannopyranoside (Z = $OC(NH)CCl_3$) in the presence of a Lewis acid such as $BF_3$ $Et_2O$ in a suitable solvent such as dichloromethane. Removal of the acetyl protecting groups of β-mannopyranoside **Ik** can be accomplished in the presence of a basic agent such as sodium methoxide, LiOH, NaOH, KOH or $K_2CO_3$ in an appropriate solvent such as methanol or a mixture of solvent such as THF/water to afford the β-galactopyranoside **Il**.

**Scheme 6**

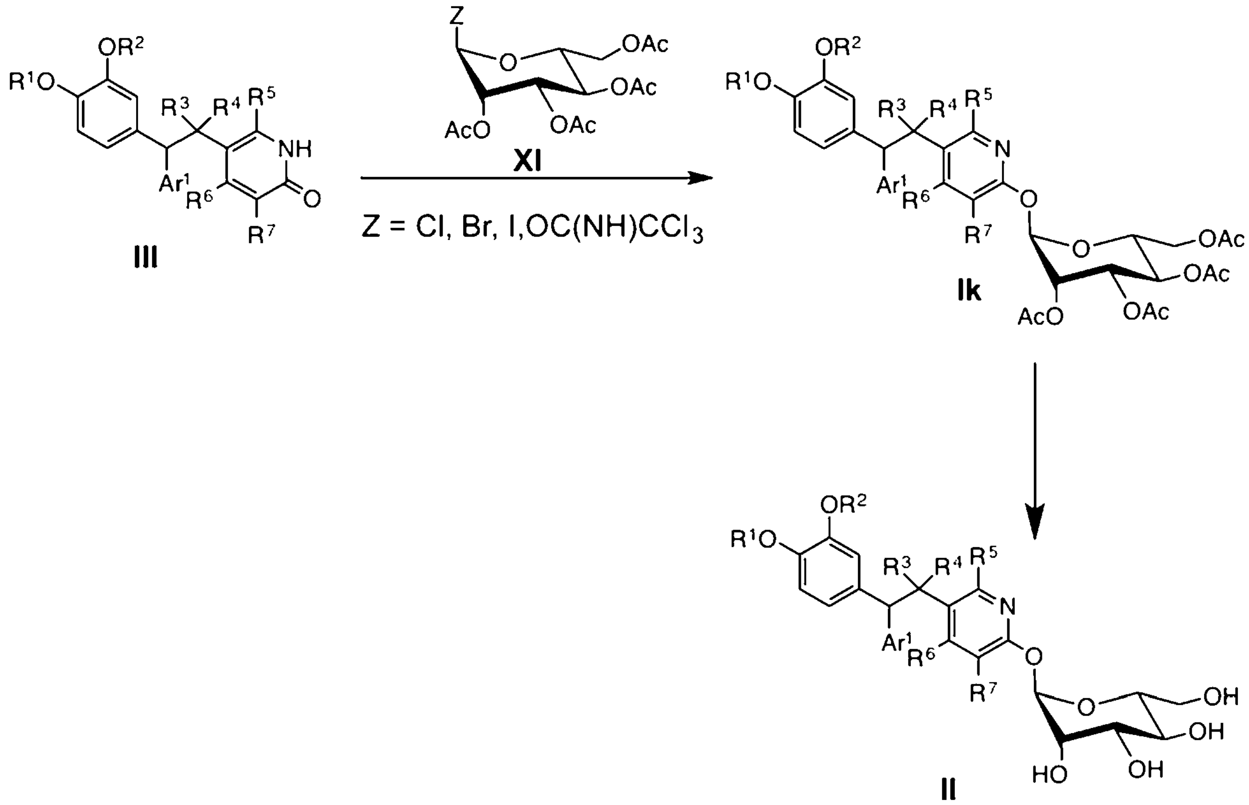


The cellobioside of Formula **Iq** and **Ir** may be prepared in a multi-step sequence from the requisite pyridone **III** and an appropriate glycosyl donor **XII** as presented in Scheme 7 below. The glycosyl donor **XII** is characterized by a functionality Z at the anomeric position that can be activated under appropriate conditions with an activating agent such as a protic acid, a Lewis acid, a silver salt or a mercuric salt. When Z = Cl, Br or I, the coupling can be accomplished under standard Koenigs-Knorr conditions known to those skilled in the art to afford the polyacetate β-cellobioside Iq. In another method, the pyridone **III** would be coupled to a 1-*O*-trichloroacetimidate-α-mannopyranoside (Z = $OC(NH)CCl_3$) in the presence of a Lewis acid such as $BF_3$ $Et_2O$ in a suitable solvent such as dichloromethane. Removal of the acetyl protecting groups of β-cellobioside Iq can be accomplished in the presence of a basic agent such as sodium methoxide, LiOH, NaOH, KOH or $K_2CO_3$ in an appropriate solvent such as methanol or a mixture of solvent such as THF/water to afford the β-cellobioside **Ir.**

**Scheme 7**

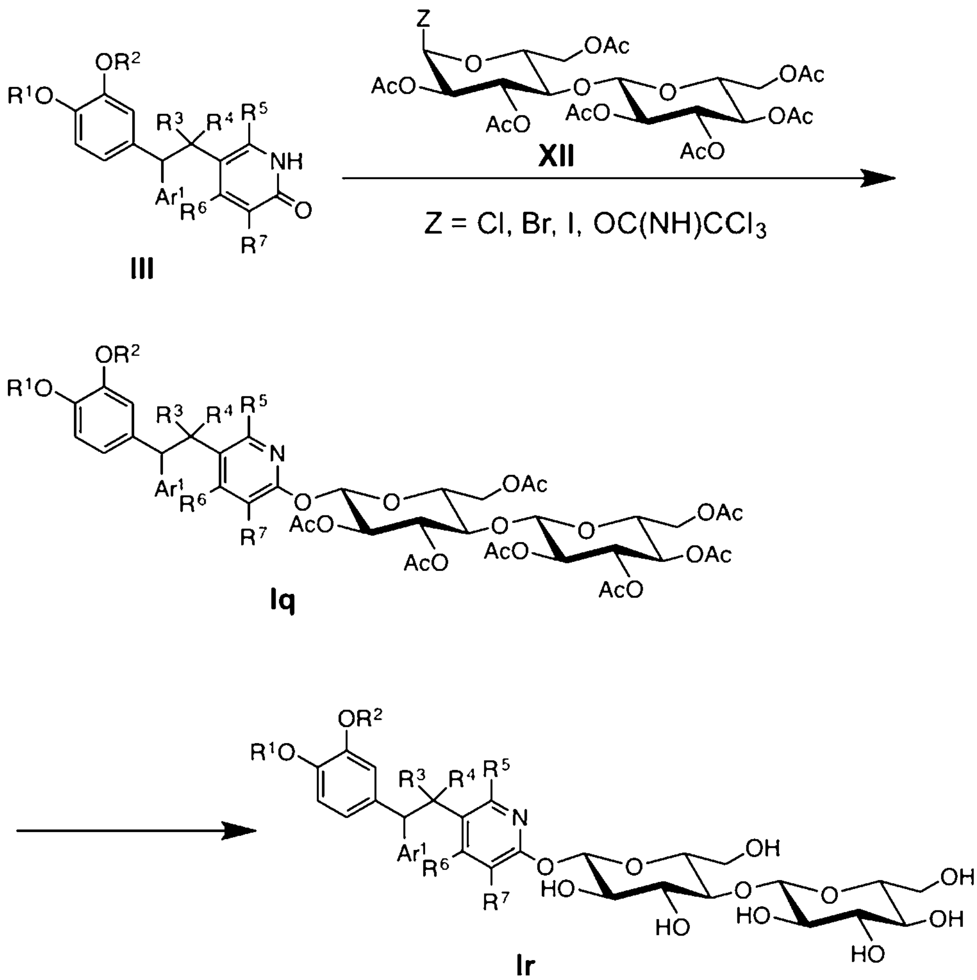


III

XII

Iq

Ir

**Compounds 1-31** are summarized in Table 1 below:

**Table 1**

| 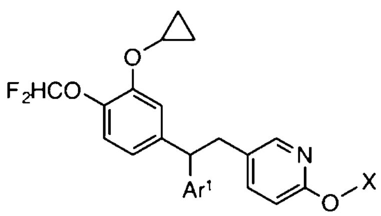 Where X is selected from (a) $\beta$-D-glucopyranoside, (b) $\beta$-D-glucuronide, (c) $\beta$-D-galactopyranoside, (d) $\alpha$-D-mannopyranoside, (e) $\alpha/\beta$-D-glucosaminide, (f) $\beta$-D-cellobioside and $Ar^1$ is selected from (a) 2-(hexafluoro-*i*-propanol)-5-thiazolyl and (b) 6-(2-hydroxy-propane-2-yl)-3-pyridyl. | | |
|---|---|---|
| **Cmpd** | **$Ar^1$** | **X** |
| **1** | 2-(hexafluoro-*i*-propanol)-5-thiazolyl | 2,3,4,6-tetra-*O*-acetyl-β-D-glucopyranosyl |
| **2** | 2-(hexafluoro-*i*-propanol)-5-thiazolyl | P-D-glucopyranosyl |
| **3** | 6-(2-hydroxy-propane-2-yl)-3-pyridyl | 2,3,4,6-tetra-*O*-acetyl-β-D-glucopyranosyl |

(continued)

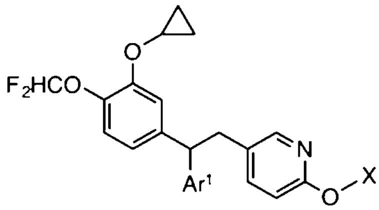

Where X is selected from (a) *β*-D-glucopyranoside, (b) *β*-D-glucuronide, (c) *β*-D-galactopyranoside, (d) *α*-D-mannopyranoside, (e) *α*/*β*-D-glucosaminide, (f) *β*-D-cellobioside and $Ar^1$ is selected from (a) 2-(hexafluoro-*i*-propanol)-5-thiazolyl and (b) 6-(2-hydroxy-propane-2-yl)-3-pyridyl.

| **Cmpd** | **$Ar^1$** | **X** |
|---|---|---|
| 4 | 6-(2-hydroxy-propane-2-yl)-3-pyridyl | P-D-glucopyranosyl |
| 5 | 6-(2-hydroxy-propane-2-yl)-3-pyridyl | 3,4,6-tri-*O*-acetyl-β-D-glucopyranosyl |
| 6 | 2-(hexafluoro-*i*-propanol)-5-thiazolyl | Methyl 2,3,4-tri-*O*-acetyl-β-D-glucuronate |
| 7 | 2-(hexafluoro-*i*-propanol)-5-thiazolyl | Methyl β-D-glucuronate |
| **8** | 6-(2-hydroxy-propane-2-yl)-3-pyridyl | Methyl 2,3,4-tri-*O*-acetyl-β-D-glucuronate |
| **9** | 6-(2-hydroxy-propane-2-yl)-3-pyridyl | Lithium *β*-D-glucuronide |
| **10** | 6-(2-hydroxy-propane-2-yl)-3-pyridyl | Methyl β-D-glucuronate |
| **11** | 6-(2-hydroxy-propane-2-yl)-3-pyridyl | Ethyl 2,3,4-tri-*O*-acetyl-β-D-glucuronate |
| **12** | 6-(2-hydroxy-propane-2-yl)-3-pyridyl | Ethyl β-D-glucuronate |
| **13** | 6-(2-hydroxy-propane-2-yl)-3-pyridyl | i-Propyl β-D-glucuronate |
| **14** | 6-(2-hydroxy-propane-2-yl)-3-pyridyl | tert-Butyl β-D-glucuronate |
| **15** | 2-(hexafluoro-*i*-propanol)-5-thiazolyl | Methyl *β*-D-glucuronamide |
| **16** | 6-(2-hydroxy-propane-2-yl)-3-pyridyl | Methyl *β*-D-glucuronamide |
| **17** | 6-(2-hydroxy-propane-2-yl)-3-pyridyl | 2,3,4,6-tetra-*O*-acetyl-*β*-D-galactopyranosyl |
| **18** | 6-(2-hydroxy-propane-2-yl)-3-pyridyl | *β*-D-galactopyranosyl |
| **19** | 6-(2-hydroxy-propane-2-yl)-3-pyridyl | 2,3,4,6-tetra-*O*-acetyl-α-D-mannopyranosyl |
| **20** | 6-(2-hydroxy-propane-2-yl)-3-pyridyl | α-D-mannopyranosyl |
| **21** | 6-(2-hydroxy-propane-2-yl)-3-pyridyl | α/β-D-glucosaminyl |

(continued)

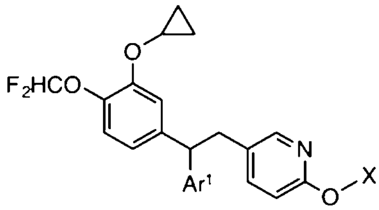

Where X is selected from (a) *β*-D-glucopyranoside, (b) *β*-D-glucuronide, (c) *β*-D-galactopyranoside, (d) *α*-D-mannopyranoside, (e) *α*/*β*-D-glucosaminide, (f) *β*-D-cellobioside and $Ar^1$ is selected from (a) 2-(hexafluoro-*i*-propanol)-5-thiazolyl and (b) 6-(2-hydroxy-propane-2-yl)-3-pyridyl.

| Cmpd | $Ar^1$ | X |
|---|---|---|
| **22** | 2-(hexafluoro-*i*-propanol)-5-thiazolyl | β-D-glucosaminyl |
| **23** | 6-(2-hydroxy-propane-2-yl)-3-pyridyl | β-D-glucosaminyl |
| **24** | 2-(hexafluoro-*i*-propanol)-5-thiazolyl | 3,4,6-tri-*O*-acetyl-*N*-acetyl-*β*-D-glucosaminyl |
| **25** | 2-(hexafluoro-*i*-propanol)-5-thiazolyl | *N*-acetyl-β-D-glucosaminyl |
| **26** | 2-(hexafluoro-*i*-propanol)-5-thiazolyl | *N,N,N*-trimethyl-β-D-glucosaminyl iodide |
| **27** | 2-(hexafluoro-*i*-propanol)-5-thiazolyl | *N,N*-dimethyl-β-D-glucosaminyl |
| **28** | 2-(hexafluoro-*i*-propanol)-5-thiazolyl | hepta-*O*-acetyl-P-D-cellobiosyl |
| **29** | 2-(hexafluoro-i-propanol)-5-thiazolyl | β-D-cellobiosyl |
| **30** | 6-(2-hydroxy-propane-2-yl)-3-pyridyl | hepta-*O*-acetyl-β-D-cellobiosyl |
| **31** | 6-(2-hydroxy-propane-2-yl)-3-pyridyl | β-D-cellobiosyl |

**Compound 1:** (2R,3R,4S,5R,6S)-2-(acetoxymethyl)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3,4,5-triyl triacetate

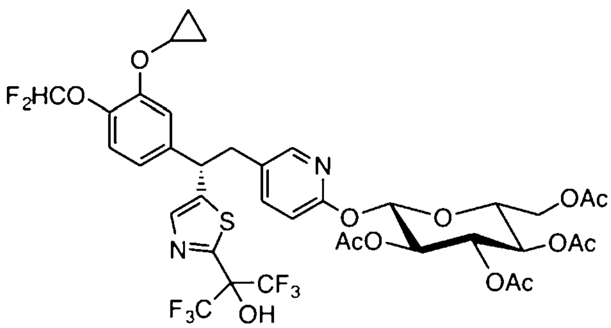

**Compound 1** was prepared by the following procedure: Step 1: (S)-5-(2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2(1H)-one. (S)-3-(2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridine 1-oxide **IIa** (1.00 g, 1.75 mmol), prepared according to O'Shea et al. (J. Org. Chem. 2005, 70, 3021), was dissolved in toluene (8.77 mL, 0.2 M). The solution was cooled to 0 °C, and triethylamine (0.73 mL, 5.25 mmol) was added, followed by dropwise addition of trifluoroacetic anhydride (0.74 mL, 5.25 mmol). Once the addition was completed, the reaction was warmed to room temperature and stirred for 30 minutes. The reaction was then cooled to 0 °C and 20 mL of saturated aqueous sodium bicarbonate solution was added. The resulting solution was stirred for 15 minutes before it was diluted with 20 mL of EtOAc and the layers were separated. The organic layer was then washed with aqueous saturated sodium bicarbonate solution (10 mL), water (10 mL) and brine (10 mL). The organic layer was dried over magnesium sulfate, concentrated, and purified by reversed phase column chromatography using 10-60% MeCN in ammonium bicarbonate buffer. The pure fractions were then combined and concentrated to afford the desired pyridone **IIIa** as an off-white solid.

Step 2: (2R,3R,4S,5R,6S)-2-(acetoxymethyl)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3,4,5-triyl triacetate. To a solution of (S)-5-(2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2(1H)-one **IIIa** (101 mg, 0.18 mmol) in toluene (0.2 M) was added silver oxide (1.5 equiv) and 1-bromo-2,3,4,6-tetra-*O*-acetyl-α-D-glucopyranoside (1.5 equiv). The resulting suspension was stirred at 110 °C for 1.5 hours. The reaction was filtered over celite, condensed under reduced pressure and purified by reversed phase column chromatography eluting with 20-80% MeCN in ammonium formate to afford after lyophilization the desired glucopyranoside as a white solid: $^1$H NMR (400 MHz, Acetone-$d_6$) δ 7.98 (d, *J* = 2.3 Hz, 1H), 7.85 (s, 1H), 7.62 (dd, *J* = 8.5, 2.4 Hz, 1H), 7.41 (d, *J* = 2.0 Hz, 1H), 7.10 (d, *J* = 8.2 Hz, 1H), 7.01 - 6.57 (m, 3H), 6.26 (d, *J* = 8.3 Hz, 1H), 5.42 (t, *J* = 9.6 Hz, 1H), 5.20 - 5.06 (m, 2H), 4.80 (dd, *J* = 9.3, 6.7 Hz, 1H), 4.27 (dd, *J* = 12.3, 4.6 Hz, 1H), 4.11 (ddd, *J* = 10.0, 4.5, 2.4 Hz, 1H), 4.03 (dd, *J* = 12.3, 2.3 Hz, 1H), 3.91 - 3.84 (m, 1H), 3.54 (dd, *J* = 13.8, 6.6 Hz, 1H), 3.44 (dd, *J* = 13.8, 9.5 Hz, 1H), 2.01 (s, 3H), 1.97 - 1.95 (m, 6H), 1.89 (s, 3H), 0.86 - 0.69 (m, 3H), 0.62 - 0.52 (m, 1H).

**Compound** 2: (2S,3R,4S,5S,6R)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-6-(hydroxymethyl)tetrahydro-2H-pyran-3,4,5-triol

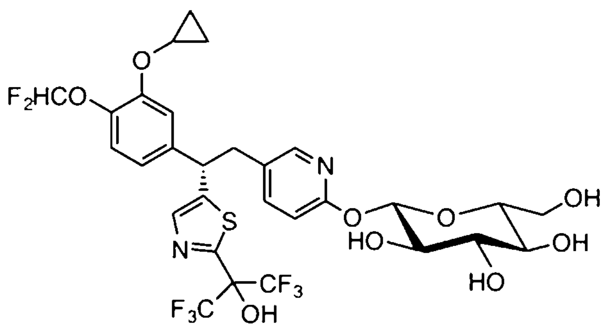

**Compound 2** was prepared by the following procedure: Step 1: (2S,3R,4S,5S,6R)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-6-(hydroxymethyl)tetrahydro-2H-pyran-3,4,5-triol. To a solution of (2R,3R,4S,5R,6S)-2-(acetoxymethyl)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3,4,5-triyl triacetate (10 mg, 0.01 mmol) in THF/Water (1:1, 0.1 M) was added LiOH (15 eq). The solution was stirred at room temperature for 30 minutes and the solution was directly loaded on a C-18 column (12g) and was purified using a gradient 0-40% MeCN in ammonium bicarbonate. The desired glucoside was obtained as a white solid after lyophilization: $^1$H NMR (400 MHz, Acetone-$d_6$) δ 7.98 (d, *J* = 2.2 Hz, 1H), 7.83 (s, 1H), 7.57 (dd, *J* = 8.5, 2.4 Hz, 1H), 7.46 (d, *J* = 2.0 Hz, 1H), 7.10 (d, *J* = 8.2 Hz, 1H), 7.03 - 6.54 (m, 3H), 5.82 (d, *J* = 7.9 Hz, 1H), 4.88 - 4.73 (m, 1H), 3.94 - 3.85 (m, 1H), 3.77 (dd, *J* = 11.7, 2.6 Hz, 1H), 3.65 (dd, *J* = 11.8, 4.8 Hz, 1H), 3.57 - 3.36 (m, 6H), 0.86 - 0.69 (m, 3H), 0.68 - 0.59 (m, 1H).

**Compound 3:** (2R,3R,4S,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4,5-triyl triacetate

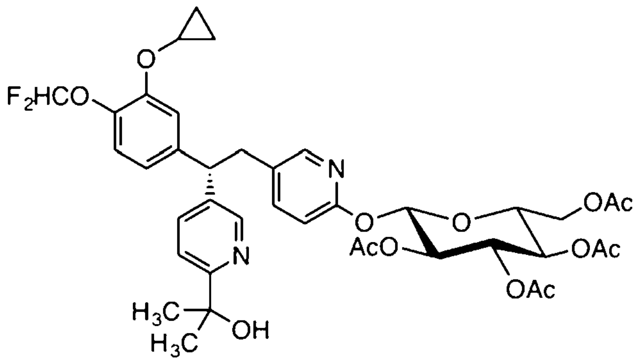

**Compound 3** was prepared by the following procedure: Step 1: (S)-5-(2-(3-cyclopropoxy-4-(difluoromethoxy)phenyl)-2-(6-(2-hydroxypropan-2-yl)pyridin-3-yl)ethyl)pyridin-2(1H)-one. (S)-3-(2-(3-cyclopropoxy-4-(difluoromethoxy)phenyl)-2-(6-(2-hydroxypropan-2-yl)pyridin-3-yl)ethyl)pyridine 1-oxide **IIb** (1.00 g, 2.20 mmol), prepared according to Friesen et al. (J. Med. Chem. 2003, 46(12), 2413), was dissolved in toluene (10 mL) and tetrahydrofuran (1 mL), (10 :1 mixture, 0.2 M). The solution was cooled to 0 °C, and triethylamine (2.46 mL, 17.60 mmol) was added, followed by dropwise addition of trifluoroacetic anhydride (1.48 mL, 8.80 mmol). Once addition was completed, the reaction was warmed to room temperature and stirred for 30 minutes. The reaction was then cooled to 0 °C and 20 mL of saturated aqueous sodium bicarbonate solution was added. The resulting solution was stirred for 2 hours. The solution was then diluted with 200 mL of EtOAc and layers were separated. The organic layer was then washed with aqueous saturated sodium bicarbonate solution (10 mL), water (10 mL) and brine (10 mL). The organic layer was dried over magnesium sulfate, concentrated, and purified by reverse phase column chromatography using 0-40% MeCN in ammonium bicarbonate buffer. The pure fractions were then combined and concentrated to afford the desired pyridone **IIIb** as a white solid.

Step 2: (2R,3R,4S,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4,5-triyl triacetate. To a solution of (S)-5-(2-(3-cyclopropoxy-4-(difluoromethoxy)phenyl)-2-(6-(2-hydroxypropan-2-yl)pyridin-3-yl)ethyl)pyridin-2(1H)-one **IIIb** (50 mg, 0.11 mmol) in toluene (0.2 M) was added silver oxide (1.5 equiv) and 1-bromo-2,3,4,6-tetra-*O*-acetyl-α-D-glucopyranoside (1.5 equiv). The resulting suspension was stirred at 110 °C for 1.5 hours. The reaction was filtered over celite, condensed under reduced pressure and purified by reversed phase column chromatography eluting with 20-80% MeCN in ammonium formate. The desired glucoside was obtained as a white solid after lyophilization: $^1$H NMR (500 MHz, Acetone-$d_6$) δ 8.53 (d, *J* = 1.9 Hz, 1H), 8.01 (d, *J* = 2.0 Hz, 1H), 7.87 (dd, *J* = 8.2, 2.3 Hz, 1H), 7.65 (dd, *J* = 8.4, 2.5 Hz, 1H), 7.60 (d, *J* = 8.1 Hz, 1H), 7.45 (d, *J* = 2.0 Hz, 1H), 7.08 (d, *J* = 8.2 Hz, 1H), 7.00 (dd, *J* = 8.3, 2.1 Hz, 1H), 6.93 - 6.56 (m, 2H), 6.27 (d, *J* = 8.3 Hz, 1H), 5.42 (t, *J* = 9.6 Hz, 1H), 5.21 - 5.09 (m, 2H), 4.49 (t, *J* = 8.1 Hz, 1H), 4.28 (dd, *J* = 12.3, 4.6 Hz, 1H), 4.12 (ddd, *J* = 10.0, 4.6, 2.4 Hz, 1H), 4.04 (dd, J= 12.3, 2.5 Hz, 1H), 3.90 (tt, *J* = 6.0, 2.8 Hz, 1H), 3.54 - 3.43 (m, 2H), 2.02 (s, 3H), 1.97 (s, 6H), 1.90 (s, 3H), 1.46 (s, 6H), 0.87 - 0.70 (m, 3H), 0.67 - 0.60 (m, 1H).

**Compound 4:** (2S,3R,4S,5S,6R)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(hydroxymethyl)tetrahydro-2H-pyran-3,4,5-triol

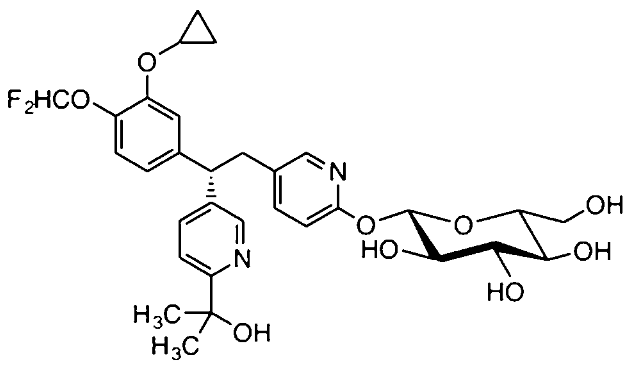

**Compound 4** was prepared by the following procedure: Step 1: (2S,3R,4S,5S,6R)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(hydroxymethyl)tetrahydro-2H-pyran-3,4,5-triol. To a solution of (2R,3R,4S,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4,5-triyl triacetate (15 mg, 0.02 mmol) in THF/Water (1:1, 0.1 M) was added LiOH (15 eq). The solution was stirred at room temperature for 30 minutes and the solution was directly loaded on a C-18 column (12g) and was purified using a gradient 0-40% MeCN in ammonium bicarbonate. The desired glucoside was obtained as a white solid after lyophilization: $^1H$ NMR (500 MHz, Acetone-$d_6$) $\delta$ 8.39 (d, $J$ = 2.1 Hz, 1H), 7.85 (d, $J$ = 2.2 Hz, 1H), 7.72 (dd, $J$ = 8.3, 2.3 Hz, 1H), 7.46 (d, $J$ = 8.1 Hz, 2H), 7.35 (d, $J$ = 2.0 Hz, 1H), 6.95 (d, $J$ = 8.2 Hz, 1H), 6.87 (dd, $J$ = 8.3, 2.0 Hz, 1H), 6.79 - 6.44 (m, 2H), 5.68 (d, $J$ = 8.0 Hz, 1H), 4.35 (t, $J$ = 8.1 Hz, 1H), 3.78 (tt, $J$ = 6.0, 2.9 Hz, 1H), 3.64 (d, $J$ = 9.3 Hz, 1H), 3.52 (dd, $J$ = 11.6, 4.7 Hz, 1H), 3.42 - 3.24 (m, 6H), 1.33 (s, 6H), 0.75 - 0.64 (m, 2H), 0.62 - 0.50 (m, 2H).

**Compound 5:** (2R,3R,4R,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-5-hydroxytetrahydro-2H-pyran-3,4-diyl diacetate

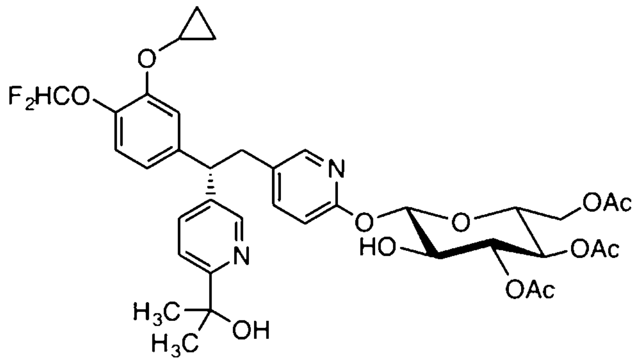

**Compound** 5 was prepared by the following procedure: Step 1: (2R,3R,4S,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-5-(2-iodoacetoxy)tetrahydro-2H-pyran-3,4-diyl diacetate. To a solution of (S)-5-(2-(3-cyclopropoxy-4-(difluoromethoxy)phenyl)-2-(6-(2-hydroxypropan-2-yl)pyridin-3-yl)ethyl)pyridin-2(1H)-one **IIIb** (5 eq) in dichloromethane (0.2M) was added AgOTf (1.2 equiv) at 0 °C.

The solution was stirred at 0 °C for 1 hour. The 1-iodo-2-*O*-iodoacetyl-3,4,6-tetra-*O*-acetyl-$\alpha$-D-glucopyranoside (30 mg, 0.05 mmol), prepared according to Ko et al. (Org. Lett. 2009, 11(3), 609), was then added and the reaction was slowly warmed to room temperature overnight. The solution was filter over celite, condensed under reduced pressure and purified by reverse phase column chromatography eluting with 5-80% MeCN in ammonium formate). The desired glucoside was obtained as a white solid after lyophilization.

Step 2: (2R,3R,4R,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-5-hydroxytetrahydro-2H-pyran-3,4-diyl diacetate. To a solution of (2R,3R,4S,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-5-(2-iodoacetoxy)tetrahydro-2H-pyran-3,4-diyl diacetate (18 mg, 0.02 mmol) in MeOH (0.05 M) was added thiourea (1 equiv). The solution was stirred at room temperature for 40 minutes. The solution was condensed under reduce pressure and purified by reverse phase column chromatography (12g column, 10-80% MeCN in ammonium bicarbonate). The desired glucopyranoside was obtained as a white solid after lyophilization: $^1H$ NMR (500 MHz, Acetone-$d_6$) $\delta$ 8.39 (d, J = 2.0 Hz, 1H), 7.89 (d, J = 2.1 Hz, 1H), 7.73 (dd, J = 8.2, 2.3 Hz, 1H), 7.49 - 7.44 (m, 2H), 7.33 (d, J = 2.1 Hz, 1H), 6.95 (d, J = 8.2 Hz, 1H), 6.87 (dd, J = 8.3, 2.1 Hz, 1H), 6.79 - 6.44 (m, 2H), 5.89 (d, J = 8.1 Hz, 1H), 5.12 (t, J = 9.5 Hz, 1H), 4.86 (dd, J = 11.9, 7.3 Hz, 1H), 4.35 (t, J = 8.1 Hz, 1H), 4.18 - 4.08 (m, 1H), 3.91 - 3.83 (m, 2H), 3.77 (tt, J = 6.1, 2.9 Hz, 1H), 3.68 - 3.58 (m, 1H), 3.41 - 3.26 (m, 2H), 1.87 (s, 3H), 1.86 (s, 3H), 1.82 (s, 3H), 1.33 (s, 6H), 0.75 - 0.62 (m, 2H), 0.62 - 0.49 (m, 2H).

**Compound 6:** (2S,3R,4S,5S,6S)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-6-(methoxycarbonyl)tetrahydro-2H-pyran-3,4,5-triyl triacetate.

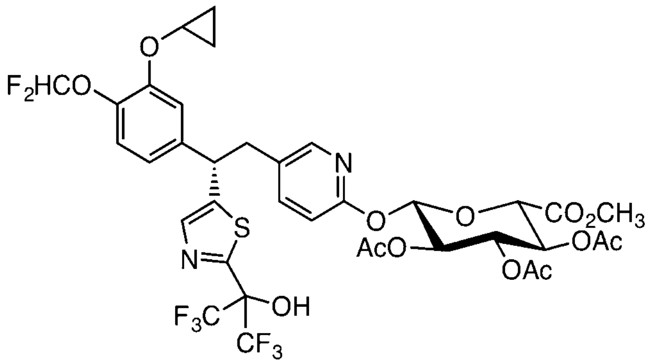

**Compound 6** was prepared by the following procedure: Step 1: (2S,3R,4S,5S,6S)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-6-(methoxycarbonyl)tetrahydro-2H-pyran-3,4,5-triyl triacetate. To a solution of (S)-5-(2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2(1H)-one **IIIa** (100 mg, 0.18 mmol) in toluene (0.18 M) was added silver oxide (1.1 equiv) and methyl 1-bromo-2,3,4-tri-*O*-actetyl-α-D-glucuronate (1.1 equiv). The resulting suspension was stirred at 110 °C for 1 hour. The suspension was then cooled to room temperature, filtered over celite, condensed and purified by reverse phase chromatography (20-80% MeCN in ammonium formate). The desired product was obtained as a white solid after lyophilization. $^1$H NMR (400 MHz, $CDCl_3$) δ 7.80 (d, *J* = 2.2 Hz, 1H), 7.58 (s, 1H), 7.24 - 7.18 (m, 1H), 7.10 (d, *J* = 8.2 Hz, 1H), 7.01 (d, *J* = 2.1 Hz, 1H), 6.76 (dd, *J* = 8.3, 2.1 Hz, 1H), 6.69 - 6.29 (m, 2H), 6.20 (d, *J* = 7.7 Hz, 1H), 5.78 (s, 1H), 5.42 - 5.23 (m, 3H), 4.38 (dd, *J* = 8.8, 6.7 Hz, 1H), 4.23 (d, *J* = 9.6 Hz, 1H), 3.74 - 3.64 (m, 4H), 3.36 (dd, *J* = 13.8, 6.6 Hz, 1H), 3.24 (dd, *J* = 13.8, 9.1 Hz, 1H), 2.04 (m, 6H), 1.97 (s, 3H), 0.85 - 0.59 (m, 4H).

**Compound 7:** (2S,3S,4S,5R,6S)-methyl 6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-3,4,5-trihydroxytetrahydro-2H-pyran-2-carboxylate

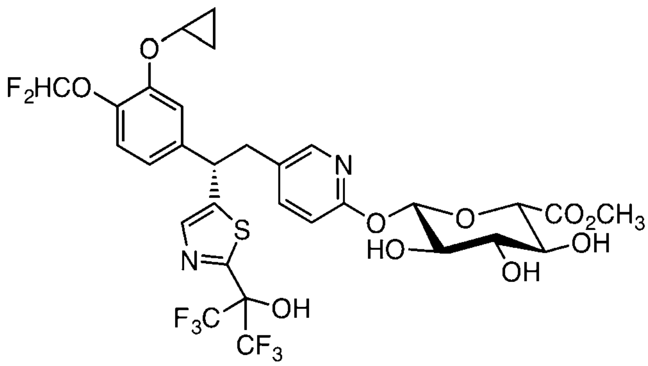

**Compound 7** was prepared by the following procedure: Step 1: lithium (2S,3S,4S,5R,6S)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-3,4,5-trihydroxytetrahydro-2H-pyran-2-carboxylate. To a solution of (2S,3R,4S,5S,6S)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-6-(methoxycarbonyl)tetrahydro-2H-pyran-3,4,5-triyl triacetate (85 mg, 0.1 mmol) in THF/Water (1:1, 0.1 M) was added LiOH (15 eq). The solution was stirred at room temperature for 30 minutes and the solution was directly loaded on a C-18 column (12g) and was purified using a gradient 0-40% MeCN in ammonium bicarbonate. The desired glucuronic acid lithium salt was obtained as a white solid after lyophilization.

Step 2: (2S,3S,4S,5R,6S)-methyl 6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-3,4,5-trihydroxytetrahydro-2H-pyran-2-carboxylate. To a solution of lithium (2S,3S,4S,5R,6S)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-3,4,5-trihydroxytetrahydro-2H-pyran-2-carboxylate (50 mg, 0.07 mmol) in MeOH (0.1 M) at 0 °C was added TMS-diazomethane (5 equiv) and the solution was stirred for 1 hour at 0 °C. The reaction was condensed under reduce pressure and was purified by reversed

phase column chromatography eluting with 0-50% MeCN in ammonium bicarbonate. The desired methyl glucuronate was obtained as a white solid after lyophilization. $^1$H NMR (400 MHz, Acetone-$d_6$) δ 7.98 (s, 1H), 7.83 (d, *J* = 2.5 Hz, 1H), 7.55 (d, *J* = 8.4 Hz, 1H), 7.43 (s, 1H), 7.12 - 7.05 (m, 1H), 7.02 - 6.55 (m, 2H), 6.68 (dd, *J* = 8.4, 3.3 Hz, 1H), 5.93 (dd, *J* = 7.7, 3.1 Hz, 1H), 4.79 (t, *J* = 7.7 Hz, 1H), 3.98 (dd, *J* = 9.5, 2.9 Hz, 1H), 3.93 - 3.82 (m, 1H), 3.70 - 3.63 (m, 4H), 3.58 (td, *J* = 8.7, 2.8 Hz, 1H), 3.55 - 3.47 (m, 2H), 3.47 - 3.38 (m, 1H), 0.87 - 0.68 (m, 3H), 0.67 - 0.58 (m, 1H).

**Compound 8:** (2S,3R,4S,5S,6S)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(methoxycarbonyl)tetrahydro-2H-pyran-3,4,5-triyl triacetate.

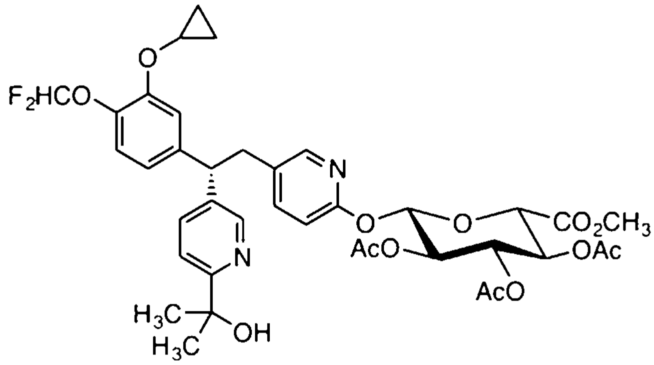

**Compound 8** was prepared by the following procedure: Step 1: (2S,3R,4S,5S,6S)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(methoxycarbonyl)tetrahydro-2H-pyran-3,4,5-triyl triacetate. To a black suspension of (S)-5-(2-(3-cyclopropoxy-4-(difluoromethoxy)phenyl)-2-(6-(2-hydroxypropan-2-yl)pyridin-3-yl)ethyl)pyridin-2(1H)-one **IIIb** (7.00 g, 15.3 mmol) and silver oxide (5.38 g, 23.0 mmol) in toluene (105 mL) was methyl 1-bromo-2,3,4-tri-*O*-acetyl-α-D-glucuronate (9.42 g, 23.0 mmol). The mixture was heated at reflux for 1.5 hr, cooled to r.t., filtered through Celite, washed with ethyl acetate (2 × 30 mL) and concentrated to a dark solid (m ~ 18 g). Purification by flash chromatography using a SNAP Ultra 200 g column and eluting with a gradient mixture of ethyl acetate in hexane afforded the desired glucuronate as a light brown solid as: $^1$H NMR (400 MHz, $CDCl_3$) δ 8.42 (d, *J* = 1.7 Hz, 1H), 7.82 (d, *J* = 2.1 Hz, 1H), 7.59 (dd, *J* = 8.3, 1.7 Hz, 1H), 7.35 (d, *J* = 8.2 Hz, 1H), 7.29 - 7.24 (m, 1H), 7.07 (d, *J* = 8.2 Hz, 1H), 7.02 (d, *J* = 2.0 Hz, 1H), 6.76 (dd, *J* = 8.3, 2.1 Hz, 1H), 6.68 - 6.65 (m, 1H), 6.46 (t, *J* = 75.1 Hz, 1H), 6.20 (d, *J* = 7.7 Hz, 1H), 5.41 - 5.24 (m, 3H), 4.24 (d, *J* = 9.5 Hz, 1H), 4.18 (t, *J* = 7.9 Hz, 1H), 3.72 - 3.67 (m, 1H), 3.67 (s, 3H), 3.30 (d, *J* = 7.9 Hz, 2H), 2.05 (s, 3H), 2.04 (s, 3H), 1.98 (s, 3H), 1.54 (s, 6H), 0.82 - 0.63 (m, 4H).

**Compound 9:** lithium (2S,3S,4S,5R,6S)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate

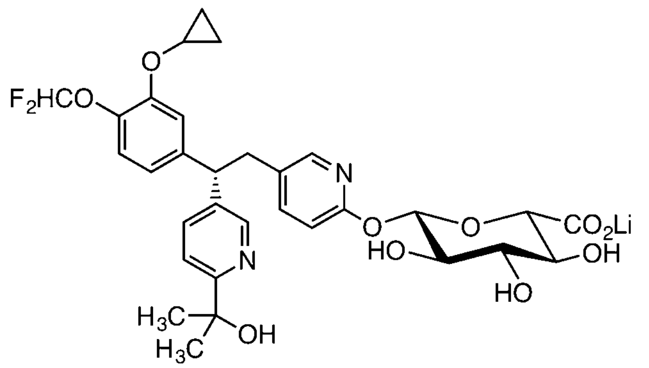

**Compound 9** was prepared by the following procedure: Step 1: To a solution of (2S,3R,4S,5S,6S)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(methoxycarbonyl)tetrahydro-2H-pyran-3,4,5-triyl triacetate (600 mg, 0.77 mmol) in a mixture of THF (3 mL), MeOH (1 mL) and water (1 mL), was added lithium hydroxide (285 mg, 11.6 mmol). The reaction was stirred at r.t. for 30 min and concentrated. Purification by flash chromatography using a SNAP C18 30 g column and eluting with a gradient mixture of acetonitrile and water afforded the desired lithium salt as a white solid after freeze drying. $^1H$ NMR (400 MHz, DMSO-$d_6$) δ 8.47 (d, *J* = 1.9 Hz, 1H), 7.96 (d, *J* = 2.3 Hz, 1H), 7.81 (dd, *J* = 8.3, 2.3 Hz, 1H), 7.61 - 7.52 (m, 2H), 7.42 (d, *J* = 1.9 Hz, 1H), 7.04 (d, *J* = 8.2 Hz, 1H), 6.96 (dd, *J* = 8.3, 2.0 Hz, 1H), 6.93 (t, *J* = 74.7 Hz, 1H), 6.70 (d, *J* = 8.5 Hz, 1H), 5.55 (d, *J* = 7.8 Hz, 1H), 5.14 (s, 1H), 5.10 - 5.04 (m, 1H), 4.94 - 4.86 (m, 1H), 4.39 (t, *J* = 8.1 Hz, 1H), 3.92 (tt, *J* = 6.0, 2.9 Hz, 1H), 3.43 - 3.33 (m, 1H), 3.25 - 3.10 (m, 3H), 3.09 - 3.00 (m, 1H), 1.38 (s, 3H), 1.37 (s, 1H), 0.87 - 0.73 (m, 2H), 0.70 - 0.56 (m, 2H).

**Compound** 10: (2S,3S,4S,5R,6S)-methyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate

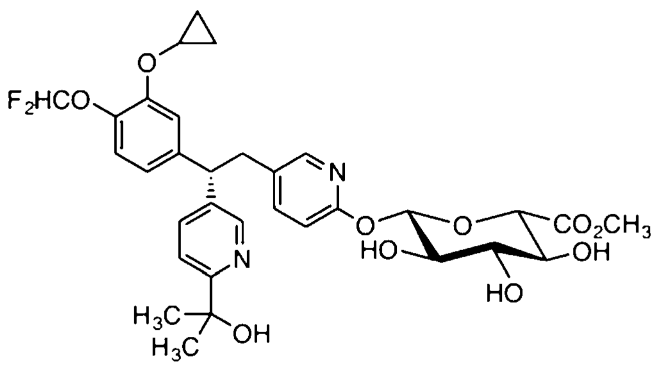

**Compound 10** was prepared by the following procedure: Step 1: (2S,3S,4S,5R,6S)-methyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate. To a solution of (2S,3R,4S,5S,6S)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(methoxycarbonyl)tetrahydro-2H-pyran-3,4,5-triyl triacetate (9.40 g, 12.2 mmol) in MeOH (95 mL) was added sodium methoxide 25wt% (1 mL). The solution was stirred at r.t. for 10 min, neutralized to pH 6-7 with the addition of $NH_4Cl$ (5 mL) and concentrated under reduced pressure. Crude mixture was purified by flash chromatography using a SNAP C18 Ultra 220 g column, eluting with a solvent mixture of acetonitrile and aqueous ammonium formate to afford the desired methyl ester as a light beige solid after freeze drying: $^1H$ NMR (400 MHz, $CDCl_3$) δ 8.33 (d, *J* = 1.2 Hz, 1H), 7.85 (s, 1H), 7.54 (dd, *J* = 8.3, 1.8 Hz, 1H), 7.32 (d, *J* = 8.2 Hz, 1H), 7.21 (d, *J* = 8.6 Hz, 1H), 7.07 (d, *J* = 1.5 Hz, 1H), 7.04 (d, *J* = 8.1 Hz, 1H), 6.77 (dd, *J* = 8.4, 1.3 Hz, 1H), 6.68 (d, *J* = 8.4 Hz, 1H), 6.46 (t, *J* = 75.1 Hz, 1H), 5.76 (d, *J* = 6.5 Hz, 1H), 4.18 (t, *J* = 7.8 Hz, 1H), 4.11 (d, *J* = 9.1 Hz, 1H), 3.84 - 3.68 (m, 4H), 3.67 (s, 3H), 3.33 - 3.17 (m, 2H), 1.50 (s, 3H), 1.49 (s, 3H), 0.80 - 0.62 (m, 4H).

**Compound 11:** (2S,3R,4S,5S,6S)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(ethoxycarbonyl)tetrahydro-2H-pyran-3,4,5-triyl triacetate

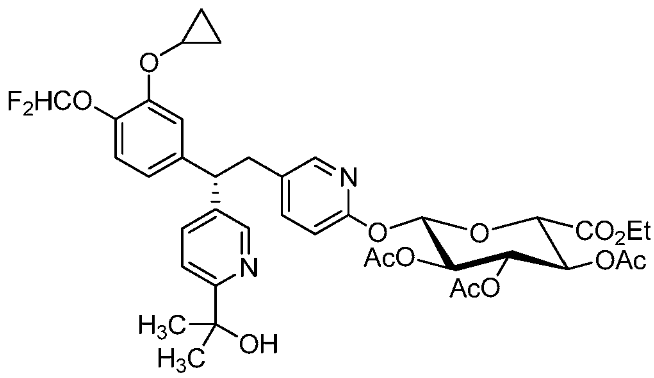

**Compound 11** was prepared by the following procedure: Step 1: (2S,3R,4S,5S,6S)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(ethoxycarbonyl)tetrahydro-2H-pyran-3,4,5-triyl triacetate. To a solution of (S)-5-(2-(3-cyclopropoxy-4-(difluoromethoxy)phenyl)-2-(6-(2-hydroxypropan-2-yl)pyridin-3-yl)ethyl)pyridin-2(1H)-one **IIIb** (100 mg, 0.11 mmol) in toluene (0.2 M) was added silver oxide (1.5 equiv) and ethyl 1-bromo-2,3,4-tri-*O*-acetyl-α-D-glucuronate (1.5 equiv) prepared according to Baddeley *et al.* (*J. Chem. Crystallogr.* **2013,** 33, 33.). The resulting suspension was stirred at 110 °C for 1.5 hours. The reaction was filtered over celite, condensed under reduced pressure and purified by normal phase column chromatography eluting with 20-100% EtOAc in $CH_2Cl_2$. The desired ethyl glucuronate was obtained as a beige solid after lyophilization. $^1H$ NMR (400 MHz, $CDCl_3$) δ 8.39 (d, *J* = 2.1 Hz, 1H), 7.82 (d, *J* = 2.1 Hz, 1H), 7.53 (t, *J* = 2.4 Hz, 1H), 7.31 (dd, *J* = 8.2, 0.7 Hz, 1H), 7.28 - 7.22 (m, 1H), 7.05 (d, *J* = 6.2 Hz, 1H), 7.01 (d, *J* = 2.1 Hz, 1H), 6.76 (dd, *J* = 8.3, 2.1 Hz, 1H), 6.67 - 6.64 (m, 1H), 6.45 (t, *J* = 75.1 Hz, 1H), 6.20 (d, *J* = 7.5 Hz, 1H), 5.41 - 5.23 (m, 3H), 4.78 (s, 1H), 4.21 (d, *J* = 6.0 Hz, 1H), 4.20 - 4.05 (m, 3H), 3.73 - 3.65 (m, 1H), 3.29 (d, *J* = 7.9 Hz, 2H), 2.04 (s, 3H), 2.03 (s, 3H), 1.97 (s, 3H), 1.51 (s, 6H), 1.22 (t, *J* = 7.5 Hz, 3H), 0.82 - 0.63 (m, 4H).

**Compound 12:** (2S,3S,4S,5R,6S)-ethyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate

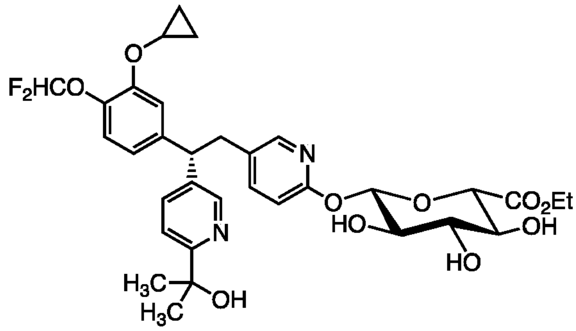

**Compound 12** was prepared by the following procedure: Step 1: (2S,3S,4S,5R,6S)-ethyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate. To a solution of lithium (2S,3S,4S,5R,6S)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate (25 mg, 0.039 mmol) and EDC (17.2 mg, 0.086 mmol) in a mixture of DMF (0.2 mL) and ethanol (0.2 mL), was added DMAP (24.2 mg, 0.196 mmol). The solution was stirred at r.t. for 16 h and concentrated under vacuum. Purification by flash chromatography using a SNAP C18 12 g column, eluting with a solvent mixture of acetonitrile and aqueous ammonium formate afforded the desired ethyl ester as a white solid after freeze drying : $^1H$ NMR (400 MHz, DMSO-$d_6$) δ 8.50 (d, *J* = 2.1 Hz, 1H), 8.01 (d, *J* = 2.3 Hz, 1H), 7.91 - 7.77 (m, 1H), 7.69 - 7.53 (m, 2H), 7.43 (d, *J* = 1.9 Hz, 1H), 7.23 - 6.69 (m, 4H), 5.76 (d, *J* = 7.8 Hz, 1H), 5.50 - 5.25 (m, 3H), 5.17 (s, 1H), 4.42 (q, *J* = 8.0 Hz, 1H), 4.23 - 4.02 (m, 2H), 3.94 (tt, *J* = 6.1, 3.0 Hz, 1H), 3.86 (d, *J* = 9.3 Hz, 1H), 3.54 - 3.22 (m, 7H), 1.41 (d, *J* = 2.5 Hz, 6H), 1.21 (t, *J* = 7.1 Hz, 3H), 0.90 - 0.74 (m, 2H), 0.72 - 0.55 (m, 2H).

**Compound 13:** (2S,3S,4S,5R,6S)-i-propyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate

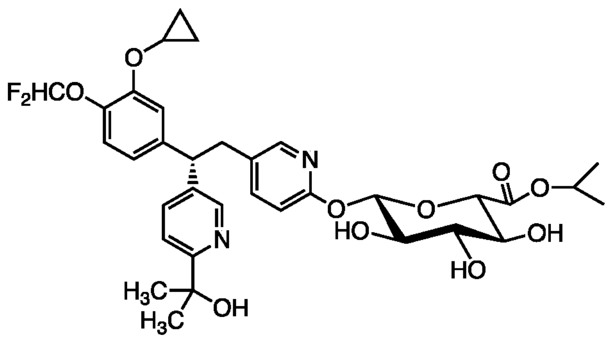

**Compound 13** was prepared by the following procedure: Step 1: (2S,3S,4S,5R,6S)-i-propyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate. To a solution of lithium (2S,3S,4S,5R,6S)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate (110 mg, 0.17 mmol) and EDC (37.8 mg, 0.18 mmol) in a mixture of DMF (0.5 mL) and *i*-propyl alcohol (0.5 mL) was added DMAP (63.8 mg, 0.51 mmol). The solution was stirred at r.t. for 16 h followed by the addition of more DMAP (63.8 mg, 0.51 mmol) and EDC (37.8 mg, 0.18 mmol). The reaction was allowed to stir for 3 days, concentrated under vacuum and purified by flash chromatography using a SNAP C18 12 g column, eluting with a solvent mixture of acetonitrile and aqueous ammonium formate to afford the desired i-propyl ester as a white solid after freeze drying: $^1$H NMR (400 MHz, DMSO-$d_6$) δ 8.51 (t, *J*= 3.9 Hz, 1H), 8.01 (d, *J* = 2.2 Hz, 1H), 7.84 (dt, *J* = 13.0, 6.5 Hz, 1H), 7.68 - 7.54 (m, 2H), 7.43 (d, *J* = 1.9 Hz, 1H), 7.17 - 6.67 (m, 4H), 5.75 (d, *J* = 7.8 Hz, 1H), 5.38 (dd, *J* = 5.2, 3.8 Hz, 2H), 5.27 (d, *J* = 4.8 Hz, 1H), 5.15 (s, 1H), 4.92 (hept, *J* = 6.3 Hz, 1H), 4.43 (t, J = 8.1 Hz, 1H), 4.00 - 3.87 (m, 1H), 3.81 (d, *J* = 9.3 Hz, 1H), 3.42 - 3.21 (m, 5H), 1.41 (d, *J* = 2.4 Hz, 6H), 1.21 (d, *J* = 6.3 Hz, 6 H), 0.90 - 0.74 (m, 2H), 0.74 - 0.57 (m, 2H).

**Compound 14:** (2S,3S,4S,5R,6S)-*tert*-butyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate

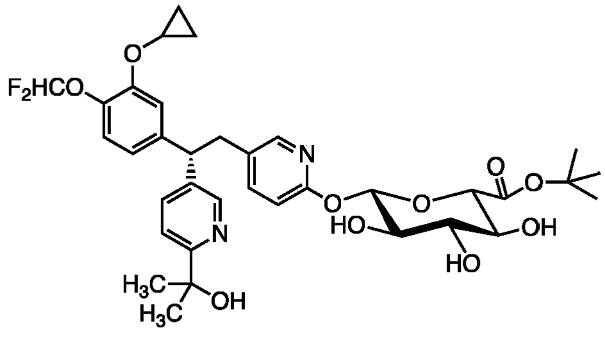

**Compound 14** was prepared by the following procedure: Step 1: (2S,3S,4S,5R,6S)-tert-butyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate. To a solution of lithium (2S,3S,4S,5R,6S)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate (25 mg, 0.031 mmol) and tert-butyl 2,2,2-trichloroacetimidate (57 μL, 0.313 mmol) in 2 mL of $CH_2Cl_2$, was added 4 drops of a stock solution of $BF_3.Et_2O$ (prepared from 0.1 mL of $BF_3.Et_2O$ in 2 mL of $CH_2Cl_2$). The solution was stirred at r.t. for 16 h and an additional 4 drops of the $BF_3.Et_2O$ stock solution was added. The reaction was stirred for an additional 24h and concentrated under reduced pressure. Purification by flash chromatography using a SNAP C18 12 g column, eluting with a solvent mixture of acetonitrile and aqueous ammonium formate afforded the desired tert-butyl ester as a white solid after freeze drying: $^1$H NMR (400 MHz, DMSO-$d_6$) δ 8.42 (d, *J* = 1.9 Hz, 1H), 7.93 (d, *J* = 2.2 Hz, 1H), 7.78 (dd, *J* = 8.4, 2.4 Hz, 1H), 7.71 - 7.41 (m, 2H), 7.35 (d, *J* = 1.8 Hz, 1H), 7.15 - 6.61 (m, 4H), 5.66 (d, *J* = 7.7 Hz, 1H), 5.28 (dd, *J* = 11.5, 5.5 Hz, 2H), 5.18 (d, *J* = 4.9 Hz, 1H), 5.09 (s, 1H), 4.36 (t, *J* = 8.2 Hz, 1H), 3.86 (tt, *J* = 5.9, 2.9 Hz, 1H), 3.64 (d, *J* = 9.2 Hz, 1H), 3.29 (m, 4H), 1.33 (m, 15 H), 0.83 - 0.67 (m, 2H), 0.66 - 0.47 (m, 2H).

**Compound 15:** (2S,3S,4S,5R,6S)-methyl 6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-3,4,5-trihydroxytetrahydro-2H-pyran-2-carboxamide

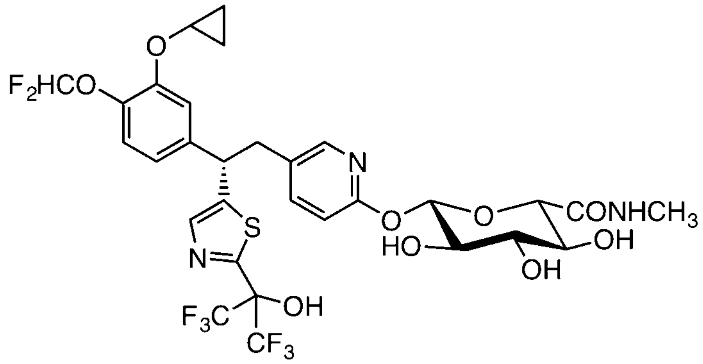

**Compound 15** was prepared by the following procedure: Step 1: (2S,3S,4S,5R,6S)-methyl 6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-3,4,5-trihydroxytetrahydro-2H-pyran-2-carboxamide. (2S,3R,4S,5S,6S)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-6-(methoxycarbonyl)tetrahydro-2H-pyran-3,4,5-triyl triacetate (40 mg, 0.045 mmol) was dissolved in 2M $MeNH_2$ in MeOH (0.1 M). The resulting solution was stirred at room temperature for 20 minutes. The solution was then condensed and purified by reverse phase (0-50% MeCN in ammonium bicarbonate). The desired product was obtained as a white solid after lyophilization: $^1H$ NMR (400 MHz, $CD_3CN$) δ 7.91 (s, 1H), 7.76 (dd, *J* = 2.5, 1.0 Hz, 1H), 7.62 - 7.45 (m, 1H), 7.33 (d, *J* = 1.9 Hz, 1H), 7.09 (d, *J* = 8.2 Hz, 1H), 6.96 - 6.88 (m, 1H), 6.76 (d, *J* = 8.4 Hz, 1H), 6.87 - 6.41 (m, 2H), 5.88 - 5.79 (m, 1H), 4.74 - 4.62 (m, 1H), 3.88 - 3.77 (m, 2H), 3.58 - 3.29 (m, 6H), 2.72 - 2.63 (m, 3H), 0.89 - 0.68 (m, 3H), 0.63 (d, *J* = 6.0 Hz, 1H).

**Compound 16:** (2S,3S,4S,5R,6S)-methyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxamide

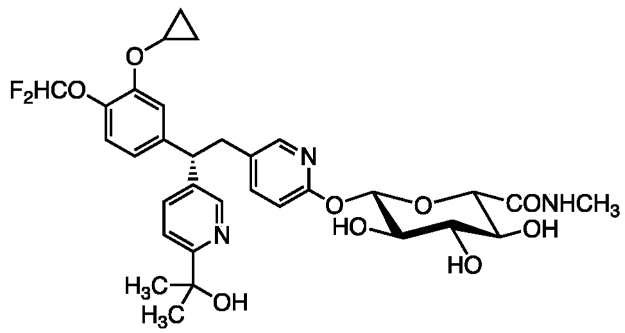

**Compound 16** was prepared by the following procedure: Step 1: (2S,3S,4S,5R,6S)-methyl 6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxamide. To a solution of lithium (2S,3S,4S,5R,6S)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-3,4,5-trihydroxytetrahydro-2H-pyran-2-carboxylate (170 mg, 0.26 mmol) and HATU (114 mg, 0.29 mmol) in 1.5 mL of_DMF was added a 2M methylamine solution in methanol (0.1 mL, 2.9 mmol). The resulting solution was stirred at r.t. for 2 h and purified by flash chromatography using a SNAP C18 30 g column, eluting with a solvent mixture of acetonitrile and aqueous ammonium formate to afford the desired N-methyl carboxamide as a white solid after freeze drying: $^1H$ NMR (400 MHz, DMSO-$d_6$) δ 8.51 (d, *J* = 2.0 Hz, 1H), 8.00 (d, *J* = 2.1 Hz, 1H), 7.95 (q, *J* = 4.5 Hz, 1H), 7.85 (dd, *J* = 8.2, 2.2 Hz, 1H), 7.67 - 7.56 (m, 2H), 7.43 (d, *J* = 1.7 Hz, 1H), 7.17 - 6.67 (m, 4H), 5.77 - 5.67 (m, 1H), 5.33 (d, *J* = 4.1 Hz, 1H), 5.23 (t, *J* = 11.4 Hz, 2H), 5.16

(s, 1H), 4.43 (t, *J* = 8.1 Hz, 1H), 4.01 - 3.88 (m, 1H), 3.67 (t, *J* = 7.4 Hz, 1H), 3.49 - 3.37 (m, 3H), 3.28 (t, *J* = 8.3 Hz, 1H), 2.57 (t, d = 4.3 Hz, 3H), 1.41 (d, *J* = 2.3 Hz, 6H), 0.90 - 0.76 (m, 2H), 0.72 - 0.60 (m, 2H).

**Compound 17:** (2R,3S,4S,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4,5-triyl triacetate

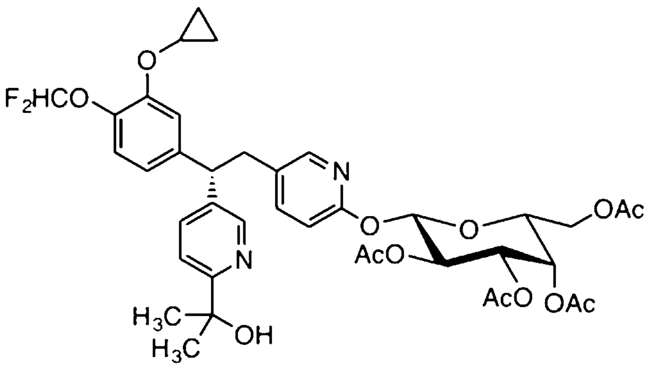

**Compound 17** was prepared by the following procedure: Step 1: (2R,3S,4S,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4,5-triyl triacetate. To a solution of (S)-5-(2-(3-cyclopropoxy-4-(difluoromethoxy)phenyl)-2-(6-(2-hydroxypropan-2-yl)pyridin-3-yl)ethyl)pyridin-2(1H)-one **IIIb** (30 mg, 0.07 mmol) in toluene (0.16 M) was added 1-bromo-2,3,4,6-tetra-*O*-acetyl-$\alpha$-D-galactopyranoside (1.5 equiv) and silver oxide (1.5 equiv). The solution was stirred at 110 °C for 1 hour. The solution was filtered over celite, condensed under reduced pressure and purified by reversed phase column chromatography eluting with 30-100% MeCN in ammonium formate. The desired compound was obtained as a white solid after lyophilization. $^1$H NMR (400 MHz, $CDCl_3$) $\delta$ 8.45 (s, 1H), 7.82 (d, *J* = 2.3 Hz, 1H), 7.64 (d, *J* = 8.2 Hz, 1H), 7.39 (d, *J* = 8.4 Hz, 1H), 7.29 - 7.24 (m, 1H), 7.07 (d, *J* = 8.2 Hz, 1H), 7.01 (d, *J* = 2.0 Hz, 1H), 6.75 (dd, *J* = 8.3, 2.0 Hz, 1H), 6.68 (d, *J* = 8.4 Hz, 1H), 6.46 (t, *J* = 75.0 Hz, 1H), 6.09 (d, *J* = 8.3 Hz, 1H), 5.53 - 5.42 (m, 2H), 5.14 (dd, *J* = 10.4, 3.4 Hz, 1H), 4.19 (t, *J*= 7.9 Hz, 1H), 4.16 - 4.07 (m, 3H), 3.72 - 3.65 (m, 1H), 3.30 (d, *J*= 7.9 Hz, 2H), 2.17 (s, 3H), 2.00 (s, 3H), 2.02 (s, 3H), 1.96 (s, 3H), 1.56 (s, 6H), 0.82 - 0.62 (m, 4H).

**Compound 18:** (2S,3R,4S,5R,6R)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(hydroxymethyl)tetrahydro-2H-pyran-3,4,5-triol

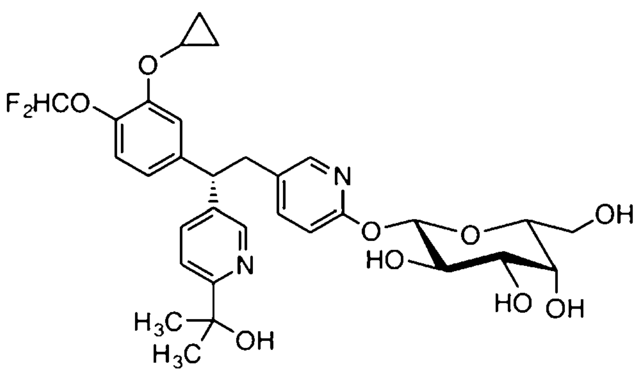

**Compound 18** was prepared by the following procedure: Step 1: (2S,3R,4S,5R,6R)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(hydroxymethyl)tetrahydro-2H-pyran-3,4,5-triol. To a solution of (2R,3S,4S,5R,6S)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4,5-triyl triacetate (10 mg, 0.01 mmol) in THF/Water (1:1, 0.06 M) was added lithium hydroxide (5 equiv). The solution was stirred at room temperature for 10 minutes and the solution was directly loaded on a C18 (12g) and purified using a 0-50%

MeCN in ammonium bicarbonate gradient. The compound was obtained as a white solid after lyophilisation. $^1$H NMR (400 MHz, $CD_3CN$) δ 8.46 (d, *J* = 2.0 Hz, 1H), 7.93 (d, *J* = 2.2 Hz, 1H), 7.77 (dd, *J* = 8.3, 2.2 Hz, 1H), 7.54 (dd, *J* = 8.5, 2.4 Hz, 1H), 7.50 (d, *J* = 8.2 Hz, 1H), 7.35 (d, *J* = 2.0 Hz, 1H), 7.06 (d, *J* = 8.3 Hz, 1H), 6.92 (dd, *J* = 8.3, 2.0 Hz, 1H), 6.73 (d, *J* = 8.4 Hz, 1H), 6.81 - 6.38 (m, 1H), 5.69 (d, *J* = 7.8 Hz, 1H), 4.40 (t, *J* = 8.2 Hz, 1H), 4.28 (s, 1H), 3.88 - 3.80 (m, 2H), 3.71 - 3.54 (m, 5H), 3.49 (s, 1H), 3.43 - 3.32 (m, 3H), 3.19 (s, 1H), 2.87 (s, 1H), 1.46 (s, 6H), 0.90 - 0.60 (m, 4H).

**Compound 19:** (2R,3R,4S,5S,6R)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4,5-triyl triacetate

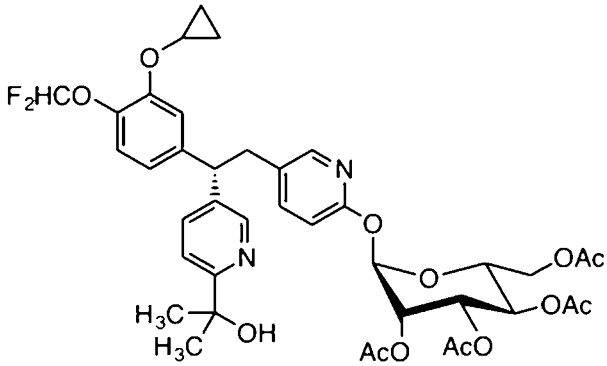

**Compound 19** was prepared by the following procedure: Step 1: (2R,3R,4S,5S,6R)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4,5-triyl triacetate. To a solution of (S)-5-(2-(3-cyclopropoxy-4-(difluoromethoxy)phenyl)-2-(6-(2-hydroxypropan-2-yl)pyridin-3-yl)ethyl)pyridin-2(1H)-one **IIIb** (30 mg, 0.07 mmol) in toluene (0.15 M) was added 1-bromo-2,3,4,6-tetra-*O*-acetyl-α-D-mannopyranoside (1.5 equiv) and silver oxide (1.5 equiv). The solution was stirred at 110 °C for 1 hour. The solution was filtered over celite, condensed under reduced pressure and purified by reversed phase column chromatography eluting with 30-100% MeCN in ammonium formate. The desired compound was obtained as a white solid after lyophilization. $^1$H NMR (400 MHz, $CDCl_3$) δ 8.37 (d, *J* = 2.1 Hz, 1H), 7.86 (d, *J* = 2.2 Hz, 1H), 7.57 - 7.51 (m, 1H), 7.35 - 7.29 (m, 1H), 7.06 (dd, *J* = 9.9, 5.2 Hz, 2H), 6.78 (dd, *J* = 8.3, 2.1 Hz, 1H), 6.70 (d, *J* = 8.4 Hz, 1H), 6.66 - 6.23 (m, 1H), 6.38 (d, *J* = 1.8 Hz, 1H), 5.52 (dd, *J* = 10.1, 3.4 Hz, 1H), 5.43 - 5.36 (m, 2H), 4.24 (dd, *J* = 12.1, 4.6 Hz, 1H), 4.20 - 4.09 (m, 2H), 4.06 (dd, *J* = 12.1, 2.5 Hz, 1H), 3.73 - 3.66 (m, 1H), 3.29 (d, *J* = 7.8 Hz, 2H), 2.19 (s, 3H), 2.05 (s, 3H), 2.02 (s, 3H), 2.00 (s, 3H) 1.52 (s, 6H), 0.81 - 0.66 (m, 4H).

**Compound 20:** (2R,3S,4S,5S,6R)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(hydroxymethyl)tetrahydro-2H-pyran-3,4,5-triol

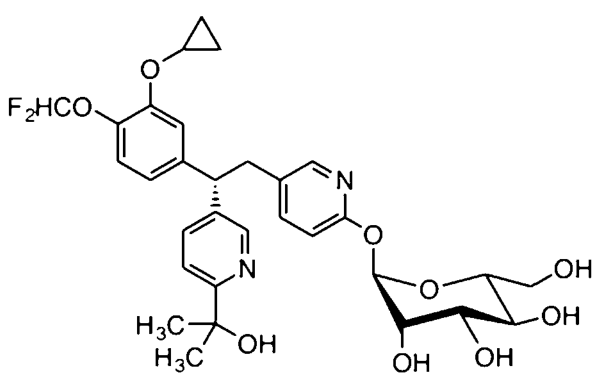

**Compound 20** was prepared by the following procedure: Step 1: (2R,3S,4S,5S,6R)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-6-(hydroxymethyl)tetrahydro-2H-pyran-3,4,5-triol. To a solution of (2R,3R,4S,5S,6R)-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4,5-triyl triacetate (10 mg, 0.01 mmol) in THF/Water (1:1, 0.06 M) was added LiOH (5 equiv). The solution was stirred at room temperature for 10 minutes and the solution was directly loaded on a C18 (12g) and purified using a 0-50% MeCN in ammonium bicarbonate gradient. The compound was obtained as a white solid after lyophilisation. $^1$H NMR (400 MHz, acetone) δ 8.50 (s, 1H), 7.97 (s, 1H), 7.85 (d, *J* = 8.2 Hz, 1H), 7.58 (d, *J* = 8.4 Hz, 2H), 7.47 (s, 1H), 7.03 (dd, *J* = 32.2, 8.3 Hz, 2H), 6.95 - 6.53 (m, 2H), 6.32 (s, 1H), 4.63 (s, 1H), 4.47 (t, *J* = 7.7 Hz, 1H), 4.15 (s, 1H), 3.98 - 3.87 (m, 2H), 3.88 - 3.73 (m, 2H), 3.72 - 3.56 (m, 3H), 3.42 (t, *J* = 18.6 Hz, 3H), 1.51 - 1.41 (m, 7H), 0.87 - 0.59 (m, 4H).

**Compound 21:** (2R,3S,4R,5R)-5-amino-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-2-(hydroxymethyl)tetrahydro-2H-pyran-3,4-diol

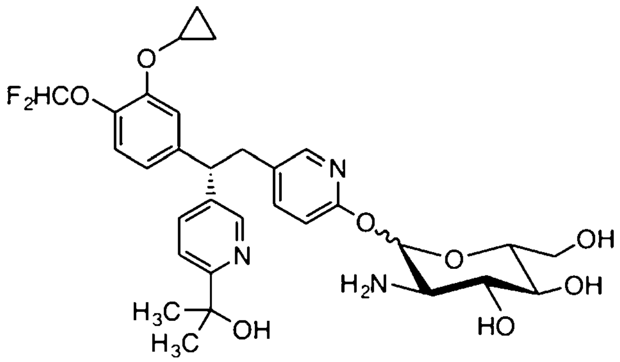

**Compound 21** was prepared by the following procedure: Step 1: (2R,3S,4R,5R)-5-azido-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-2-(hydroxymethyl)tetrahydro-2H-pyran-3,4-diyl diacetate. To a solution of (S)-5-(2-(3-cyclopropoxy-4-(difluoromethoxy)phenyl)-2-(6-(2-hydroxypropan-2-yl)pyridin-3-yl)ethyl)pyridin-2(1H)-one (50 mg, 0.11 mmol) in toluene (0.2 M) was added silver oxide (1.5 equiv) and the 1-chloro-2-azido-3,4,6-tri-*O*-acetyl-α-D-glucopyranoside (1.5 equiv) prepared according to the procedure of St-Pierre et *al.* (*Synthesis* **2016,** 48, 3575.). The resulting suspension was stirred at 110 °C for 1.5 hours. The reaction was filtered over celite, condensed under reduced pressure and purified by reversed phase column chromatography eluting with 20-80% MeCN in ammonium formate. The desired anomeric mixture of azido glucoside was obtained as a white solid after lyophilization.

Step 2: (2R,3S,4R,5R)-5-azido-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-2-(hydroxymethyl)tetrahydro-2H-pyran-3,4-diol. To a solution of (2R,3S,4R,5R)-5-azido-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-2-(hydroxymethyl)tetrahydro-2H-pyran-3,4-diyl diacetate (18 mg, 0.02 mmol) in THF/Water (1:1, 0.1 M) was added LiOH (15 eq). The solution was stirred at room temperature for 30 minutes and the solution was directly loaded on a C-18 column (12g) and was purified using a gradient 10-50% MeCN in ammonium bicarbonate. The desired deacetylated azido glucoside was obtained as a white solid after lyophilization.

Step 3: (2R,3S,4R,5R)-5-amino-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-2-(hydroxymethyl)tetrahydro-2H-pyran-3,4-diol. To a solution of (2R,3S,4R,5R)-5-azido-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-2-(hydroxymethyl)tetrahydro-2H-pyran-3,4-diol (8 mg, 0.01 mmol) in MeOH (0.1 M) was added Pd/C (2 mg, 25% w/w). The suspension was bubbled with hydrogen for 10 minutes and was stirred under a hydrogen atmosphere for 24 hours. The solution was filtered over celite, condensed under reduced pressure and purified by reversed phase column chromatography eluting with 0-40% MeCN in ammonium bicarbonate. A mixture of α and β-D-glucosamine prodrug was obtained as a white solid after lyophilization: $^1$H NMR (500 MHz, $CD_3CN$) δ 8.45 (d, *J* = 2.1 Hz, 1H), 7.99-7.93 (m, 1H), 7.77 (dd, *J* = 8.3, 1.9 Hz, 1H), 7.57-7.52 (m, 1H), 7.50 (d, *J* = 8.2 Hz, 1H), 7.36-7.34 (m, 1H), 7.07 (d, *J* = 8.2 Hz, 1H), 6.94-6.92 (m, 1H), 6.79 - 6.72 (m, 1H), 6.61 (t, *J* = 75.4 Hz, 1H), 6.22 (d, *J* = 3.5 Hz, 0.5H), 5.62 (d, *J* = 8.3 Hz, 0.25H), 4.41 (t, *J* = 8.2 Hz, 1H), 3.89 - 3.82 (m, 1H), 3.67-3.63 (m, 0.25H), 3.59 - 3.50 (m, 3H), 3.44-3.31 (m, 3H), 2.70 (dd, *J* = 9.9, 3.6 Hz, 0.78H), 1.46 (s, 6H), 0.85 - 0.80 (m, 2H), 0.71-0.65 (m, 2H).

**Compound 22:** (2R,3S,4R,5R,6S)-5-amino-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-2-(hydroxymethyl)tetrahydro-2 H-pyran-3,4-diol

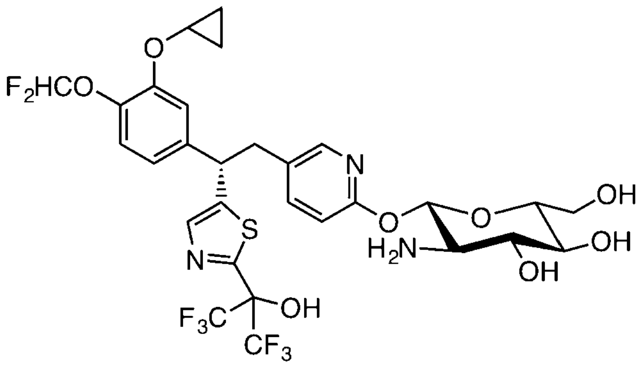

**Compound 22** was prepared by the following procedure: Step 1: (2R,3S,4R,5R,6S)-2-(acetoxymethyl)-5-(((benzyloxy)carbonyl)amino)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3,4-diyl diacetate. To a solution of (S)-5-(2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2(1H)-one **IIIa** (100 mg, 0.18 mmol) in toluene (0.1 M) was added silver oxide (1.5 equiv) and (2R,3S,4R,5R,6R)-2-(acetoxymethyl)-5-(((benzyloxy)carbonyl)amino)-6-chlorotetrahydro-2H-pyran-3,4-diyl diacetate (1.5 equiv) and the solution was stirred at 110°C for 1 hour. The reaction was cooled to room temperature, filtered over celite, condensed and purified by reverse phase chromatography (20-80% MeCN in Ammonium formate). The desired product was obtained as a beige solid after lyophilization.

Step 2: benzyl ((2S,3R,4R,5S,6R)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-4,5-dihydroxy-6-(hydroxymethyl)tetrahydro-2H-pyran-3-yl)carbamate. To a solution of (2R,3S,4R,5R,6S)-2-(acetoxymethyl)-5-(((benzyloxy)carbonyl)amino)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3,4-diyl diacetate (69 mg, 0.08 mmol) in THF/Water (1:1, 0.1 M) was added LiOH (12 equiv). The solution was stirred at room temperature for minutes. The solution was directly loaded on a C18 column (12g) and purified using a 10-70% gradient of MeCN in ammonium bicarbonate. The desired compound was obtained as a white solid after lyophilization.

Step 3: (2R,3S,4R,5R,6S)-5-amino-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-2-(hydroxymethyl)tetrahydro-2H-pyran-3,4-diol. To a solution of benzyl ((2S,3R,4R,5S,6R)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-4,5-dihydroxy-6-(hydroxymethyl)tetrahydro-2H-pyran-3-yl)carbamate (108 mg, 0.12 mmol) in MeOH (0.1 M) was added Pd/C (20% wt) and the solution was stirred over an hydrogen atmosphere for 16 hours. The solution was filtered over celite, condensed and purified by reverse phase chromatography (0-50% MeCN in ammonium bicarbonate). The desired glucosamine was obtained as a white solid after lyophilization. $^1$H NMR (400 MHz, $CD_3CN$) δ 7.91 (d, *J* = 2.3 Hz, 1H), 7.73 (s, 1H), 7.50 (dd, *J* = 8.5, 2.5 Hz, 1H), 7.32 (d, *J* = 2.1 Hz, 1H), 7.08 (d, *J* = 8.2 Hz, 1H), 6.91 (dd, *J* = 8.3, 2.1 Hz, 1H), 6.73 (d, *J* = 8.4 Hz, 1H), 6.85 - 6.41 (m, 1H), 5.63 (d, *J* = 8.3 Hz, 1H), 4.73 - 4.61 (m, 1H), 3.89 - 3.79 (m, 1H), 3.76 - 3.68 (m, 1H), 3.60 (dd, *J* = 11.8, 4.8 Hz, 1H), 3.51 - 3.39 (m, 1H), 3.39 - 3.27 (m, 4H), 2.78 - 2.70 (m, 1H), 0.94 - 0.67 (m, 3H), 0.67 - 0.55 (m, 1H).

**Compound 23:** (2R,3S,4R,5R,6S)-5-amino-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-2-(hydroxymethyl)tetrahydro-2H-pyran-3,4-diol

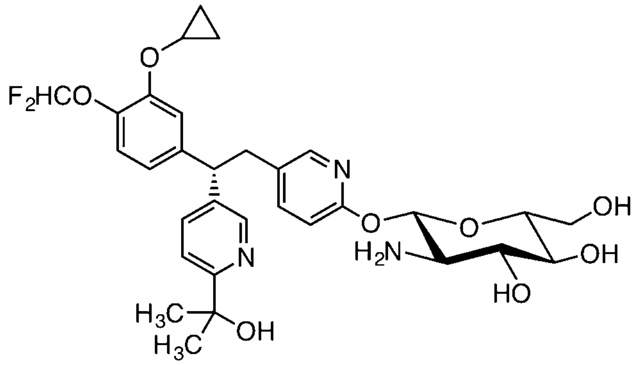

**Compound 23** was prepared by the following procedure: Step 1: (2R,3S,4R,5R,6S)-2-(acetoxymethyl)-5-(((benzyloxy)carbonyl)amino)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4-diyl diacetate. To a solution of (S)-5-(2-(3-cyclopropoxy-4-(difluoromethoxy)phenyl)-2-(6-(2-hydroxypropan-2-yl)pyridin-3-yl)ethyl)pyridin-2(1H)-one **IIIb** (25 mg, 0.05 mmol) in toluene (0.1 M) was added silver oxide (1.5 equiv) and (2R,3S,4R,5R,6R)-2-(acetoxymethyl)-5-(((benzyloxy)carbonyl)amino)-6-chlorotetrahydro-2H-pyran-3,4-diyl diacetate (1.5 equiv) and the solution was stirred at 110°C for 1 hour. The reaction was cooled to room temperature, filtered over celite, condensed and purified by reverse phase chromatography (20-80% MeCN in Ammonium formate). The desired product was obtained as a beige solid after lyophilization.

Step 2: benzyl ((2S,3R,4R,5S,6R)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-4,5-dihydroxy-6-(hydroxymethyl)tetrahydro-2H-pyran-3-yl)carbamate. To a solution of (2R,3S,4R,5R,6S)-2-(acetoxymethyl)-5-(((benzyloxy)carbonyl)amino)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3,4-diyl diacetate (23 mg, 0.03 mmol) in THF/Water (1:1, 0.1 M) was added LiOH (12 equiv). The solution was stirred at room temperature for minutes. The solution was directly loaded on a C18 column (12g) and purified using a 10-70% gradient of MeCN in ammonium bicarbonate. The desired compound was obtained as a white solid after lyophilization.

Step 3: (2R,3S,4R,5R,6S)-5-amino-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-2-(hydroxymethyl)tetrahydro-2H-pyran-3,4-diol. To a solution of benzyl ((2S,3R,4R,5S,6R)-2-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-4,5-dihydroxy-6-(hydroxymethyl)tetrahydro-2H-pyran-3-yl)carbamate (14 mg, 0.02 mmol) in MeOH (0.1 M) was added Pd/C (20% wt) and the solution was stirred over an hydrogen atmosphere for 2 hours. The solution was filtered over celite, condensed and purified by reverse phase chromatography (0-50% MeCN in ammonium bicarbonate). The desired glucosamine was obtained as a white solid after lyophilization. $^1$H NMR (400 MHz, $CD_3CN$) $\delta$ 8.46 (d, *J* = 1.9 Hz, 1H), 7.92 (d, *J* = 2.0 Hz, 1H), 7.77 (dd, *J* = 8.3, 2.2 Hz, 1H), 7.55 (dd, *J* = 8.5, 2.4 Hz, 1H), 7.50 (d, *J* = 8.2 Hz, 1H), 7.35 (d, *J* = 1.9 Hz, 1H), 7.06 (d, *J* = 8.2 Hz, 1H), 6.92 (dd, *J* = 8.3, 2.0 Hz, 1H), 6.72 (d, *J* = 8.5 Hz, 1H), 6.82 - 6.37 (m, 1H), 5.61 (d, *J* = 8.2 Hz, 1H), 4.40 (t, *J* = 8.3 Hz, 1H), 4.29 (s, 1H), 3.85 (tt, *J* = 6.0, 2.8 Hz, 1H), 3.70 (d, *J* = 11.8 Hz, 1H), 3.59 (d, *J* = 8.7 Hz, 1H), 3.43 - 3.23 (m, 5H), 2.71 (t, *J* = 8.7 Hz, 2H), 1.45 (s, 6H), 0.92 - 0.74 (m, 2H), 0.76 - 0.61 (m, 2H).

**Compound 24:** (2R,3S,4R,5R,6S)-5-acetamido-2-(acetoxymethyl)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3,4-diyl diacetate

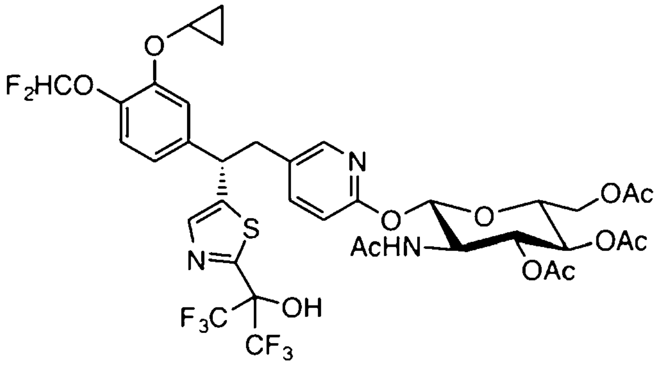

**Compound 24** was prepared by the following procedure: Step 1: (2R,3S,4R,5R,6S)-5-acetamido-2-(acetoxymethyl)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3,4-diyl diacetate. To a solution of (2R,3S,4R,5R,6S)-5-amino-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-2-(hydroxymethyl)tetrahydro-2H-pyran-3,4-diol (169 mg, 0.23 mmol) in pyridine (0.2 M) was added acetic anhydride (4 equiv) and the resulting solution was stirred at room temperature overnight. The solution was loaded directly on a c-18 column (40g) and was purified using 20-80% MeCN in ammonium formate. The desired product was obtained as a white solid after lyophilization. $^1$H NMR (400 MHz, $CD_3CN$) δ 7.88 (d, *J* = 2.2 Hz, 1H), 7.80 - 7.74 (m, 1H), 7.57 - 7.46 (m, 1H), 7.32 - 7.24 (m, 1H), 7.12 - 7.04 (m, 1H), 6.97 - 6.86 (m, 1H), 6.85 - 6.39 (m, 3H), 6.21 - 6.09 (m, 1H), 5.36 - 5.26 (m, 1H), 5.10 - 4.98 (m, 1H), 4.73 - 4.62 (m, 1H), 4.24 - 4.17 (m, 1H), 4.17 - 4.06 (m, 1H), 4.05 - 3.98 (m, 1H), 3.95 - 3.87 (m, 1H), 3.86 - 3.78 (m, 1H), 3.50 - 3.40 (m, 1H), 3.40 - 3.30 (m, 1H), 2.01 - 1.99 (m, 3H), 1.99 - 1.93 (m, 6H), 1.77 - 1.71 (m, 3H), 0.88 - 0.67 (m, 3H), 0.56 (s, 1H).

**Compound 25:** *N*-((2S,3R,4R,5S,6R)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-4,5-dihydroxy-6-(hydroxymethyl)tetrahydro-2H-pyran-3-yl)acetamide

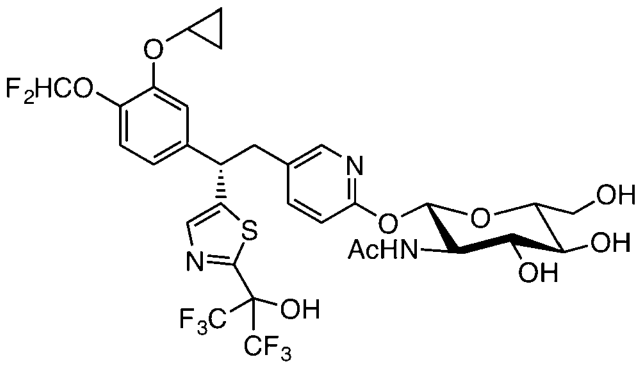

**Compound 25** was prepared by the following procedure: Step 1: N-((2S,3R,4R,5S,6R)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-4,5-dihydroxy-6-(hydroxymethyl)tetrahydro-2H-pyran-3-yl)acetamide. To a solution of (2R,3S,4R,5R,6S)-5-amino-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-2-(hydroxymethyl)tetrahydro-2H-pyran-3,4-diol (76 mg, 0.10 mmol) in MeOH (0.1 M) was added $Et_3N$ (3 equiv) followed by acetic anhydride (3 equiv) at 0°C. The solution was stirred at room temperature for 5 minutes. The solution was condensed and purified by reverse phase chromatography (0-100% MeCN in water). The desired product was obtained as a white solid after lyophilization. $^1$H NMR (500 MHz, $CD_3CN$) δ 7.88 (d, *J* = 2.1 Hz, 1H), 7.76 (d, *J* = 0.8 Hz, 1H), 7.52 (dd, *J* = 8.5, 2.5 Hz, 1H), 7.31 (d, *J* = 2.1 Hz, 1H), 7.09 (d, *J* = 8.3 Hz, 1H), 6.92 (dd, *J* = 8.3, 2.1 Hz, 1H), 6.68 (d, *J* = 8.4 Hz, 1H), 6.82 - 6.45 (m, 2H), 5.89 (d, *J* = 8.8 Hz, 1H), 4.74 - 4.64 (m, 1H), 3.87 - 3.80 (m, 2H), 3.76 - 3.71 (m, 1H), 3.61 (dd, *J* = 11.9, 4.9 Hz, 1H), 3.56 - 3.50 (m, 1H), 3.47 - 3.32 (m, 4H), 1.81 (s, 3H), 0.88 - 0.69 (m, 3H), 0.66 - 0.56 (m, 1H).

**Compound 26:** (2S,3R,4R,5S,6R)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-4,5-dihydroxy-6-(hydroxymethyl)-*N*,*N*,*N*-trimethyltetrahydro-2H-pyran-3-aminium iodide

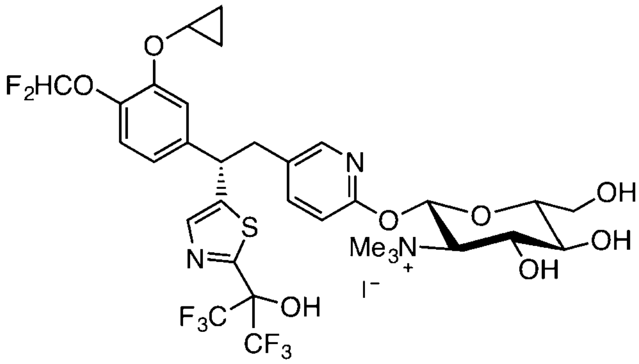

**Compound 26** was prepared by the following procedure: Step 1: (2S,3R,4R,5S,6R)-2-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-4,5-dihydroxy-6-(hydroxymethyl)-*N*,*N*,*N*-trimethyltetrahydro-2H-pyran-3-aminium iodide. To a solution of (2R,3S,4R,5R,6S)-5-amino-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-2-(hydroxymethyl)tetrahydro-2H-pyran-3,4-diol (4 mg. 0.01 mmol) in THF (0.05 M) was added MeI (5 equiv) followed by $(iPr)_2NEt$ (5 equiv). The reaction was left to stir overnight at room temperature. The solution was condensed and purified by reverse phase chromatography (0-100% MeCN in ammonium formate). The desired quaternary amine was obtained as a white solid after lyophilization. $^1H$ NMR (400 MHz, $CD_3CN$) $\delta$ 8.35 (s, 1H), 7.94 (d, *J* = 2.2 Hz, 1H), 7.68 (s, 1H), 7.55 (dd, *J* = 8.5, 2.4 Hz, 1H), 7.36 (d, *J* = 2.0 Hz, 1H), 7.10 (d, *J* = 8.2 Hz, 1H), 6.93 (dd, *J* = 8.3, 2.0 Hz, 1H), 6.78 (d, *J* = 8.4 Hz, 1H), 6.85 - 6.40 (m, 1H), 6.60 (d, *J* = 3.4 Hz, 1H), 4.74 - 4.59 (m, 1H), 4.04 (t, *J* = 8.1 Hz, 1H), 3.94 (t, *J* = 9.1 Hz, 1H), 3.88 - 3.80 (m, 1H), 3.71 - 3.59 (m, 3H), 3.56 (dd, *J* = 11.1, 5.5 Hz, 1H), 3.40 (qd, *J* = 14.0, 8.2 Hz, 2H), 3.25 (s, 9H), 0.88 - 0.69 (m, 3H), 0.67 - 0.59 (m, 1H).

**Compound 27:** (2R,3S,4R,5R,6S)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-5-(dimethylamino)-2-(hydroxymethyl)tetrahydro-2H-pyran-3,4-diol

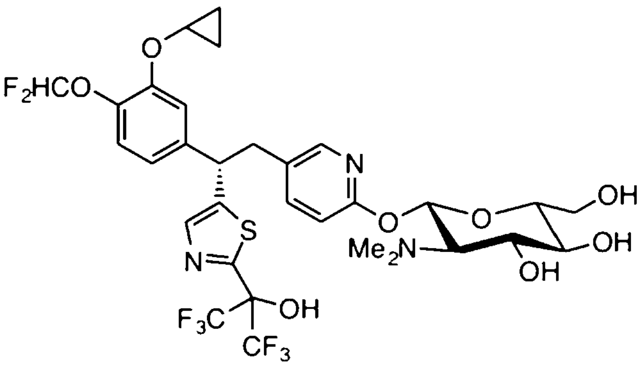

**Compound 27** was prepared by the following procedure: Step 1: (2R,3S,4R,5R,6S)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-5-(dimethylamino)-2-(hydroxymethyl)tetrahydro-2H-pyran-3,4-diol. To a solution of (2R,3S,4R,5R,6S)-5-amino-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-2-(hydroxymethyl)tetrahydro-2H-pyran-3,4-diol (84 mg, 0.11 mmol) in MeOH (0.1 M) was added formaldehyde (10 equiv) followed by sodium cyanoborohydride (3 equiv) and the resulting solution was stirred overnight at room temperature. The solution was condensed and purified by reverse phase chromatography using a 10-50% MeCN

in ammonium bicarbonate. The desired *N*,*N*-dimethyl glucosamine was obtained as a white solid after lyophilization. $^1$H NMR (400 MHz, Acetone-$d_6$) δ 7.97 (s, 1H), 7.84 (dd, *J* = 1.9, 0.4 Hz, 1H), 7.58 (dd, *J* = 8.4, 2.5 Hz, 1H), 7.43 (d, *J* = 2.2 Hz, 1H), 7.10 (dd, *J* = 8.2, 2.5 Hz, 1H), 7.04 - 6.54 (m, 3H), 6.12 (dd, *J* = 8.7, 2.7 Hz, 1H), 4.80 (t, *J* = 7.9 Hz, 1H), 3.88 (dt, *J* = 8.9, 3.0 Hz, 1H), 3.75 (d, *J* = 11.6 Hz, 1H), 3.70 - 3.27 (m, 6H), 2.54 - 2.43 (m, 1H), 2.44 - 2.37 (m, 6H), 0.88 - 0.67 (m, 3H), 0.67 - 0.55 (m, 1H).

**Compound 28:** (2R,3R,4S,5R,6S)-2-(acetoxymethyl)-6-(((2R,3R,4S,5R,6S)-4,5-diacetoxy-2-(acetoxymethyl)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3-yl)oxy)tetrahydro-2H-pyran-3,4,5-triyl triacetate.

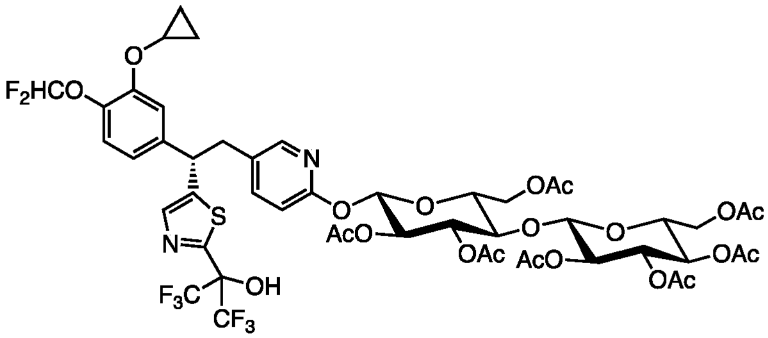

**Compound 28** was prepared by the following procedure: Step 1: (2R,3R,4S,5R,6S)-2-(acetoxymethyl)-6-(((2R,3R,4S,5R,6S)-4,5-diacetoxy-2-(acetoxymethyl)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3-yl)oxy)tetrahydro-2H-pyran-3,4,5-triyl triacetate. To a solution of (S)-5-(2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2(1H)-one **IIIa** (152 mg, 0.27 mmol) in 5.4 mL of toluene, was added (2R,3R,4S,5R,6R)-2-(acetoxymethyl)-6-(((2R,3R,4S,5R,6R)-4,5-diacetoxy-2-(acetoxymethyl)-6-bromotetrahydro-2H-pyran-3-yl)oxy)tetrahydro-2H-pyran-3,4,5-triyl triacetate (280 mg, 0.4 mmol) and silver oxide (185 mg, 0.8 mmol). The resulting black suspension was refluxed for 2 hours, cooled to r.t., filtered through Celite, washed with ethyl acetate (2 × 20 mL) and concentrated to a dark solid. Purification by flash chromatography using a SNAP C18 12 g column, eluting with a solvent mixture of acetonitrile and water afforded the desired disaccharide polyacetate as a brown solid: 1H NMR (400MHz, $CDCl_3$) δ 7.78 (d, *J* = 2.4 Hz, 1H), 7.57 (s, 1H), 7.22 (dd, *J* = 8.4, 2.4 Hz, 1H), 7.11 (d, *J* = 8.3 Hz, 1H), 7.03 (d, *J* = 1.8 Hz, 1H), 6.78 (dd, *J* = 8.2, 1.9 Hz, 1H), 6.66 (d, *J* = 8.4 Hz, 1H), 6.50 (t, *J* = 74.9 Hz, 1H), 6.06 (d, *J* = 8.2 Hz, 1H), 5.84 (s, 1H), 5.30 (t, *J* = 9.1 Hz, 1H), 5.25 - 5.04 (m, 3H), 4.95 (t, *J* = 8.5 Hz, 1H), 4.53 (d, *J* = 7.9 Hz), 4.51 - 4.45 (m, 1H), 4.43 - 4.35 (m, 2H), 4.12 (dd, *J* = 12.2, 4.4 Hz, ), 4.07 (dd, *J* = 12.4,H 1.8 Hz, 1H), 3.91 (t, *J*= 9.3 Hz, 1H), 3.84 - 3.76 (m, 1H), 3.74 - 3.61 (m, 2H), 3.36 (dd, *J*= 14.0, 6.7 Hz, 1H), 3.26 (dd, *J* = 13.8, 8.9 Hz, 1H), 2.11 (s, 3H), 2.09 (s, 3H), 2.05 (s, 6H), 2.02 (s, 3H), 2.00 (s, 3H), 1.97 (s, 3H), 0.90 - 0.59 (m, 4H).

**Compound 29:** (2S,3R,4S,5S,6R)-2-(((2R,3S,4R,5R,6S)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-4,5-dihydroxy-2-(hydroxymethyl)tetrahydro-2H-pyran-3-yl)oxy)-6-(hydroxymethyl)tetrahydro-2H-pyran-3,4,5-triol

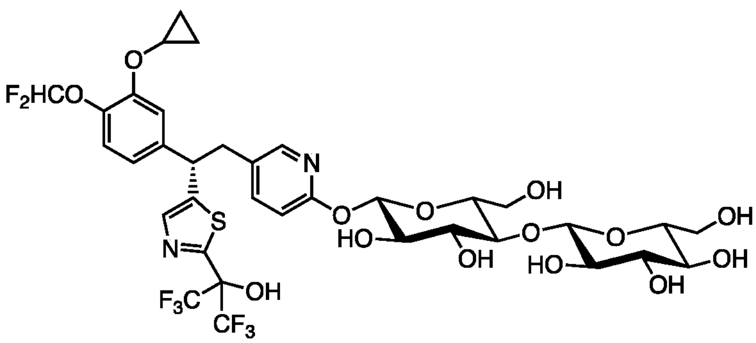

**Compound 29** was prepared by the following procedure: Step 1: (2S,3R,4S,5S,6R)-2-(((2R,3S,4R,5R,6S)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)-4,5-dihydroxy-2-(hydroxymethyl)tetrahydro-2H-pyran-3-yl)oxy)-6-(hydroxymethyl)tetrahydro-2H-pyran-3,4,5-triol. To a solution of (2R,3R,4S,5R,6S)-2-(acetoxymethyl)-6-(((2R,3R,4S,5R,6S)-4,5-diacetoxy-2-(acetoxymethyl)-6-((5-((S)-2-(3-cyclopropoxy-4-(difluoromethoxy)phenyl)-2-(2-(1,1,1,3,3,3-hexafluoro-2-hydroxypropan-2-yl)thiazol-5-yl)ethyl)pyridin-2-yl)oxy)tetrahydro-2H-pyran-3-yl)oxy)tetrahydro-2H-pyran-3,4,5-triyl triacetate (56 mg, 0.047 mmol) in THF/MeOH/Water (0.9/0.3/0.3 mL) was added lithium hydroxide (31.2 mg, 0.71 mmol). The resulting solution was stirred at room temperature for 1 hour and concentrated under reduced pressure. Purification by flash chromatography using a SNAP C18 12 g column, eluting with a solvent mixture of acetonitrile and water afforded the desired disaccharide as a white solid: $^{1}H$ NMR (400MHz, DMSO-$d_6$) δ 7.92 (s, 1H), 7.60 (bs, 1 H), 7.55 (dd, *J* = 8.7, 1.9 Hz, 1H), 7.32 (s, *J* = 11.7 Hz, 1H), 7.03 (d, *J* = 8.0 Hz, 1H), 6.95 (t, *J* = 74.6 Hz, 1H), 6.88 (d, *J* = 7.4 Hz, 1H), 6.70 (d, *J* = 8.4 Hz, 1H), 5.69 (d, *J* = 8.1 Hz, 1H), 5.58 - 5.07 (m, 2H), 5.03 - 4.87 (m, 1H), 4.82 - 4.55 (m, 3H), 4.29 (d, *J* = 7.7 Hz, 1H), 3.94 - 3.81 (m, 1H), 3.74 - 3.55 (m, 3H), 3.50 - 3.15 (m, 12H), 3.12 - 2.96 (m, 2H), 0.86 - 0.70 (m, 2H), 0.70 - 0.61 (m, 1H), 0.59 - 0.49 (m, 1H).

**Compound 30:** (2R,3R,4S,5R,6S)-2-(acetoxymethyl)-6-(((2R,3R,4S,5R,6S)-4,5-diacetoxy-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3-yl)oxy)tetrahydro-2H-pyran-3,4,5-triyl triacetate

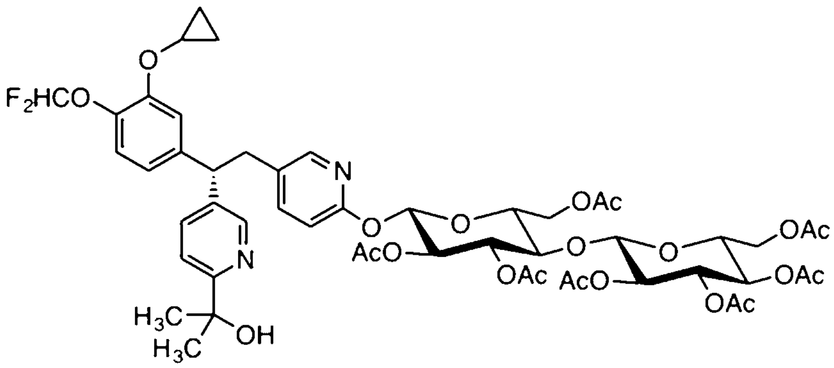

**Compound 30** was prepared by the following procedure: Step 1: (2R,3R,4S,5R,6S)-2-(acetoxymethyl)-6-(((2R,3R,4S,5R,6S)-4,5-diacetoxy-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3-yl)oxy)tetrahydro-2H-pyran-3,4,5-triyl triacetate. To a solution of (S)-5-(2-(3-cyclopropoxy-4-(difluoromethoxy)phenyl)-2-(6-(2-hydroxypropan-2-yl)pyridin-3-yl)ethyl)pyridin-2(1H)-one **IIIb** (85 mg, 0.19 mmol) in toluene (0.16 mmol) was added (2R,3R,4S,5R,6R)-2-(acetoxymethyl)-6-(((2R,3R,4S,5R,6R)-4,5-diacetoxy-2-(acetoxymethyl)-6-bromotetrahydro-2H-pyran-3-yl)oxy)tetrahydro-2H-pyran-3,4,5-triyl triacetate (1.5 equiv) followed by silver oxide (1.5 equiv). The resulting suspension was stirred at 110°C for 1.5 hours. The reaction was filtered over celite, condensed under reduced pressure and purified by reversed phase column chromatography eluting with 20-70% MeCN in ammonium formate. The desired product was obtained as a white solid after lyophilization. $^{1}H$ NMR (400 MHz, $CDCl_3$) δ 8.39 (d, *J* = 2.1 Hz, 1H), 7.79 (d, *J* = 2.1 Hz, 1H), 7.55 (d, *J* = 8.3 Hz, 1H), 7.33 (d, *J* = 8.2 Hz, 1H), 7.23 (dd, *J* = 8.5, 2.4 Hz, 1H), 7.07 (d, *J* = 8.2 Hz, 1H), 7.01 (d, *J* = 2.1 Hz, 1H), 6.76 (dd, *J* = 8.3, 2.1 Hz, 1H), 6.64 (s, 1H), 6.45 (t, *J* = 70.5 Hz, 1H), 6.04 (d, *J* = 8.1 Hz, 1H), 5.32 - 5.25 (m, 1H), 5.22 - 5.03 (m, 3H), 4.94 (dd, *J* = 9.1, 8.0 Hz, 1H), 4.51 (d, *J* = 7.9 Hz, 1H), 4.46 (dd, *J* = 12.1, 1.9 Hz, 1H), 4.38 (dd, *J* = 12.5, 4.4 Hz, 1H), 4.20 - 4.08 (m, 2H), 4.05 (dd, *J* = 12.4, 2.2 Hz, 1H), 3.94 - 3.85 (m, 1H), 3.80 (ddd, *J* = 9.8, 4.3, 2.0 Hz, 1H), 3.67 (tdd, *J* = 6.7, 5.0, 2.8 Hz, 2H), 3.28 (d, *J* = 7.9 Hz, 2H), 2.10 (s, 3H), 2.06 (s, 3H), 2.04 (s, 6H), 2.01 (s, 3H), 1.98 (s, 3H), 1.95 (s, 3H), 1.52 (s, 6H), 0.80 - 0.63 (m, 4H).

**Compound 31:** (2S,3R,4S,5S,6R)-2-(((2R,3S,4R,5R,6S)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-4,5-dihydroxy-2-(hydroxymethyl)tetrahydro-2H-pyran-3-yl)oxy)-6-(hydroxymethyl)tetrahydro-2H-pyran-3,4,5-triol

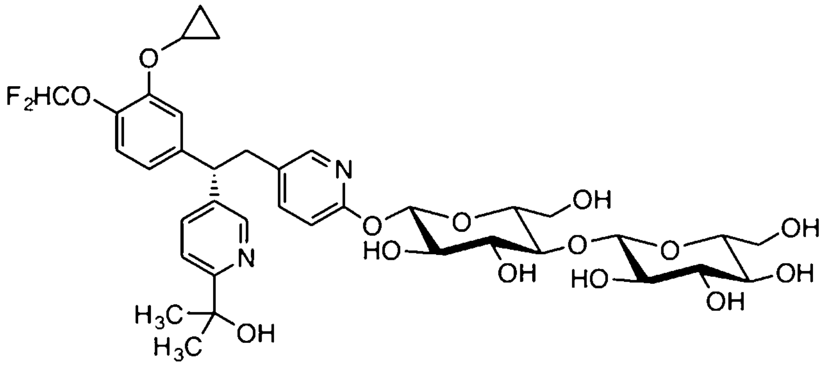

**Compound 31** was prepared by the following procedure: Step 1: (2S,3R,4S,5S,6R)-2-(((2R,3S,4R,5R,6S)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)-4,5-dihydroxy-2-(hydroxymethyl)tetrahydro-2H-pyran-3-yl)oxy)-6-(hydroxymethyl)tetrahydro-2H-pyran-3,4,5-triol. To a solution of (2R,3R,4S,5R,6S)-2-(acetoxymethyl)-6-(((2R,3R,4S,5R,6S)-4,5-diacetoxy-2-(acetoxymethyl)-6-((5-((S)-2-(3-cyclopropoxy-4-(difluoromethoxy)phenyl)-2-(6-(2-hydroxypropan-2-yl)pyridin-3-yl)ethyl)pyridin-2-yl)oxy)tetrahydro-2H-pyran-3-yl)oxy)tetrahydro-2H-pyran-3,4,5-triyl triacetate (50 mg, 0.05 mmol) in THF/Water (1/1, 0.1M) was added lithium hydroxide (15 equiv). The resulting solution was stirred at room temperature for 1 hour. The solution was then directly loaded on a C18 column (30g) and purified using a 10-60% MeCN in ammonium bicarbonate gradient. The desired disaccharide was obtained as a white solid after lyophilization. $^1$H NMR (400 MHz, CD3CN) $\delta$ 8.45 (d, J = 1.9 Hz, 1H), 7.93 (d, J = 2.1 Hz, 1H), 7.76 (dd, J = 8.3, 2.3 Hz, 1H), 7.54 (dd, J = 8.4, 2.3 Hz, 1H), 7.50 (d, J = 8.2 Hz, 1H), 7.35 (d, J = 2.0 Hz, 1H), 7.06 (d, J = 8.2 Hz, 1H), 6.92 (dd, J = 8.3, 2.0 Hz, 1H), 6.72 (d, J = 8.5 Hz, 1H), 6.81 - 6.39 (m, 1H), 5.80 (d, J = 8.1 Hz, 1H), 4.41 (dd, J = 8.0, 4.3 Hz, 2H), 3.90 - 3.16 (m, 21H), 1.45 (s, 6H), 0.90 - 0.77 (m, 2H), 0.75 - 0.59 (m, 2H).

## EXAMPLE 2

## ASSAYS DEMONSTRATING BIOLOGICAL ACTIVITY

Inhibitory potency measurement assay protocol against PDE4 isozymes. Potency ($IC_{50}$) of compounds which inhibit the hydrolysis of cAMP to AMP by the type-IV cAMP-specific phosphodiesterases was measured using the following protocol: the serial dilution of the test compounds was first performed in 100% DMSO. Each intermediate compound dilution (in 100% DMSO) was then diluted 10-fold into assay buffer for an intermediate DMSO concentration of 10%. 5 $\mu$l of this dilution were added to a 50 $\mu$l reaction to obtain 1% DMSO in all the reactions. The enzymatic reactions were conducted at room temperature for 60 minutes in a 50 $\mu$l mixture containing PDE assay buffer, 100 nM FAM-cAMP, a PDE enzyme (PDE4A1A 2 ng/reaction, PDE4B1 0.04 ng/reaction, PDE4D2 0.013 ng/reaction) and the test compound. After the enzymatic reaction, 100 $\mu$l of a binding solution (1:100 dilution of the binding agent with the binding agent diluent) was added to each well and the plate was incubated for additional 15 minutes. Fluorescence intensity was measured at an excitation of 470 nm and an emission of 528 nm using a Tecan Infinite M1000 microplate reader. PDE activity assays were performed in duplicate at each concentration. Fluorescence intensity was converted to fluorescence polarization using the Tecan Magellan6 software. The fluorescence polarization data were analyzed using the computer software Graphpad Prism. The fluorescence polarization (FPt) in absence of the compound in each data set was defined as 100% activity. In the absence of PDE and the compound, the value of fluorescent polarization ($FP_b$) in each data set was defined as 0% activity. The percent activity in the presence of the compound was calculated according to the following equation: % activity = $(FP-FP_b)/(FP_t-FP_b)\times 100\%$, where FP= the fluorescence polarization in the presence of the compound. The values of % activity versus a series of compound concentrations were then plotted using non-linear regression analysis of Sigmoidal dose-response curve generated with the equation $Y=B+(T-B)/1+10^{((LogEC50-X)\times \text{Hill Slope})}$, where Y=percent activity, B=minimum percent activity, T=maximum percent activity, X= logarithm of compound and Hill Slope=slope factor or Hill coefficient. The $IC_{50}$ value was determined by the concentration at half-maximal percent activity. Parent PDE4 inhibitors $IC_{50}$ values should be less than about 1000 nM, preferably less than about 100 nM, and even more preferable less than about 10 nM. The $IC_{50}$ values measured for parent PDE4 inhibitor compounds **IIIa** and **IIIb** against the isozyme PDE4A 1A, PDE4B1 and PDE4D2 were less than 10 nM. $IC_{50}$ values of PDE4 inhibitor glycosides represented by Formula (**I**) should be more than 10-fold the $IC_{50}$ value of its corresponding parent PDE4 inhibitor, preferably 100-fold the $IC_{50}$ value of its corresponding parent PDE4 inhibitor and advantageously 1000-fold the $IC_{50}$ value of its corresponding parent PDE4 inhibitor. The $IC_{50}$ value measured for **compound 22**, was more than 100 nM even though it was contaminated with 0.17% of **IIIa**. Such a contamination, based on the

potency of **IIIa** and the mathematical projection (100/0.17 × $IC_{50}$ of **IIIa**), accounts for 100% of the potency recorded for **compound 22.**

TNFα inhibition assay protocol. Potency of PDE4 inhibitors in the LPS-induced TNFα assay in human whole blood was measured using the following protocol: fresh blood is collected in heparinized tubes by venipuncture from healthy human volunteers (male and female). These subjects should not have apparent inflammatory conditions, no symptoms of bacterial/viral infection, no fever and should not take any NSAIDs for at least 1 week prior to blood collection. Blood from each donor was dispensed in a 96-deep well plate, 500μL per well. Blood was pre-incubated with either 2 μL of vehicle (DMSO) or test compound at 37°C /5% $CO_2$ for 15 min. This was followed by addition of 10 μL lipopolysaccharide from *E. coli* serotype 0111:B4 (LPS, Sigma-Aldrich, Saint-Louis, MO, USA) diluted in 0.1% bovine serum albumin fraction V (BSA, Sigma-Aldrich, Saint-Louis, MO, USA) in phosphate buffered saline (PBS) at 37°C /5% $CO_2$ for 24 h at a final concentration of 1 μg/mL. Duplicate independent incubations were performed for each inhibitor concentration with blood from each donor. Appropriate PBS controls (no LPS) were used as blanks (4 wells) while blood samples stimulated with LPS controls (no PDE4 inhibitor) served as positive controls (4 wells). Once incubation finished, samples were centrifuged at 1500xg at 4°C for 10 min. Plasma was recovered (approximately 200 μL per well) and kept at -80°C for analysis by ELISA. Plasma TNFα was quantified by ELISA (Invitrogen, Frederick, MD, USA) following the manufacturer's instructions. In each case positive control values from the same patient, on the same plate, were used in calculation of % inhibition. From each blood assay sample, 4 μL of plasma were diluted in 196 μL in diluent buffer (1:50 dilution), which provided optical density values (OD) within the linear portion of the standard curve (data not shown). Assay plates were read on an Infinite F200 PRO-Tecan reader at 450nm. Absorbance values were converted to % inhibition based on positive and negative control values on each plate, and resulting % inhibition data were fit to a four-parameter logistic by nonlinear regression using GraphPad Prism (version 6.00, GraphPad Software, Inc., La Jolla, CA, USA). $IC_{50}$ values reported are those from the four parameter fit, the concentration of test inhibitor giving half-maximum TNFα inhibition. Using blood from 12 healthy volunteers, the average $IC_{50}$ values measured for compound **IIIa and IIIb** was less than 50 nM.

*In vitro* glycoside prodrug permeability assessment. The permeability of the glycoside prodrugs were measured using the bidirectional Caco-2 assay. Caco-2 cells were seeded onto permeable polycarbonate supports in 12-well Costar Transwell plates and allowed to grow and differentiate for 21-25 days. On day 24, culture medium (DMEM supplemented with 10% FBS, 1% non-essential amino acids and penicillin/streptomycin) was removed from both sides of the transwell inserts and cells were rinsed with warm HBSS. After the rinse step, the chambers were filled with warm transport buffer (apical: HBSS containing 25 mM MES, 0.25% BSA, pH 6.0; Basolateral: HBSS containing 25 mM HEPES, 0.25% BSA, pH 7.4) and the plates were incubated at 37°C for 30 min prior to TEER (Trans Epithelial Electric Resistance) measurements. The buffer in the donor chamber (apical side for A-to-B assay, basolateral side for B-to-A assay) was removed and replaced with the working solution (10 μM test article in transport buffer). The plates were then placed at 37°C under light agitation. At designated time points (30, 60 and 90 min); an aliquot of transport buffer from the receiver chamber was removed and replenished with fresh transport buffer. Samples were quenched with ice-cold $CH_3CN$ containing internal standard and then centrifuged to precipitate the protein. Resulting supernatants were further diluted with 50/50 ACN/$H_2O$ ($H_2O$ only for Atenolol) and submitted for LC/MS/MS analysis. Reported apparent permeability (Papp) represents the average of 2 determinations. Atenolol and propranolol were tested as low and moderate permeability references. Bidirectional transport of digoxin was assessed to demonstrate P-gp activity/expression. The apparent permeability ($P_{app}$, measured in cm/s) of a compound is determined according to the following formula:

$$P_{app} = \frac{(dQ)/(dt)}{60(A \times C_0)}$$

Where dQ/dt is the net rate of appearance in the receiver compartment, A is the area of the Transwell measured in $cm^2$ (1.12 $cm^2$), $C_0$ is the initial concentration of compound added to the donor chamber and 60 is the conversion factor for the minute to second. As the glycoside prodrugs aim at delivering the active PDE4 inhibitor in the colonic compartment of the gastrointestinal tract (GIT), minimal absorption of the compound of Formula (**I**) from the upper GIT is desirable. As the correlation between Caco-2 $P_{app}$ and *in vivo* intestinal absorption have been demonstrated (Artursson P.; Karlsson J. 1991 Biochem. Biophys. Res. Commun. 175 (3), 880), the permeability of the glycoside prodrugs, as measured by the A-to-B $P_{app}$, should be inferior the corresponding parent PDE4 inhibitor, preferably a measured $P_{app}$ less than $1 \times 10^{-6}$cm/sec and even more preferable less than $0.1 \times 10^{-6}$cm/sec. Glycoside prodrugs of Formula (**I**) were found to have a Caco-2 $P_{app}$ inferior to their corresponding PDE4 inhibitors **III** (Table 2).

**Table 2**

| Cmpd | A-to-B $P_{app}$ ($X10^{-6}$cm/sec) |
|---|---|
| **IIIa** | 19.1 |
| **IIIb** | 8.6 |
| **2** | 0.07 |
| **3** | 1.4 |
| **4** | 0.08 |
| **5** | 0.9 |
| **7** | 1.0 |
| **8** | 0.6 |
| **10** | 0.3 |
| **15** | 1.5 |
| **18** | 0.1 |
| **20** | 0.2 |
| **22** | 0.2 |
| **26** | 2.8 |
| **27** | 2.4 |
| **30** | 0.5 |
| **31** | 0.02 |

*In vitro* rate of glycoside prodrugs hydrolysis. The metabolic stability of prodrug candidates to release the parent PDE4 inhibitor could be assessed *in vitro* according, but not restricted, to the following protocol: feces were collected overnight on a mixture of dry and wet ice in a metabolic cage. Ten volumes (v/w; 30 mL) of 100 mM phosphate buffer (pH 6.5) were added to 3 g of freshly collected rodent feces. The mixture was homogenized with a Stomacher Circulator 400 (2 × 1 min-cycles at 225 rpm). The mixture was transferred to a 50 mL tubes, centrifuged at 10 000xg for 10 min (4°C) and the supernatant was passed through a 0.45 μm-filter. In a 96 deep-well plate, 198 μL of feces supernatant were aliquoted in 2 wells per compound. Each compound, including positive control, was tested in duplicate. Then 2 μL of 10 mM stock solution of each test compound were spiked in individual wells (duplicate) at a final concentration of 100 μM and samples were sealed and mixed 30 sec at 1000 rpm using a vortexer. Samples were incubated at 37°C for the desired time in the Thermomixer with constant agitation (300 rpm). At desired time-point, 25 μL of reaction mixture was added to 225 μL of ice-cold quenching solution (acetonitrile + 0,1% (v/v) formic acid) to stop the bacterial enzymatic activity. The quenched incubates were mixed and centrifuged 10 min at 21 000xg at 4°C. A real $t_0$ (no metabolism, 100% total recovery of unchanged prodrug) was generated by adding 22.5 μL of feces supernatant to 225 μL of ice-cold quenching solution (acetonitrile + 0,1% (v/v) formic acid). Then 2.5 μL of 1 mM solution of each test compounds was added, sealed, mixed thoroughly and centrifuged 10 min at 21 000x*g* at 4°C. All samples, including analytical standards, were analyzed by LC-MS/MS to measure the concentration of the parent PDE4 inhibitor released and the concentration of the corresponding prodrug remaining. A set criteria of 100% recovery is targeted where the summation of the % of prodrug remaining and the % of parent PDE4 inhibitor released should be 100% with an experimental error tolerance of ± 20%. When prodrugs represented by Formula (**I**) were incubated 24 hours under the conditions described above using mouse, rat and dog feces, the glycoside prodrugs were hydrolyzed to release the corresponding parent PDE4 inhibitor **III** (Table 3). As a negative control, when **compound 4, 10** and **21** were incubated 24 hours under the conditions described above but in the absence of enzyme activities, using mouse feces extract supernatant inactivated by boiling the supernatant 10 minutes, 100 % of the prodrugs were recovered unchanged. As a positive control, when **compound 9** and **10** were incubated for 24 hours in a phosphate buffer (pH 6.5) at 37°C in the presence of β-D-glucuronidase (50U/mL), 85% and 37% of **IIIb** were respectively released while 2% of **compound 9** and 64% of **compound 10** were recovered unchanged.

Table 3

| Cmpd | Species | Prodrug Remaining @ 24 hr (%) | Parent Recovered @ 24 hr (%) |
|---|---|---|---|
| **2** | Rat | 0.5 | 110 |
| **3** | Rat | 0.1 | 109 |
| **4** | Mouse | 0.1 | 95 |
| | Rat | 0.0 | 97 |
| **5** | Mouse | 0.0 | 96 |
| | Rat | 0.0 | 105 |
| **6** | Rat | 23 | 58 |
| **7** | Rat | 35 | 70 |
| **9** | Mouse | 3.3 | 81 |
| | Rat | 4.2 | 102 |
| **10** | Mouse | 74 | 27 |
| | Rat | 47 | 57 |
| | Dog | 67 | 50 |
| **15** | Rat | 74 | 30 |
| **17** | Rat | 0.0 | 99 |
| **18** | Rat | 0.0 | 102 |
| **19** | Rat | 0.7 | 102 |
| **20** | Rat | 0.0 | 116 |
| **22** | Mouse | 62 | 30 |
| **23** | Mouse | 65 | 21 |
| | Dog | 75 | 45 |
| **26** | Mouse | 96 | 0.0 |
| **27** | Rat | 93 | 16 |
| **30** | Rat | 10 | 72 |
| **31** | Rat | 0.6 | 122 |

*In vivo* glycoside prodrugs bioactivation. *In vivo* pharmacokinetic experiments were performed using pre-clinical species such as but not restricted to mice and rats. After oral dosing of the PDE4 inhibitor glycosides of Formula (**I**), distribution of the prodrug and of the corresponding parent PDE4 inhibitor in function of time was assessed in tissues such as blood and intestines. The amount of prodrug and corresponding parent excreted in feces was also measured. This will allow the investigators to assess the *in vivo* bioactivation of the glycoside prodrug to release the parent PDE4 inhibitor and the extent of colon specificity for the glycoside prodrug delivery system. Advantageously, the parent PDE4 inhibitor should not be released before reaching the colonic compartment of the GIT and the prodrug should not be absorbed in the upper part of the GIT. More advantageously, the local exposure of the parent PDE4 inhibitor measured in feces and/or colon should be superior to the parent systemic exposure measured in blood. When **compound 10** was orally administered to rats as an aqueous suspension, no prodrug or its corresponding metabolite **compound 9** were detected in blood over 24 hr. In the same study, the corresponding PDE4 inhibitor **IIIb** was not detected in blood before T = 2 hours which represent the orocaecal transit time. Over 24 hours, the extent of prodrug bioactivation, as measured in excreted feces by the ratio of parent PDE4 inhibitor **IIIb** over the residual prodrug **10,** was 96%. When **compound 10** was orally administered to C57Bl/6 mice as a labrasol/5%dextrose solution, the extent of prodrug bioactivation over 48 hours, as measured in excreted feces, was >98% with only trace amount of unchanged prodrug detected. 81% of the administered dose of **compound 10** was excreted in feces as the parent PDE4 inhibitor **IIIb.** In the same study, the measured exposure ($AUC_{0-24}$) of released parent PDE4 inhibitor **IIIb** in the colonic tissues (proximal and distal colon) was 200-fold superior to the one measured in blood.

The emetic threshold of the PDE4 inhibitor glycosides can be assessed as a measure of tolerability improvement compared to the corresponding parent PDE4 inhibitor. In this observational model, animals such as but not restricted to dogs, ferrets or non-human primates are dosed with the PDE4 inhibitor glycoside or its corresponding parent PDE4 inhibitor at equivalent doses, and emesis is recorded. Improvement in tolerability would be defined as a ratio of the PDE4 inhibitor glycoside emetic dose over the corresponding parent PDE4 inhibitor emetic dose >3, advantageously this ratio would be >10, even more advantageously this ratio would be >30.

## Claims

1. A compound of Formula (**I**):

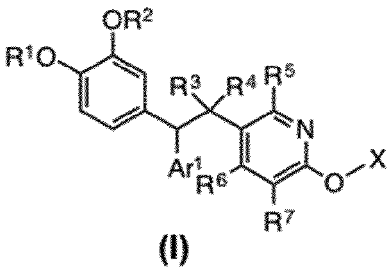

**(I)**

or a pharmaceutically acceptable salt thereof, wherein

X is a *β*-D-glucuronide, an *α*-D-glucuronide, a *β*-D-glucopyranoside, an *α*-D-glucopyranoside, a *β*-D-galactopyranoside, an *α*-D-galactopyranoside, a *β*-D-mannopyranoside, an *α*-D-mannopyranoside, an *N*-acetyl-*β*-D-glucosaminide, an *N*-acetyl-*α*-D-glucosaminide, an *N*-acetyl-*β*-D-galactosaminide, an *N*-acetyl-*α*-D-galactosaminide, a *β*-D-glucosaminide, an *α*-D-glucosaminide, a *β*-D-galactosaminide, an *α*-D-galactosaminide, a *β*-D-fucopyranoside, an *α*-L-fucopyranoside, an *α*-L-rhamnopyranoside, an *α*-L-arabinofuranoside, a *β*-D-ribofuranoside, a *β*-D-cellobioside, an *α*-D-cellobioside, a *β*-*N*,*N*-diacetyl chitobioside, a D-xylopyranoside, a D-xylofuranoside, a *β*-D-galacturonide or an *α*-D-galacturonide;
$R^1$ and $R^2$ are each independently -$C_{1-6}$alkyl, -$C_{3-6}$cycloalkyl, any of which is unsubstituted or substituted with 1-6 independent halogen;
$R^3$ and $R^4$ are each independently H, or-$C_{1-6}$alkyl;
$R^5$, $R^6$ and $R^7$ are each independently H, halogen, -$C_{1-6}$alkyl, -C(O)$C_{1-6}$alkyl, or CN;
$Ar^1$ is independently selected from the group consisting of:

(a) 6-$R^8$-3-pyridyl or 6-$R^9$-3-pyridyl,
(b) 2-$R^8$-5-thiazolyl or 5-$R^8$-2-thiazolyl,
(c) 2-$R^8$-5-pyrimidinyl or 2-$R^9$-5-pyrimidinyl,
(d) 6-$R^8$-3-pyridazinyl or 6-$R^9$-3-pyridazinyl,
(e) 5-$R^8$-2-furyl,
(f) 5-$R^8$-2-thienyl,
(g) 2-$R^8$-5-oxazolyl or 5-$R^8$-2-oxazolyl,
(h) 5-$R^8$-3-isoxazolyl or 3-$R^8$-5-isoxazolyl,
(i) 5-$R^8$-3-isothiazolyl or 3-$R^8$-5-isothiazolyl, and
(j) *p*-$R^8$-phenyl;

$R^8$ is selected from the group consisting of: H, halogen, -$C_{1-6}$alkyl, -$C_{3-6}$cycloalkyl, -$C_{1-6}$alkyl$Ar^2$, $Ar^2$, $C_{1-6}$alkoxy, $C_{1-6}$alkylthio, CN, -C($R^{10}$)($R^{11}$)OH, -C($R^{10}$)($R^{11}$)O$C_{1-6}$alkyl, -C($R^{10}$)($R^{11}$)O$Ar^2$, -$CO_2$H, -$CO_2C_{1-6}$alkyl, -C(O)N$R^{12}R^{13}$, -$SO_2$NHC(O)$Ar^2$, -C(O)$C_{1-6}$alkyl and -C(O)$Ar^2$;
$R^9$ is selected from the group consisting of: -N$R^{12}R^{13}$, -N$R^{12}$C(O)$R^{13}$, - N$R^{12}$C(O)NH$R^{13}$, -N$R^{12}SO_2Ar^2$, and -N$R^{12}CO_2Ar^2$;
$R^{10}$ and $R^{11}$ are each independently H, -$C_{1-6}$alkyl, -$C_{1-6}$haloalkyl, -$C_{3-6}$cycloalkyl, or $Ar^2$;
or when $R^{10}$ and $R^{11}$ are -$C_{1-6}$alkyl, they may connect together through a $C_{1-3}$alkyl to form $C_{3-6}$cycloalkyl;
$R^{12}$ and $R^{13}$ are each independently H, -$C_{1-6}$alkyl, -$C_{3-6}$cycloalkyl, or -$C_{1-6}$alkyl$Ar^2$;
or when $R^{12}$ and $R^{13}$ are -$C_{1-6}$alkyl, they may connect together through a $C_{1-3}$alkyl to form a $C_{3-6}$heterocycloalkyl;
$Ar^2$ is selected from the group consisting of: phenyl, pyridinyl, quinolinyl, isoquinolinyl, pyridazinyl, pyrimidinyl,

pyrazinyl, quinoxalinyl, furyl, benzofuryl, dibenzofuryl, thienyl, benzothienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl, and tetrazolyl;

each $Ar^2$ being unsubstituted or substituted with 1-3 members selected from the group consisting of: halo, $-C_{1-6}$alkyl, $-C_{1-6}$haloalkyl, CN, $C_{1-6}$alkoxy, $C_{1-6}$alkylthio, $-C(R^{10})(R^{11})OH$, $-CO_2H$, $-CO_2C_{1-6}$alkyl, $-C(O)NR^{12}R^{13}$, and $-SO_2CH_3$.

**2.** The compound according to claim 1, or a pharmaceutically acceptable salt thereof, wherein the $\beta$-D-glucuronide is $\beta$-D-glucuronic acid, methyl $\beta$-D-glucuronidate, methyl 2,3,4-triO-acetyl-$\beta$-*D*-glucuronidate, ethyl 2,3,4-tri-*O*-acetyl-$\beta$-D-glucuronidate, ethyl $\beta$-D-glucuronidate, *i*-propyl $\beta$-D-glucuronidate, *tert*-butyl $\beta$-D-glucuronidate, or methyl $\beta$-D-glucuronamide;

wherein the $\beta$-D-glucopyranoside is $\beta$-D-glucopyranosyl, 2,3,4,6-tetra-O-acetyl-$\beta$-D-glucopyranosyl, or 3,4,6-tri-O-acetyl-$\beta$-D-glucopyranosyl;

wherein the $\beta$-D-galactopyranoside is $\beta$-D-galactopyranosyl, or 2,3,4,6-tetra-O-acetyl-$\beta$-D-galactopyranosyl;

wherein the $\alpha$-D-mannopyranoside is $\alpha$-D-mannopyranosyl, or 2,3,4,6-tetra-*O*-acetyl-$\alpha$-D-mannopyranosyl;

wherein the $\beta$-D-glucosaminide is $\beta$-D-glucosaminyl, $\alpha$-D-glucosaminyl, *N*,*N*,*N*-trimethyl-$\beta$-D-glucosaminyl, or *N*,*N*-dimethyl-$\beta$-D-glucosaminyl;

wherein the *N*-acetyl-$\beta$-D-glucosaminide is *N*-acetyl-$\beta$-D-glucosaminyl, or 3,4,6-tri-*O*-acetyl-*N*-acetyl-$\beta$-D-glucosaminyl; and

wherein the $\beta$-D-cellobioside is $\beta$-D-cellobiosyl, or 2,3,6,2',3',4',6'-hepta-*O*-acetyl-$\beta$-D-cellobiosyl.

**3.** The compound according to any one of claims 1 - 2, or a pharmaceutically acceptable salt thereof, wherein $Ar^1$ is 6-$R^8$-3-pyridyl or 2-$R^8$-5-thiazolyl.

**4.** The compound according to any one of claims 1 - 2, or a pharmaceutically acceptable salt thereof, wherein $Ar^1$ is 6-$R^8$-3-pyridyl.

**5.** The compound according to claim 4, or a pharmaceutically acceptable salt thereof, wherein $R^3$ and $R^4$ are each H.

**6.** The compound according to claim 5, or a pharmaceutically acceptable salt thereof, wherein $R^5$, $R^6$ and $R^7$ are each H.

**7.** The compound according to claim 5, or a pharmaceutically acceptable salt thereof, wherein $R^8$ is $-C(R^{10})(R^{11})OH$.

**8.** The compound of claim 1, wherein the $\beta$-D-glucuronide is a $\beta$-D-glucuronyl.

**9.** The compound of claim 8, wherein the $\beta$-D-glucuronyl is methyl glucuronate.

**10.** The compound of claim 1 wherein the compound of formula (**I**) is one of the following compounds:

| Cmpd | Structure | Cmpd | Structure |
| --- | --- | --- | --- |
| 1 | 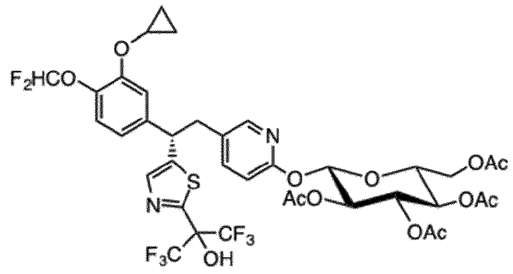 | 17 | 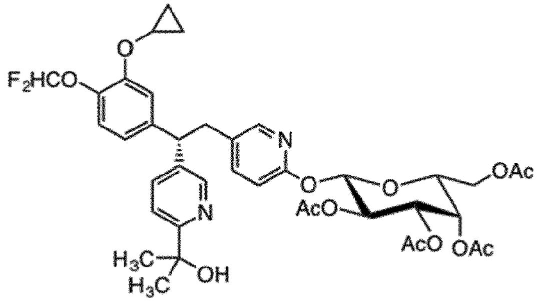 |
| 2 | 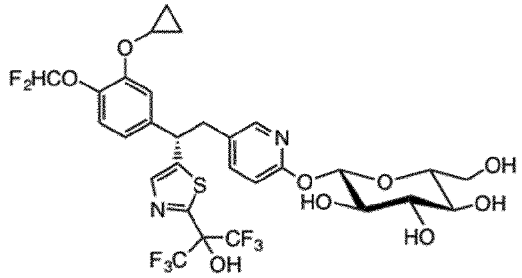 | 18 | 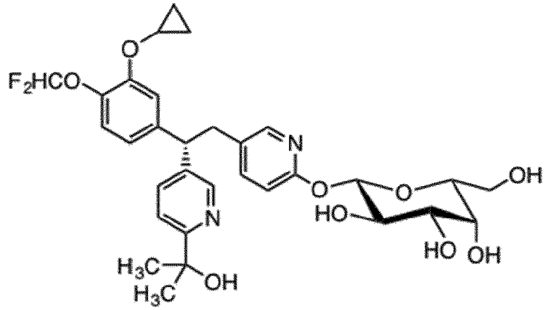 |
| 3 | 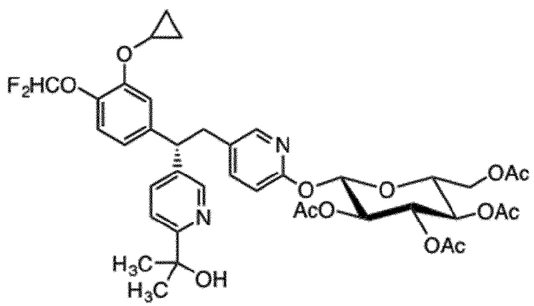 | 19 | 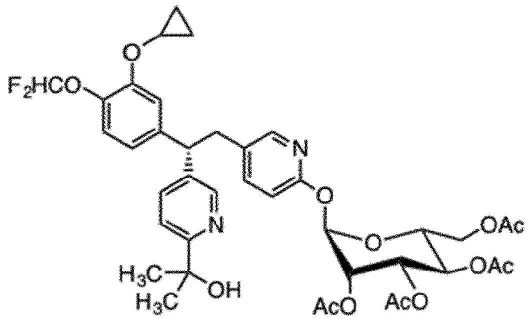 |

(continued)

| Cmpd | Structure | Cmpd | Structure |
|---|---|---|---|
| 4 | 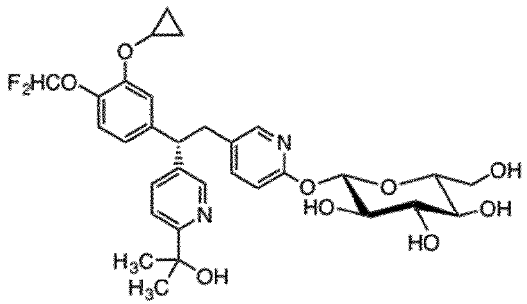 | 20 | 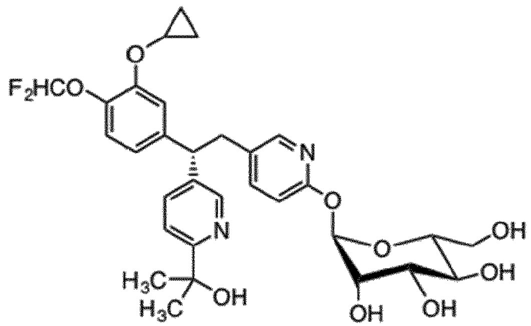 |
| 5 | 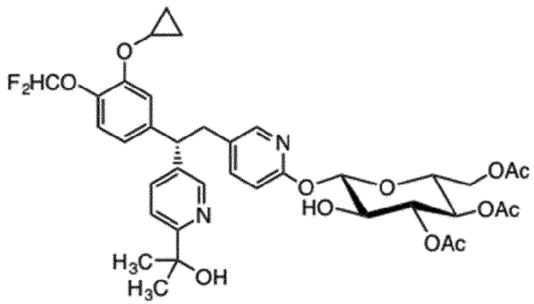 | 21 | 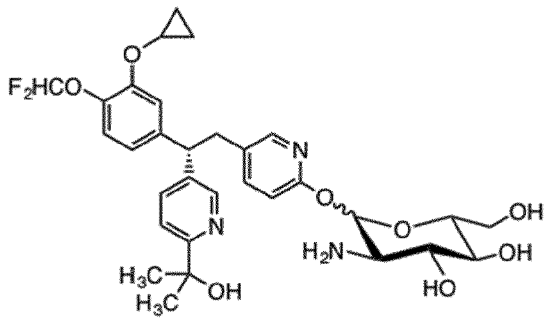 |
| 6 | 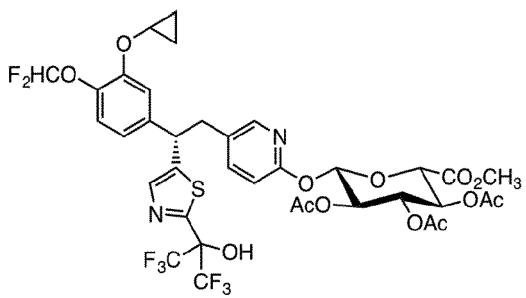 | 22 | 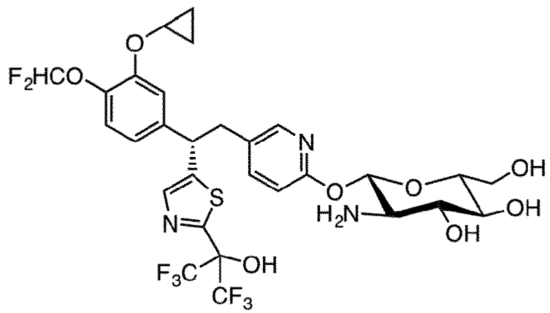 |

(continued)

| Cmpd | Structure | Cmpd | Structure |
|---|---|---|---|
| 7 | 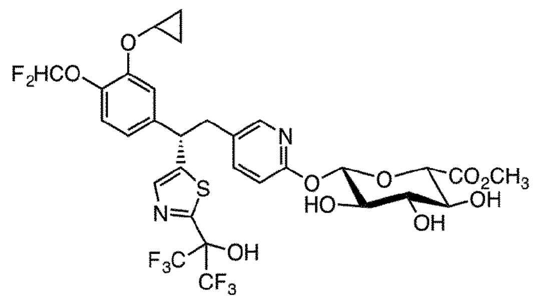 | 23 | 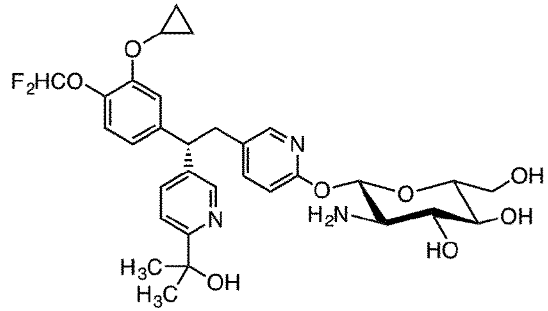 |
| 8 | 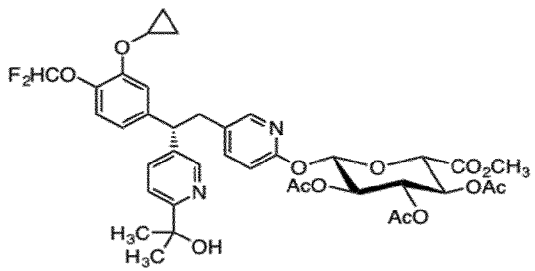 | 24 | 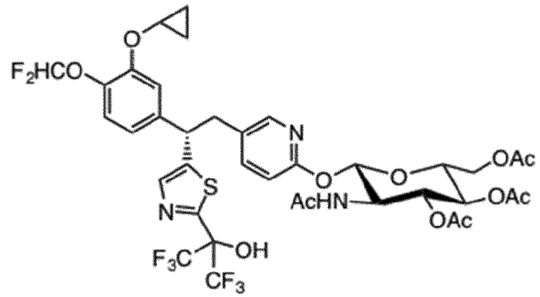 |
| 9 | 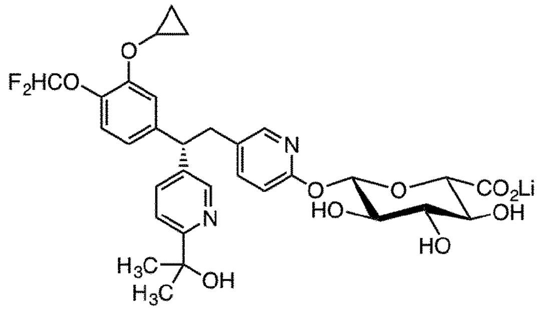 | 25 | 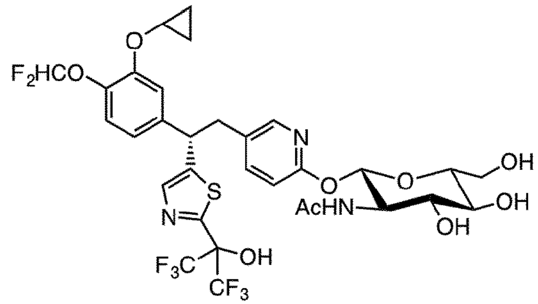 |

(continued)

| Cmpd | Structure | Cmpd | Structure |
|---|---|---|---|
| 10 | 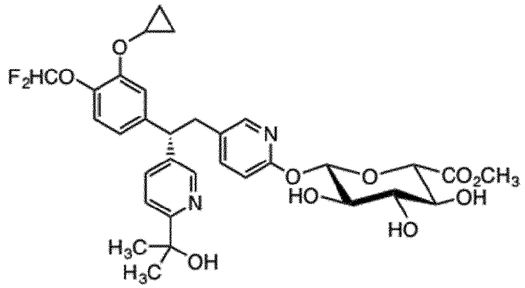 | 26 | 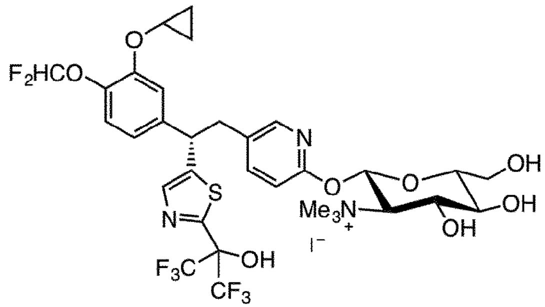 |
| 11 | 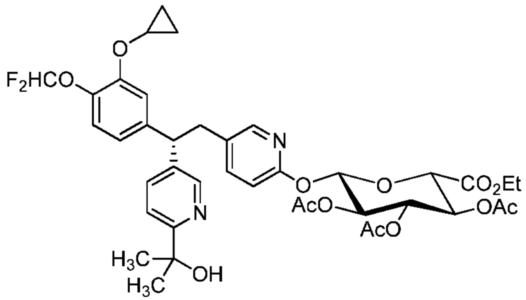 | 27 | 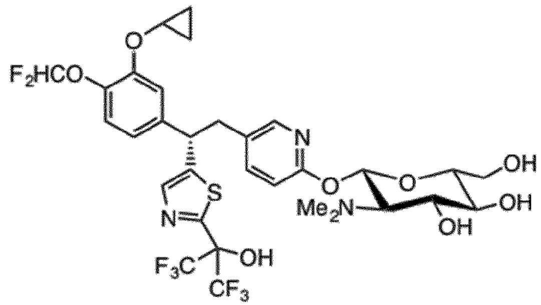 |
| 12 | 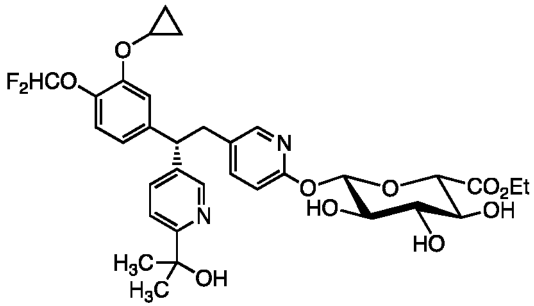 | 28 | 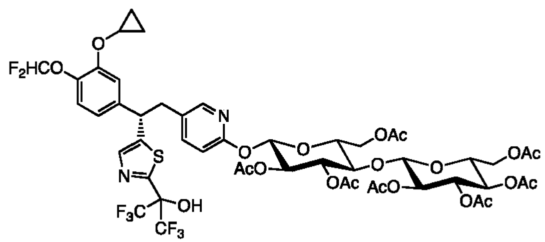 |

(continued)

| Cmpd | Structure | Cmpd | Structure |
|---|---|---|---|
| 13 | 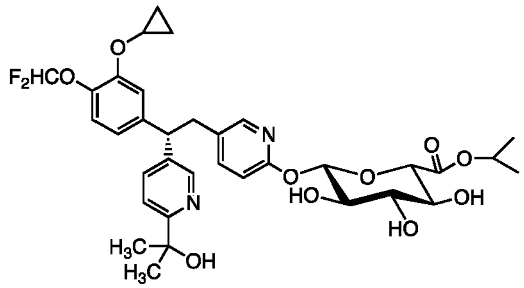 | 29 | 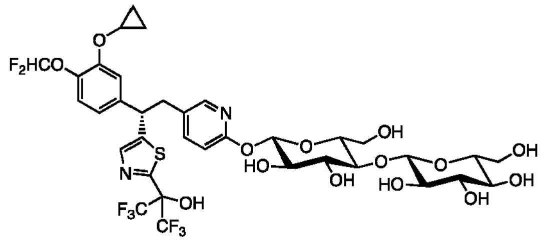 |
| 14 | 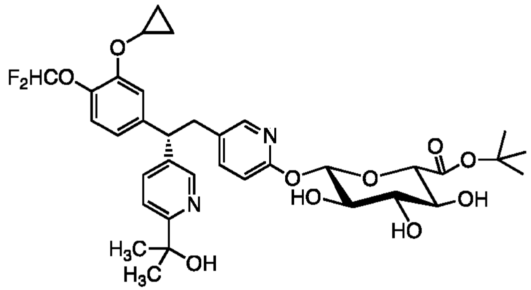 | 30 | 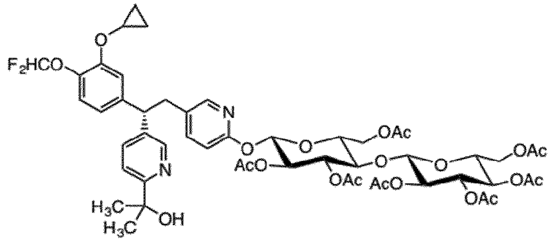 |
| 15 | 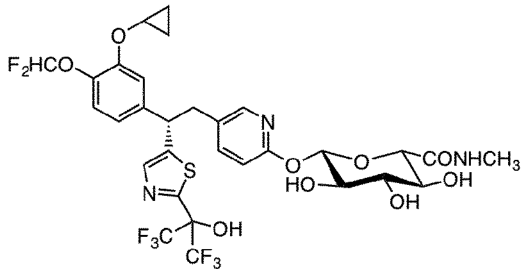 | 31 | 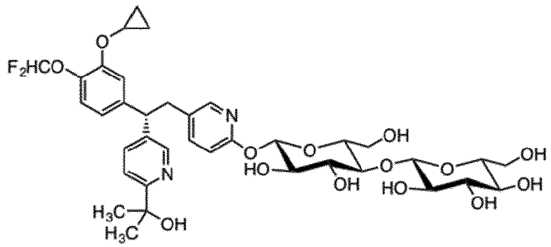 |

(continued)

| Cmpd | Structure | Cmpd | Structure |
|---|---|---|---|
| 16 | 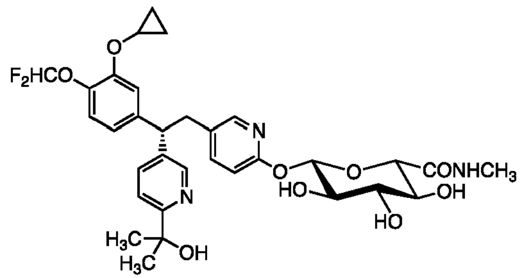 | | |

or a pharmaceutically acceptable salt thereof.

**11.** A pharmaceutical composition comprising a therapeutically effective amount of the compound of formula (**I**), as claimed in any one of claims 1 to 2, or a pharmaceutically acceptable salt thereof, and a pharmaceutically acceptable carrier or diluent or excipients.

**12.** The pharmaceutical composition of claim 11, wherein said therapeutically effective amount is about 0.001 mg, 0.005 mg, 0.025mg, 0.1 mg, 0.5 mg, 2.5 mg, 10 mg, 50 mg, 250 mg, or 1000 mg of the compound of formula (I).

**13.** The pharmaceutical composition of any one of claims 11-12, wherein said composition is at least one of an immediate release formulation, a sustained release formulation or a delayed release formulation, or a combination thereof, in the form of a lotion, a cream, an ointment, or a liquid.

**14.** The pharmaceutical composition of to any one of claims 11-13, further comprising a leukotriene receptor antagonist, a leukotriene biosynthesis inhibitor, an M2/M3 antagonist, a corticosteroid, an HI receptor antagonist, a $\beta_2$ adrenoceptor agonist, a selective COX-2 inhibitor, an NSAID, an immunomodulator, 5-ASA, a 5-ASA prodrug, a janus kinase inhibitor or a combination thereof.

**15.** The compound formula (**I**), as defined in any one of claims 1 to 10, or of the composition of any one of claims 11 to 14 for use in the treatment or prevention of asthma, chronic bronchitis, chronic obstructive pulmonary disease (COPD), adult respiratory distress syndrome, infant respiratory distress syndrome, cough, chronic obstructive pulmonary disease in animals, ulcerative colitis, Crohn's disease, diverticulitis, irritable bowel syndrome, hypersecretion of gastric acid, sepsis or septic shock, endotoxic shock, an endotoxic shock associated condition, spinal cord trauma, head injury, neurogenic inflammation, pain, reperfusion injury of the brain, psoriatic arthritis, rheumatoid arthritis, ankylosing spondylitis, osteoarthritis, inflammation and cytokine-mediated chronic tissue degeneration, allergic rhinitis, allergic conjunctivitis, eosinophilic granuloma, depression, memory impairment, monopolar depression, Parkinson disease, Alzheimer's disease, acute and chronic multiple sclerosis, psoriasis, a benign proliferative skin disease, a malignant proliferative skin disease, atopic dermatitis, urticaria, cancer, tumor growth, cancerous invasion of normal tissues, diabetes insipidus, osteoporosis, arterial restenosis, atherosclerosis, reperfusion injury of the myocardium, chronic glomerulonephritis, vernal conjunctivitis, transplant rejection and graft versus host disease, and cachexia.

**Patentansprüche**

**1.** Verbindung der Formel (I):

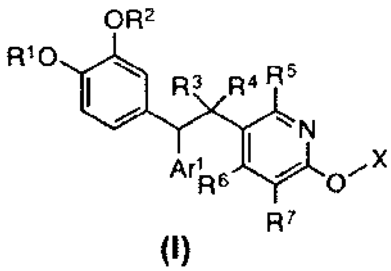

**(I)**

oder ein pharmazeutisch unbedenkliches Salz davon, wobei X für ein $\beta$-D-Glucuronid, ein $\alpha$-D-Glucuronid, ein $\beta$-D-Glucopyranosid, ein $\alpha$-D-Glucopyranosid, ein $\beta$-D-Galactopyranosid, ein $\alpha$-D-Galactopyranosid, ein $\beta$-D-Mannopyranosid, ein $\alpha$-D-Mannopyranosid, ein *N*-Acetyl-$\beta$-D-glucosaminid, ein *N*-Acetyl-$\alpha$-D-glucosaminid, ein *N*-Acetyl-$\beta$-D-galactosaminid, ein N-Acetyl-$\alpha$-D-galactosaminid, ein $\beta$-D-Glucosaminid, ein $\alpha$-D-Glucosaminid, ein $\beta$-D-Galactosaminid, ein $\alpha$-D-Galactosaminid, ein $\beta$-D-Fucopyranosid, ein $\alpha$-L-Fucopyranosid, ein $\alpha$-L-Rhamnopyranosid, ein $\alpha$-L-Arabinofuranosid, ein $\beta$-D-Ribofuranosid, ein $\beta$-D-Cellobiosid, ein $\alpha$-D-Cellobiosid, ein $\beta$-*N,N*-Diacetylchitobiosid, ein D-Xylopyranosid, ein D-Xylofuranosid, ein $\beta$-D-Galacturonid oder ein $\alpha$-D-Galacturonid steht;

$R^1$ und $R^2$ jeweils unabhängig für -$C_{1-6}$-Alkyl oder -$C_{3-6}$-Cycloalkyl stehen, wobei jede dieser Gruppen unsubstituiert oder durch 1-6 unabhängige Halogene substituiert ist;

$R^3$ und $R^4$ jeweils unabhängig für H oder -$C_{1-6}$-Alkyl stehen;

$R^5$, $R^6$ und $R^7$ jeweils unabhängig für H, Halogen, $-C_{1-6}$-Alkyl, $-C(O)C_{1-6}$-Alkyl oder CN stehen;
$Ar^1$ unabhängig ausgewählt ist aus der Gruppe bestehend aus:

(a) 6-$R^8$-3-Pyridyl oder 6-$R^9$-3-Pyridyl,
(b) 2-$R^8$-5-Thiazolyl oder 5-$R^8$-2-Thiazolyl,
(c) 2-$R^8$-5-Pyrimidinyl oder 2-$R^9$-5-Pyrimidinyl,
(d) 6-$R^8$-3-Pyridazinyl oder 6-$R^9$-3-Pyridazinyl,
(e) 5-$R^8$-2-Furyl,
(f) 5-$R^8$-2-Thienyl,
(g) 2-$R^8$-5-Oxazolyl oder 5-$R^8$-2-Oxazolyl,
(h) 5-$R^8$-3-Isoxazolyl oder 3-$R^8$-5-Isoxazolyl,
(i) 5-$R^8$-3-Isothiazolyl oder 3-$R^8$-5-Isothiazolyl und
(j) *p*-$R^8$-Phenyl;

$R^8$ ausgewählt ist aus der Gruppe bestehend aus: H, Halogen, $-C_{1-6}$-Alkyl, $-C_{3-6}$-Cycloalkyl, $-C_{1-6}$-Alkyl-$Ar^2$, $Ar^2$, $C_{1-6}$-Alkoxy, $C_{1-6}$-Alkylthio, CN, $-C(R^{10})(R^{11})OH$, - $C(R^{10})(R^{11})OC_{1-6}$-Alkyl, $-C(R^{10})(R^{11})OAr^2$, $-CO_2H$, $-CO_2C_{1-6}$-Alkyl, $-C(O)NR^{12}R^{13}$, $-SO_2NHC(O)Ar^2$, $-C(O)C_{1-6}$-Alkyl und - $C(O)Ar^2$;
$R^9$ ausgewählt ist aus der Gruppe bestehend aus: $-NR^{12}R^{13}$, $-NR^{12}C(O)R^{13}$, $-NR^{12}C(O)NHR^{13}$, $-NR^{12}SO_2Ar^2$ und $-NR^{12}CO_2Ar^2$;
$R^{10}$ und $R^{11}$ jeweils unabhängig für H, $-C_{1-6}$-Alkyl, $-C_{1-6}$-Halogenalkyl, $-C_{3-6}$-Cycloalkyl oder $Ar^2$ stehen;
oder $R^{10}$ und $R^{11}$ dann, wenn sie für $-C_{1-6}$-Alkyl stehen, über ein $C_{1-3}$-Alkyl unter Bildung von $C_{3-6}$-Cycloalkyl miteinander verbunden sein können;
$R^{12}$ und $R^{13}$ jeweils unabhängig für H, $-C_{1-6}$-Alkyl, $-C_{3-6}$-Cycloalkyl oder $-C_{1-6}$-Alkyl-$Ar^2$ stehen;
oder $R^{12}$ und $R^{13}$ dann, wenn sie für $-C_{1-6}$-Alkyl stehen, über ein $C_{1-3}$-Alkyl unter Bildung von $C_{3-6}$-Heterocycloalkyl miteinander verbunden sein können;
$Ar^2$ ausgewählt ist aus der Gruppe bestehend aus: Phenyl, Pyridinyl, Chinolinyl, Isochinolinyl, Pyridazinyl, Pyrimidinyl, Pyrazinyl, Chinoxalinyl, Furyl, Benzofuryl, Dibenzofuryl, Thienyl, Benzothienyl, Pyrrolyl, Indolyl, Pyrazolyl, Indazolyl, Oxazolyl, Isoxazolyl, Thiazolyl, Isothiazolyl, Imidazolyl, Benzimidazolyl, Oxadiazolyl, Thiadiazolyl, Triazolyl und Tetrazolyl;
wobei $Ar^2$ jeweils unsubstituiert oder durch 1-3 Mitglieder aus der Gruppe bestehend aus Halogen, $-C_{1-6}$-Alkyl, $-C_{1-6}$-Halogenalkyl, CN, $C_{1-6}$-Alkoxy, $C_{1-6}$-Alkylthio, $-C(R^{10})(R^{11})OH$, $-CO_2H$, $-CO_2C_{1-6}$-Alkyl, $-C(O)NR^{12}R^{13}$ und - $SO_2CH_3$ substituiert ist.

**2.** Verbindung nach Anspruch 1 oder ein pharmazeutisch unbedenkliches Salz davon, wobei es sich bei dem β-D-Glucuronid um *β*-D-Glucuronsäure, Methyl-*β*-D-glucuronidat, Methyl-2,3,4-tri-*O*-acetyl-β-D-glucuronidat, Ethyl-2,3,4-tri-*O*-acetyl-*β*-D-glucuronidat, Ethyl-*β*-D-glucuronidat, *i*-Propyl-*β*-D-glucuronidat, ***tert***-Butyl-*β*-D-glucuronidat oder Methyl-*β*-D-glucuronamid handelt;

wobei es sich bei dem *β*-D-Glucopyranosid um *β*-D-Glucopyranosyl, 2,3,4,6-Tetra-*O*-acetyl-*β*-D-glucopyranosyl oder 3,4,6-Tri-*O*-acetyl-*β*-D-glucopyranosyl handelt;
wobei es sich bei dem *β*-D-Galactopyranosid um *β*-D-Galactopyranosyl oder 2,3,4,6-Tetra-*O*-acetyl-*β*-D-galactopyranosyl handelt;
wobei es sich bei dem *α*-D-Mannopyranosid um *α*-D-Mannopyranosyl oder 2,3,4,6-Tetra-*O*-acetyl-*α*-D-mannopyranosyl handelt;
wobei es sich bei dem *β*-D-Glucosaminid um *β*-D-Glucosaminyl, *α*-D-Glucosaminyl, *N,N,N*-Trimethyl-β-D-glucosaminyl oder *N,N*-Dimethyl-*β*-D-glucosaminyl handelt; wobei es sich bei dem *N*-Acetyl-β-D-glucosaminid um *N*-Acetyl-*β*-D-glucosaminyl oder 3,4,6-Tri-*O*-acetyl-*N*-acetyl-*β*-D-glucosaminyl handelt; und
wobei es sich bei dem *β*-D-Cellobiosid um *β*-D-Cellobiosyl oder 2,3,6,2',3',4',6'-Hepta-*O*-acetyl-β-D-cellobiosyl handelt.

**3.** Verbindung nach einem der Ansprüche 1-2 oder ein pharmazeutisch unbedenkliches Salz davon, wobei $Ar^1$ für 6-$R^8$-3-Pyridyl oder 2-$R^8$-5-Thiazolyl steht.

**4.** Verbindung nach einem der Ansprüche 1-2 oder ein pharmazeutisch unbedenkliches Salz davon, wobei $Ar^1$ für 6-$R^8$-3-Pyridyl steht.

**5.** Verbindung nach Anspruch 4 oder ein pharmazeutisch unbedenkliches Salz davon, wobei $R^3$ und $R^4$ jeweils für H stehen.

**6.** Verbindung nach Anspruch 5 oder ein pharmazeutisch unbedenkliches Salz davon, wobei $R^5$, $R^6$ und $R^7$ jeweils für H stehen.

**7.** Verbindung nach Anspruch 5 oder ein pharmazeutisch unbedenkliches Salz davon, wobei $R^8$ für $-C(R^{10})(R^{11})OH$ steht.

**8.** Verbindung nach Anspruch 1, wobei es sich bei dem *β*-D-Glucuronid um ein β-D-Glucuronyl handelt.

**9.** Verbindung nach Anspruch 8, wobei es sich bei dem *β*-D-Glucuronyl um Methylglucuronat handelt.

**10.** Verbindung nach Anspruch 1, wobei es sich bei der Verbindung der Formel (I) um eine der folgenden Verbindungen handelt:

| Verb. | Struktur | Verb. | Struktur |
|---|---|---|---|
| 1 | 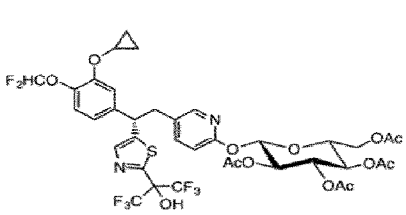 | 17 | 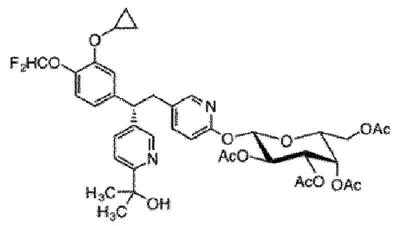 |
| 2 | 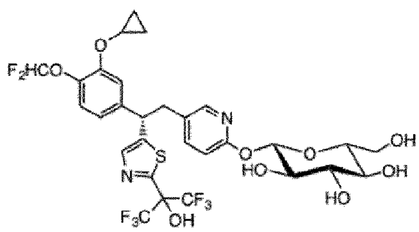 | 18 | 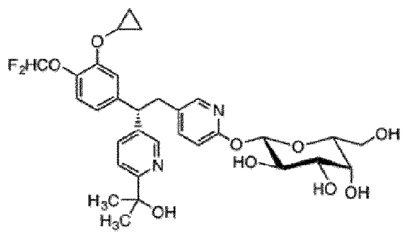 |
| 3 | 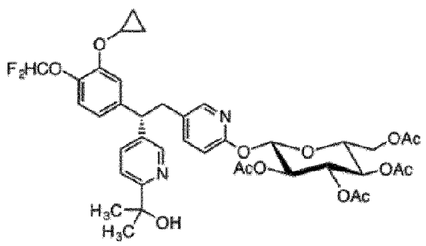 | 19 | 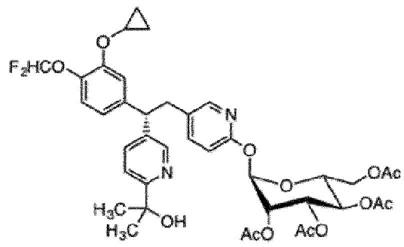 |
| 4 | 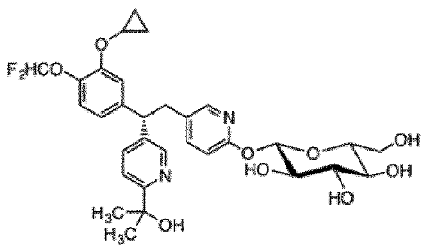 | 20 | 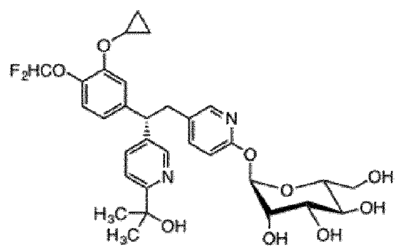 |
| 5 | 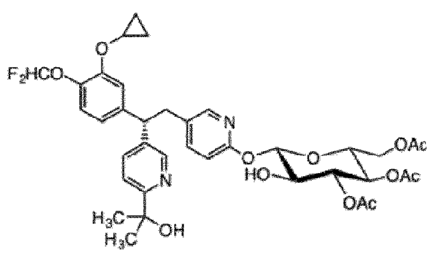 | 21 | 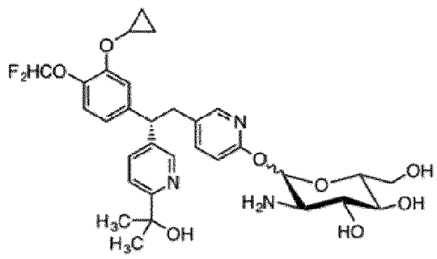 |

(fortgesetzt)

| Verb. | Struktur | Verb. | Struktur |
|---|---|---|---|
| 6 | 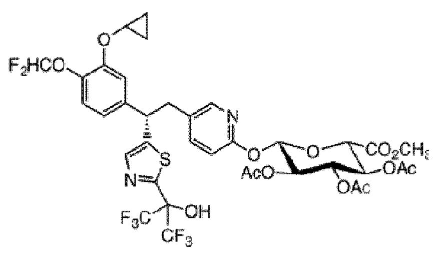 | 22 | 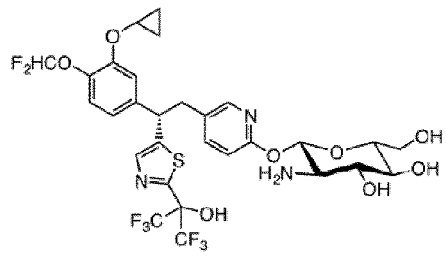 |
| 7 | 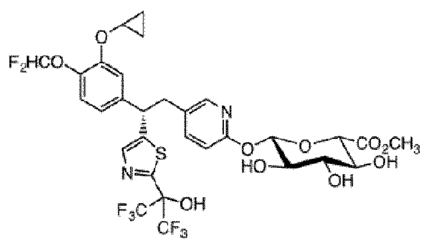 | 23 | 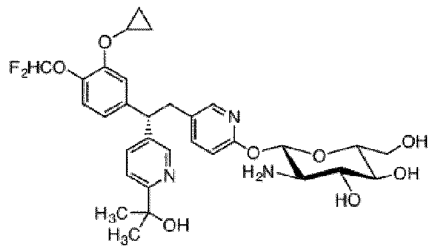 |
| 8 | 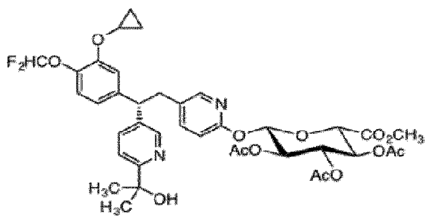 | 24 | 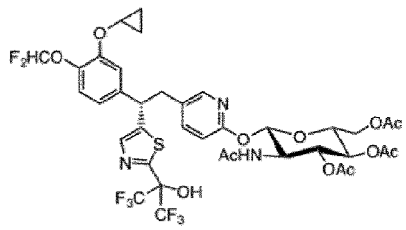 |
| 9 | 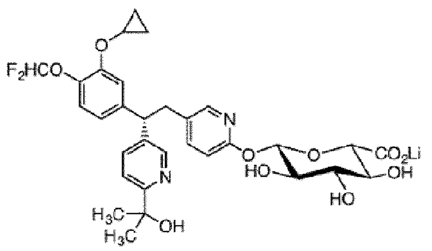 | 25 | 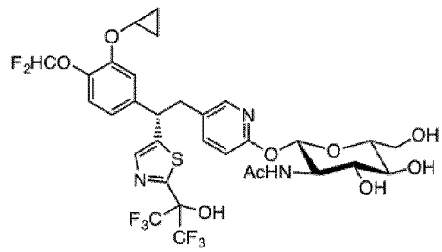 |
| 10 | 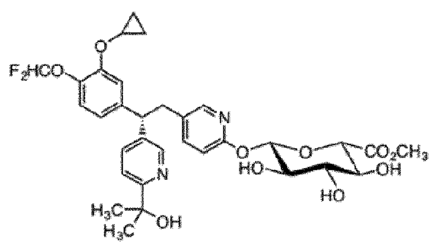 | 26 | 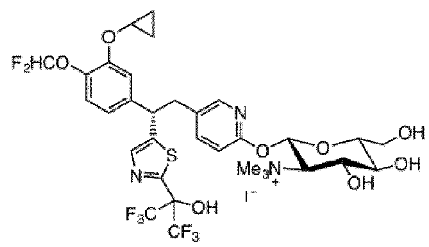 |
| 11 | 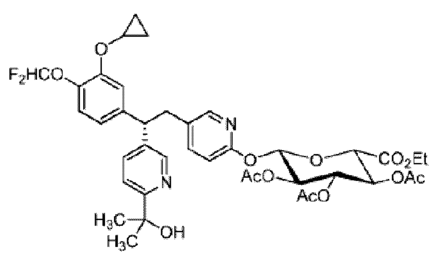 | 27 | 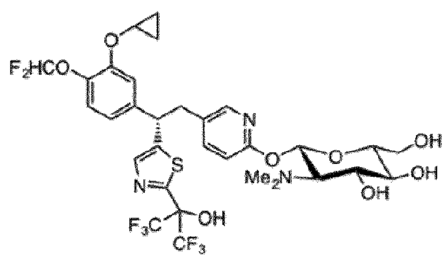 |

(fortgesetzt)

| Verb. | Struktur | Verb. | Struktur |
|---|---|---|---|
| 12 | 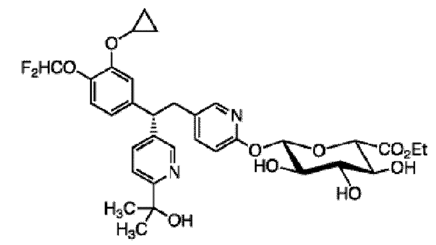 | 28 | 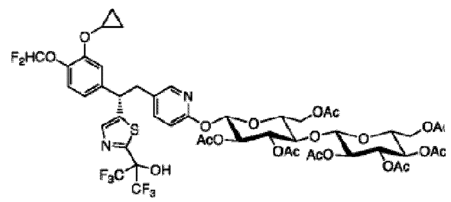 |
| 13 | 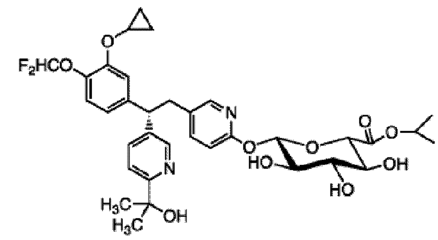 | 29 | 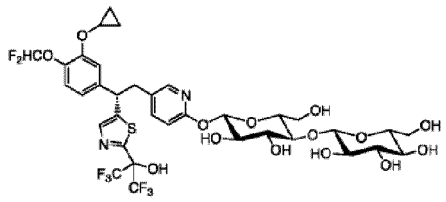 |
| 14 | 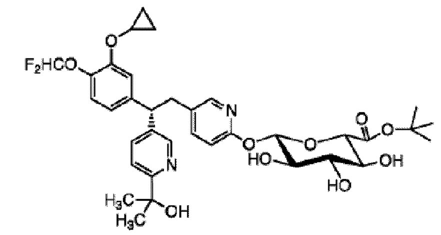 | 30 | 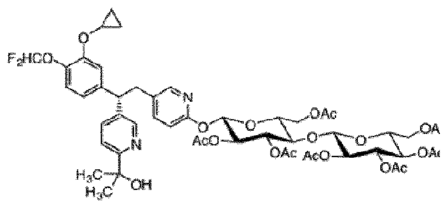 |
| 15 | 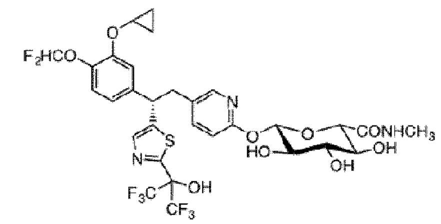 | 31 | 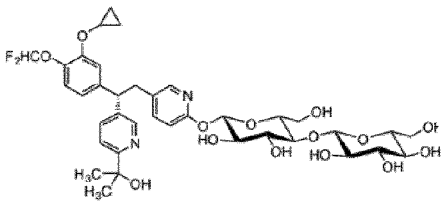 |
| 16 | 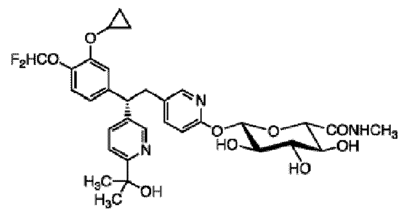 | | |

oder ein pharmazeutisch unbedenkliches Salz davon.

**11.** Pharmazeutische Zusammensetzung, umfassend eine therapeutisch wirksame Menge der Verbindung der Formel (I) nach einem der Ansprüche 1 bis 2 oder ein pharmazeutisch unbedenkliches Salz davon und einen pharmazeutisch unbedenklichen Träger oder ein pharmazeutisch unbedenkliches Verdünnungsmittel oder pharmazeutisch unbedenkliche Hilfsstoffe.

**12.** Pharmazeutische Zusammensetzung nach Anspruch 11, wobei die therapeutisch wirksame Menge etwa 0,001 mg, 0, 005 mg, 0, 025 mg, 0, 1 mg, 0,5 mg, 2,5 mg, 10 mg, 50 mg, 250 mg oder 1000 mg der Verbindung der Formel (I) beträgt.

**13.** Pharmazeutische Zusammensetzung nach einem der Ansprüche 11-12, wobei es sich bei der Zusammensetzung um mindestens eine von einer Formulierung mit sofortiger Wirkstofffreisetzung, einer Formulierung mit anhaltender

Wirkstofffreisetzung oder einer Formulierung mit verzögerter Wirkstofffreisetzung oder eine Kombination davon in Form einer Lotion, einer Creme, einer Salbe oder einer Flüssigkeit handelt.

**14.** Pharmazeutische Zusammensetzung nach einem der Ansprüche 11-13, ferner umfassend einen Leukotrienrezeptorantagonisten, einen Leukotrienbiosyntheseinhibitor, einen M2/M3-Antagonisten, ein Kortikosteroid, einen HI-Rezeptor-Antagonisten, einen $\beta_2$-Adrenorezeptor-Agonisten, einen selektiven COX-2-Inhibitor, ein NSAID, einen Immunmodulator, 5-ASA, ein 5-ASA-Prodrug, einen Januskinaseinhibitor oder eine Kombination davon.

**15.** Verbindung der Formel (I) gemäß einem der Ansprüche 1 bis 10 oder Zusammensetzung nach einem der Ansprüche 11 bis 14 zur Verwendung bei der Behandlung oder Prävention von Asthma, chronischer Bronchitis, chronisch-obstruktiver Lungenerkrankung (Chronic Obstructive Pulmonary Disease, COPD), akutem Atemnotsyndrom, Atemnotsyndrom des Neugeborenen, Husten, chronisch-obstruktiver Lungenerkrankung bei Tieren, Colitis ulcerosa, Morbus Crohn, Divertikulitis, Reizdarmsyndrom, Hypersekretion von Magensäure, Sepsis oder septischem Schock, Endotoxinschock, einem mit Endotoxinschock assoziierten Leiden, Rückenmarkstrauma, Kopfverletzung, neurogener Entzündung, Schmerzen, Reperfusionsverletzung des Gehirns, Arthritis psoriatica, rheumatoider Arthritis, Spondylitis ankylosans, Osteoarthritis, entzündungs- und zytokinvermittelter chronischer Gewebedegeneration, allergischer Rhinitis, allergischer Konjunktivitis, eosinophilem Granulom, Depression, Gedächtnisschwäche, monopolarer Depression, Parkinson-Krankheit, Alzheimer-Krankheit, akuter und chronischer multipler Sklerose, Psoriasis, einer gutartigen proliferativen Hauterkrankung, einer bösartigen proliferativen Hauterkrankung, atopischer Dermatitis, Nesselsucht, Krebs, Tumorwachstum, kanzeröser Invasion von normalen Geweben, Diabetes insipidus, Osteoporose, arterieller Restenose, Atherosklerose, Reperfusionsverletzung des Myokards, chronischer Glomerulonephritis, Frühjahrskonjunktivitis, Transplantatabstoßung und Graft-versus-Host-Krankheit und Kachexie.

## Revendications

**1.** Un composé de Formule (I) :

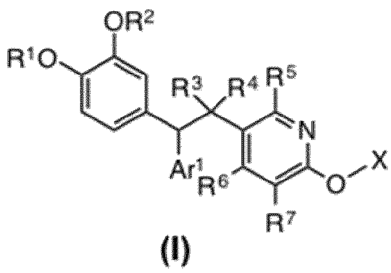

**(I)**

ou un sel pharmaceutiquement acceptable de celui-ci, **caractérisé en ce que**

X est un $\beta$-D-glucuronide, un $\alpha$-D-glucuronide, un $\beta$-D-glucopyranoside, un $\alpha$-D-glucopyranoside, un $\beta$-D-galactopyranoside, un $\alpha$-D-galactopyranoside, un $\beta$-D-mannopyranoside , un $\alpha$-D-mannopyranoside, un N-acétyl-$\beta$-D-glucosaminide, un N-acétyl-$\alpha$-D-glucosaminide, un N-acétyl-$\beta$-D-galactosaminide, un N-acétyl-$\alpha$-D- galactosaminide, un $\beta$-D-glucosaminide, un $\alpha$-D-glucosaminide, un $\beta$-D-galactosaminide, un $\alpha$-D-galactosaminide, un $\beta$-D-fucopyranoside, un $\alpha$-L-fucopyranoside, un $\alpha$-L-rhamnopyranoside, un $\alpha$-L-arabinofuranoside, un $\beta$-D-ribofuranoside, un $\beta$-D-cellobioside, un $\alpha$-D-cellobioside, un $\beta$-N,N-diacétyle chitobioside, un D-xylopyranoside, un D-xylofuranoside, un $\beta$-D-galacturonide ou an $\alpha$-D-galacturonide;
$R^1$ et $R^2$ sont chacun indépendamment-$C_{1-6}$alkyle, -$C_{3-6}$cycloalkyle; dont l'un est non substitué ou substitué par 1-6 halogène indépendant;
$R^3$ et $R^4$ représentent indépendamment H or -$C_{1-6}$alkyle;
$R^5$, $R^6$ et $R^7$ sont indépendamment H, halogène, -$C_{1-6}$alkyle, -C(O)$C_{1-6}$alkyle ou CN ;
$Ar^1$ est sélectionné indépendamment dans le groupe constitué de :

(a) 6-$R^8$-3-pyridyle ou 6-$R^9$-3-pyridyle,
(b) 2-$R^8$-5-thiazolyle ou 5-$R^8$-2-thiazolyle,
(c) 2-$R^8$-5-pyrimidinyle ou 2-$R^9$-5-pyrimidinyle,
(d) 6-$R^8$-3-pyridazinyle ou 6-$R^9$-3-pyridazinyle,
(e) 5-$R^8$-2-furyle,

(f) 5-$R^8$-2-thiényle,
(g) 2-$R^8$-5-oxazolyl ou 5-$R^8$-2-oxazolyl,
(h) 5-$R^8$-3-isoxazolyl ou 3-$R^8$-5-isoxazolyl,
(i) 5-$R^8$-3-isothiazolyl ou 3-$R^8$-5-isothiazolyl, et
(j) *p*-$R^8$-phényle;

$R^8$ est choisi dans le groupe constitué de H, halogène, -$C_{1-6}$ alkyle, -$C_{3-6}$ cycloalkyle, -$C_{1-6}$ alkyl$Ar^2$, $Ar^2$, $C_{1-6}$ alcoxy, $C_{1-6}$ alkylthio, CN, -C($R^{10}$)($R^{11}$)OH, -C($R^{10}$)($R^{11}$)O$C_{1-6}$ alkyle, -C($R^{10}$)($R^{11}$)O$Ar^2$, -$CO_2$H, -$CO_2C_{1-6}$alkyle, -C(O)N$R^{12}R^{13}$, -$SO_2$NHC(O)$Ar^2$, -C(O)$C_{1-6}$alkyle et -C(O)$Ar^2$;
$R^9$ est sélectionné dans le groupe constitué de : -N$R^{12}R^{13}$, -N$R^{12}$C(O)$R^{13}$, -N$R^{12}$C(O)NH$R^{13}$, -N$R^{12}SO_2Ar^2$ et -N$R^{12}CO_2Ar^2$ ;
$R^{10}$ et $R^{11}$ sont indépendamment H, -$C_{1-6}$alkyle, -$C_{1-6}$halogénoalkyle, -$C_{3-6}$cycloalkyle ou $Ar^2$ ;
ou lorsque $R^{10}$ et $R^{11}$ sont -$C_{1-6}$alkyle, ils peuvent se connecter ensemble via un $C_{1-3}$ alkyle pour former $C_{3-6}$cycloalkyle ;
$R^{12}$ et $R^{13}$ sont indépendamment H, -$C_{1-6}$alkyle, -$C_{3-6}$cycloalkyle ou -$C_{1-6}$alkyl$Ar^2$ ;
ou lorsque $R^{12}$ et $R^{13}$ sont -$C_{1-6}$ alkyle, ils peuvent se connecter ensemble par l'intermédiaire d'un $C_{1-3}$alkyle pour former un $C_{3-6}$hétérocycloalkyle ;
$Ar^2$ est sélectionné dans le groupe constitué de : phényle, pyridinyle, quinolinyle, isoquinolinyle, pyridazinyle, pyrimidinyle, pyrazinyle, quinoxalinyle, furyle, benzofuryle, dibenzofuryle, thiényle, benzothiényle, pyrrolyle, indolyle, pyrazolyle, indazolyle, oxazolyle, isoxazolyle, thiazolyle, isothiazolyle, imidazolyle, benzimidazolyle, oxadiazolyl, thiadiazolyle, triazolyl et tétrazolyle;
chaque $Ar^2$ étant non substitué ou substitué par 1 à 3 membres sélectionné dans le groupe constitué de : halogéno, -$C_{1-6}$alkyle, -$C_{1-6}$halogénoalkyle, CN, $C_{1-6}$alcoxy, $C_{1-6}$alkylthio, -C($R^{10}$)($R^{11}$)OH, -$CO_2$H, -$CO_2C_{1-6}$alkyle, -C(O)N$R^{12}R^{13}$ et -$SO_2CH_3$.

**2.** Le composé selon la revendication 1, ou un sel pharmaceutiquement acceptable de celui-ci, **caractérisé en ce que** le $\beta$-D-glucuronide est acide $\beta$-D-glucuronique, $\beta$-D-glucuronidate de méthyle, méthyl 2,3,4-tri-*O*-acétyl-$\beta$-D-glucuronidate, 2,3,4-tri-*O*-acétyl-$\beta$-D-glucuronidate d'éthyle, $\beta$-D-glucuronidate d'éthyle, $\beta$-D-glucuronidate d'*i*-propyle, $\beta$-D-glucuronidate de *tert*-butyle, ou méthyl $\beta$-D-glucuronamide ;

**caractérisé en ce que** le $\beta$-D-glucopyranoside est $\beta$-D-glucopyranosyl, 2,3,4,6-tétra-*O*-acétyl-$\beta$-D-glucopyranosyl ou 3,4,6-tri-*O*-acétyl-$\beta$-D-glucopyranosyl;
**caractérisé en ce que** le $\beta$-D-galactopyranoside est $\beta$-D-galactopyranosyle ou 2,3,4,6-tétra-*O*-acétyl-$\beta$-D-galactopyranosyle ;
**caractérisé en ce que** le $\alpha$-D-mannopyranoside est $\alpha$-D-mannopyranosyle ou 2,3,4,6-tétra-*O*-acétyl-$\alpha$-D-mannopyranosyle ;
**caractérisé en ce que** le $\beta$-D-glucosaminide est $\beta$-D-glucosaminyl, $\alpha$-D-glucosaminyl, *N*,*N*,*N*-triméthyl-$\beta$-D-glucosaminyl ou *N*,*N*-diméthyl-$\beta$-D-glucosaminyl ;
**caractérisé en ce que** le N-acétyl-$\beta$-D-glucosaminide est N-acétyl-$\beta$-D-glucosaminyl, ou 3,4,6-tri-*O*-acétyl-*N*-acétyl-$\beta$-D-glucosaminyl ; et
**caractérisé en ce que** le $\beta$-D-cellobiosyle est $\beta$-D-cellobiosyle, ou 2,3,6,2',3',4',6'-hepta-*O*-acétyl-$\beta$-D-cellobiosyle.

**3.** Le composé selon l'une quelconque des revendications 1 - 2, ou un sel pharmaceutiquement acceptable de celui-ci, **caractérisé en ce que** $Ar^1$ est 6-$R^8$-3-pyridyle ou 2-$R^8$-5-thiazolyle.

**4.** Le composé selon l'une quelconque des revendications 1 - 2, ou un sel pharmaceutiquement acceptable de celui-ci, **caractérisé en ce que** $Ar^1$ est 6-$R^8$-3-pyridyle.

**5.** Le composé selon la revendication 4, ou un sel pharmaceutiquement acceptable de celui-ci, **caractérisé en ce que** $R^3$ et $R^4$ sont chacun H.

**6.** Le composé selon la revendication 5, ou un sel pharmaceutiquement acceptable de celui-ci, **caractérisé en ce que** $R^5$, $R^6$ et $R^7$ sont chacun H.

**7.** Le composé selon la revendication 5, ou un sel pharmaceutiquement acceptable de celui-ci, **caractérisé en ce que** $R^8$ est -C($R^{10}$)($R^{11}$)OH.

**8.** Le composé selon la revendication 1, ou un sel pharmaceutiquement acceptable de celui-ci, **caractérisé en ce que** le $\beta$-D-glucuronide est $\beta$-D-glucuronyle.

**9.** Le composé selon la revendication 1, ou un sel pharmaceutiquement acceptable de celui-ci, **caractérisé en ce que** le $\beta$-D-glucuronyle est glucuronate de méthyle.

**10.** Le composé selon la revendication 1, **caractérisé en ce que** le composé de formule (I) est l'un des composés suivants :

| Composé | Structure | Composé | Structure |
|---|---|---|---|
| 1 | 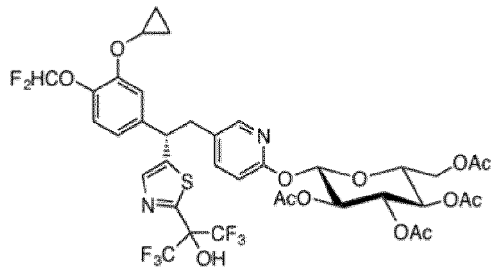 | 17 | 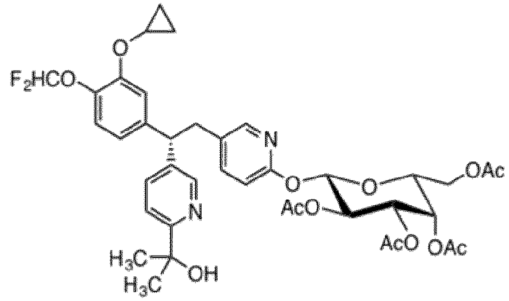 |
| 2 | 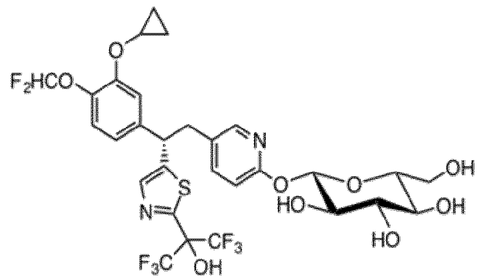 | 18 | 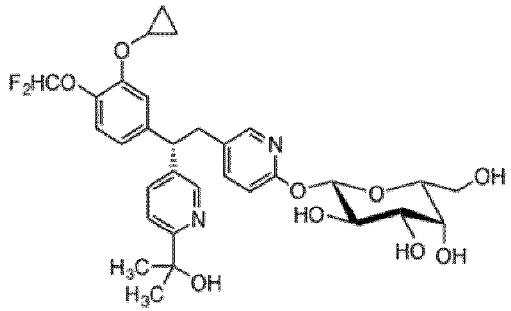 |
| 3 | 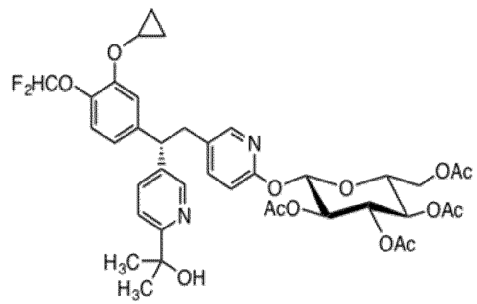 | 19 | 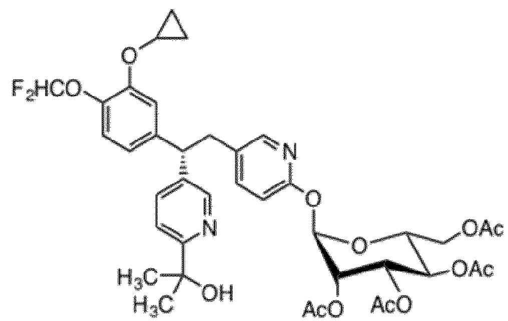 |

(suite)

| Composé | Structure | Composé | Structure |
|---|---|---|---|
| 4 | 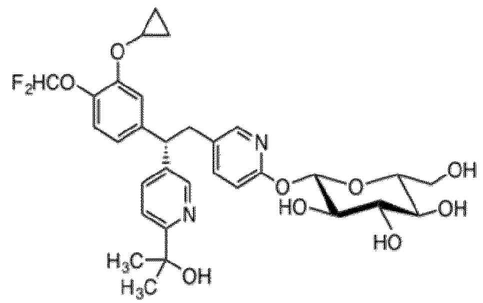 | 20 | 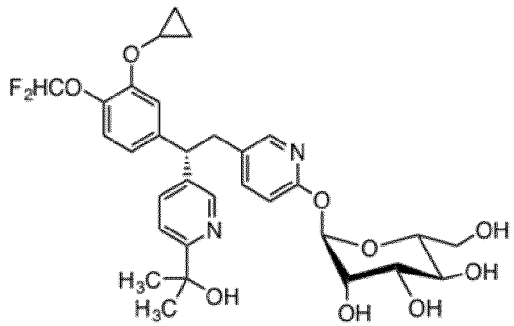 |
| 5 | 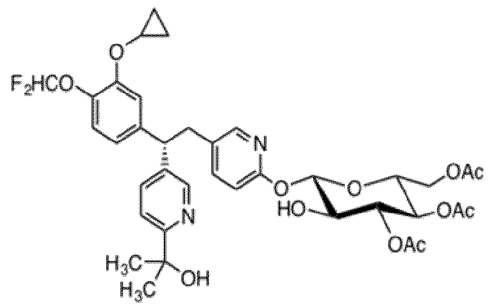 | 21 | 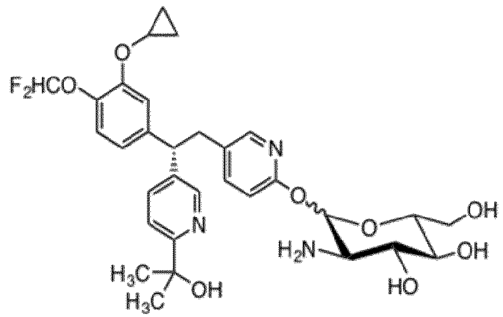 |
| 6 | 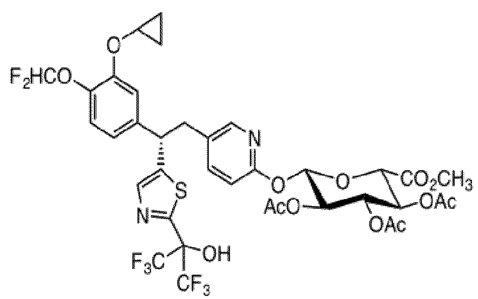 | 22 | 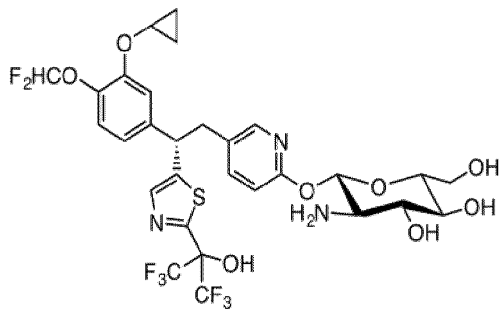 |

(suite)

| Composé | Structure | Composé | Structure |
|---|---|---|---|
| 7 | 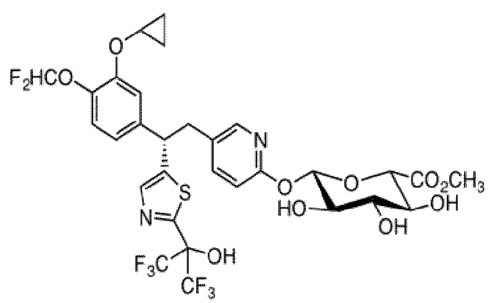 | 23 | 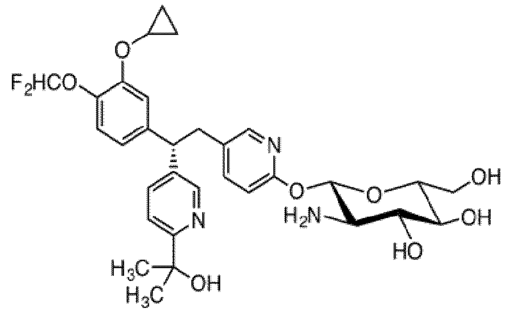 |
| 8 | 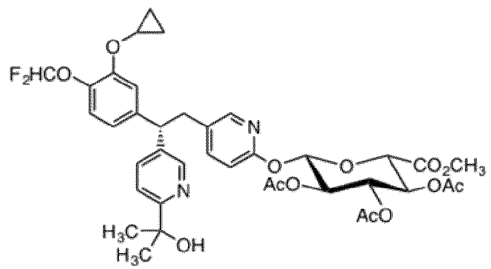 | 24 | 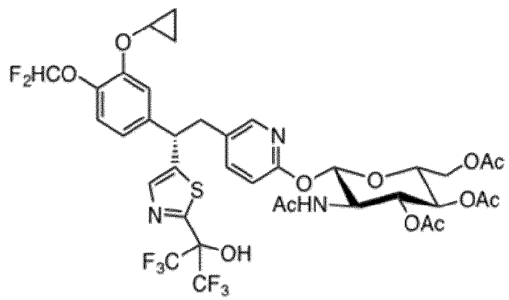 |
| 9 | 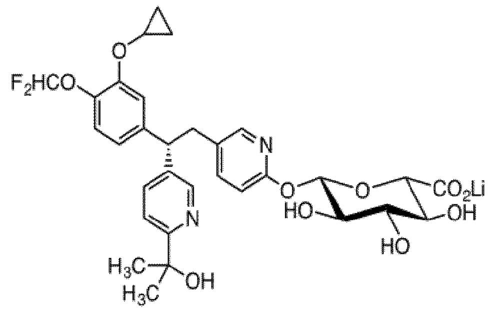 | 25 | 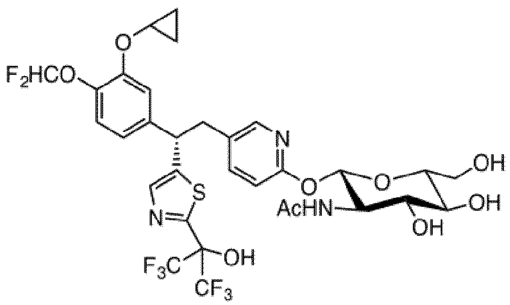 |

(suite)

| Composé | Structure | Composé | Structure |
|---|---|---|---|
| 10 | 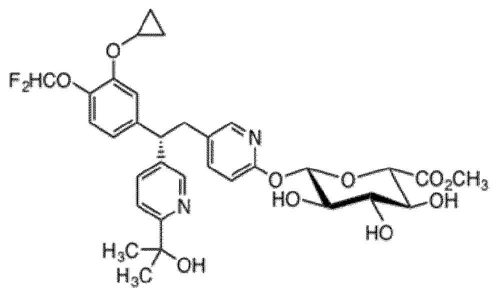 | 26 | 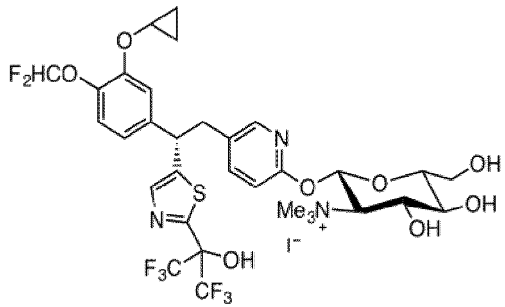 |
| 11 | 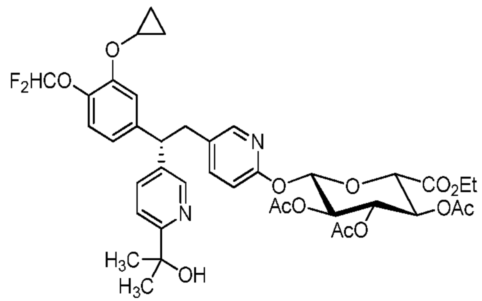 | 27 | 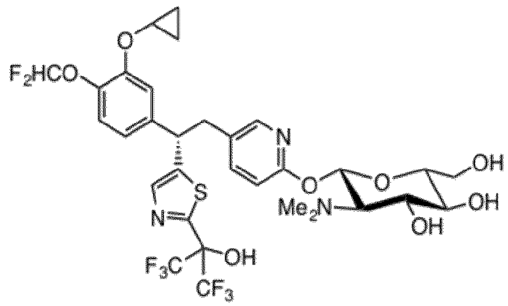 |
| 12 | 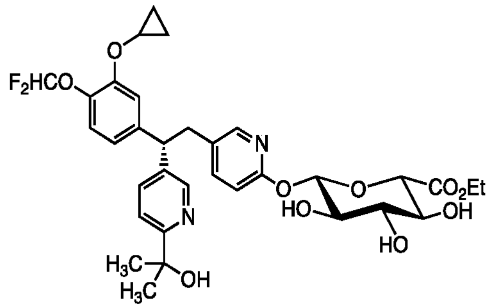 | 28 | 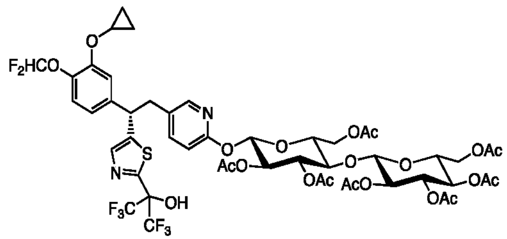 |

(suite)

| Composé | Structure | Composé | Structure |
|---|---|---|---|
| 13 | 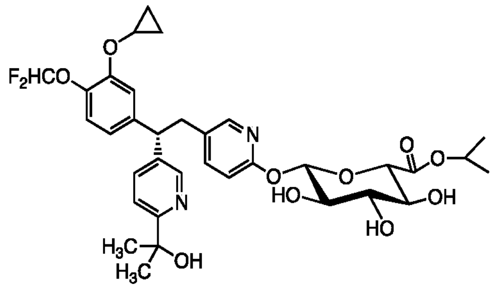 | 29 | 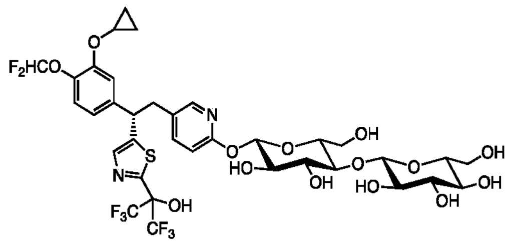 |
| 14 | 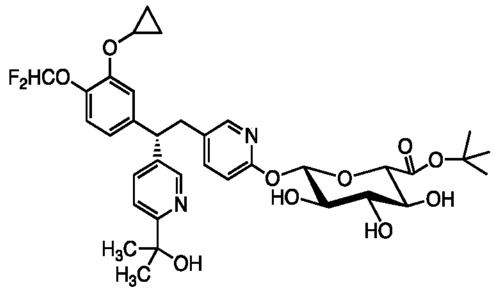 | 30 | 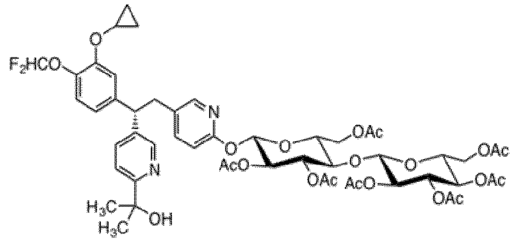 |
| 15 | 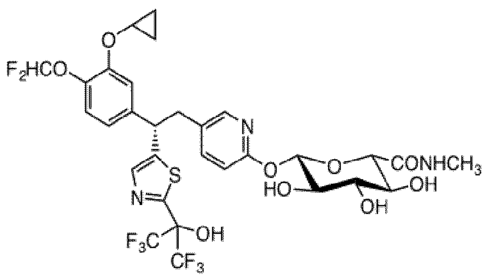 | 31 | 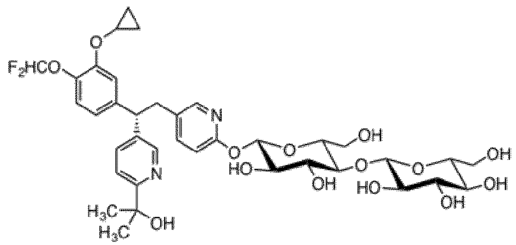 |

(suite)

| Composé | Structure | Composé | Structure |
|---|---|---|---|
| 16 | 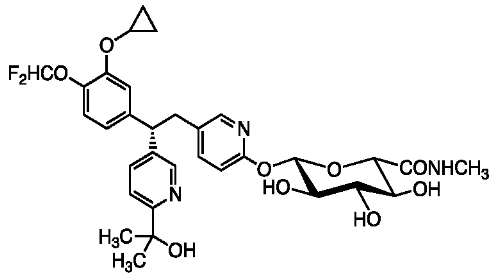 | | |

ou un sel pharmaceutiquement acceptable de celui-ci.

**11.** Une composition pharmaceutique comprenant une quantité thérapeutiquement efficace du composé de formule (I), tel que revendiqué dans l'une quelconque des revendications 1 à 2, ou un sel pharmaceutiquement acceptable de celui-ci, et un véhicule ou diluant ou excipients pharmaceutiquement acceptables.

**12.** La composition pharmaceutique selon la revendication 11, dans laquelle ladite quantité thérapeutiquement efficace est d'environ 0,001 mg, 0,005 mg, 0,025 mg, 0,1 mg, 0,5 mg, 2,5 mg, 10 mg, 50 mg, 250 mg ou 1 000 mg du composé de formule (I).

**13.** La composition pharmaceutique selon l'une quelconque des revendications 11-12, dans laquelle ladite composition est au moins une formulation parmi une formulation à libération immédiate, une formulation à libération prolongée ou une formulation à libération retardée, ou une combinaison de celles-ci, sous la forme d'une lotion, d'une crème, une pommade ou un liquide.

**14.** La composition pharmaceutique selon l'une quelconque des revendications 11-13, comprenant en outre un antagoniste des récepteurs des leucotriènes, un inhibiteur de la biosynthèse des leucotriènes, un antagoniste M2/M3, un corticostéroïde, un antagoniste des récepteurs HI, un agoniste des récepteurs adrénergiques $\beta_2$, un inhibiteur COX-2 sélectif, un AINS, un immunomodulateur, 5-ASA, un promédicament de 5-ASA, un inhibiteur de janus kinase ou une combinaison de ceux-ci.

**15.** Le composé de formule (I), tel que défini dans l'une quelconque des revendications 1 à 10, ou la composition selon l'une quelconque des revendications 11 à 14 pour utilisation dans le traitement ou la prévention de l'asthme, de la bronchite chronique, de la bronchopneumopathie chronique obstructive (BPCO), du syndrome de détresse respiratoire de l'adulte, du syndrome de détresse respiratoire du nourrisson, de la toux, de la maladie pulmonaire obstructive chronique chez les animaux, de la colite ulcéreuse, de la maladie de Crohn, de la diverticulite, du syndrome du côlon irritable, de l'hypersécrétion d'acide gastrique, la septicémie ou choc septique, du choc endotoxique, une condition associée à un choc endotoxique , du traumatisme de la moelle épinière, du traumatisme crânien, de l'inflammation neurogène, de la douleur, de la lésion de reperfusion cérébrale, du rhumatisme psoriasique, de la polyarthrite rhumatoïde, du spondylarthrite ankylosante, de l'arthrose, de l'inflammation et dégénérescence tissulaire chronique médiée par les cytokines, de la rhinite allergique, de la conjonctivite allergique, du granulome éosinophile, de la dépression, des troubles de la mémoire, de la dépression monopolaire, de la maladie de Parkinson, de la maladie d'Alzheimer, de la sclérose en plaques aiguë et chronique, du psoriasis, de la maladie cutanée proliférative bénigne, de la maladie cutanée proliférative maligne, de la dermatite atopique, de l'urticaire, du cancer, de la croissance tumorale, de l'invasion cancéreuse des tissus normaux, du diabète insipide, de l'ostéoporose, de la resténose artérielle, de l'athérosclérose, de la lésion de reperfusion du myocarde, de la glomérulonéphrite chronique, de la conjonctivite vernale, du rejet de greffe et de la maladie du greffon contre l'hôte et de la cachexie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63031023
- WO 2004005258 A1
- WO 0170738 A2
- CA 2369323 A1
- US 3845770 A
- US 3916899 A
- US 3536809 A
- US 5059595 A
- WO 2011147296 A


### Non-patent literature cited in the description


- **SANDBORN et al.** *Gastroenterology,* 2012, vol. 143, 1218
- **FLINT et al.** *Gut Microbes,* 2012, vol. 3 (4), 289
- **DANESE, S. et al.** *Clin. Gastroenterol. Hepato.,* 2020, vol. 18 (11), 2526-2534
- **LI CHUN et al.** *Drug Metabolism and Disposition, Pharmacology and Experimental Therapeutics,* 2001, vol. 39 (3), 232-241
- **TANNERGREN et al.** *Mol. Pharmacol.,* 2009, vol. 6 (1), 60
- Remington's Pharmaceutical Sciences. Mack Publishing, 2000
- **JENS T. CARSTENSEN.** Drug stability: Principles & Practice. Marcel Dekker, 1995, 379-80
- **FRIESEN et al.** *J. Med. Chem.,* 2003, vol. 46 (12), 2413
- **O'SHEA et al.** *J. Org. Chem.,* 2005, vol. 70, 3021
- **SAAD et al.** *Curr. Org. Synth.,* 2012, vol. 9 (3), 413
- **SOKOLOV et al.** *Russian J. General Chem.,* 2002, vol. 72 (5), 806
- **KO et al.** *Org. Lett.,* 2009, vol. 11 (3), 609
- **ZHANG et al.** *Tetrahedron,* 2012, vol. 68, 4194
- **AREWANG et al.** *Carbohydr. Res.,* 2007, vol. 342 (7), 970
- **BERRANG et al.** *Synth. Commun.,* 1975, vol. 5, 231
- **FRIEND et al.** *J. Med. Chem.,* 1985, vol. 28, 51
- **ST-PIERRE et al.** *Synthesis,* 2016, vol. 48, 3575
- **MORAIS et al.** *Carbohydr. Res,* 2003, vol. 338, 1369
- **ARTURSSON P. ; KARLSSON J.** *Biochem. Biophys. Res. Commun.,* 1991, vol. 175 (3), 880